(19) Europäisches Patentamt
European Patent Office
Office européen des brevets

(11) EP 0 805 795 B1

(12) **FASCICULE DE BREVET EUROPEEN**

(45) Date de publication et mention de la délivrance du brevet: **23.08.2000 Bulletin 2000/34**

(21) Numéro de dépôt: **96901850.6**

(22) Date de dépôt: **25.01.1996**

(51) Int Cl.[7]: **C07C 233/65**, A01N 37/18, A01N 37/22

(86) Numéro de dépôt international: **PCT/FR96/00123**

(87) Numéro de publication internationale: **WO 96/22965 (01.08.1996 Gazette 1996/35)**

(54) **NOUVEAUX AMIDES AROMATIQUES, LEUR PROCEDE DE PREPARATION, LES COMPOSITIONS LES CONTENANT ET LEUR UTILISATION COMME PESTICIDES**

AROMATISCHE AMIDE, VERFAHREN ZU IHRER DARSTELLUNG, DIESE ENTHALTENDE ZUSAMMENSETZUNGEN UND IHRE VERWENDUNG ALS PESTIZIDE

NOVEL AROMATIC AMIDES, METHOD FOR PREPARING AND COMPOSITIONS CONTAINING SAME, AND USE THEREOF AS PESTICIDES

(84) Etats contractants désignés:
**AT BE CH DE DK ES FR GB GR IE IT LI LU NL PT SE**

(30) Priorité: **26.01.1995 FR 9500889**

(43) Date de publication de la demande:
**12.11.1997 Bulletin 1997/46**

(73) Titulaire: **Hoechst Schering AgrEvo S.A.**
**75020 Paris (FR)**

(72) Inventeurs:
- **DEMASSEY, Jacques**
  **F-77144 Montevrain (FR)**
- **GOHAR, Michel**
  **F-13790 Rousset (FR)**
- **WEHREY, Christian**
  **F-93250 Villemomble (FR)**

(74) Mandataire: **Rippel, Hans Christoph, Dr. et al**
**Aventis CropScience GmbH**
**Patent- und Lizenzabteilung**
**Gebäude K 801**
**65926 Frankfurt am Main (DE)**

(56) Documents cités:
**EP-A- 0 369 762** **EP-A- 0 524 041**
**EP-A- 0 549 418** **WO-A-91/16301**

- **CHEMICAL ABSTRACTS, vol. 118, no. 25, 21 Juin 1993 Columbus, Ohio, US; abstract no. 254810, J. UPADHYAY ET AL.: page 873; XP002002502 & J. INST. CHEM., vol. 64, no. 2, 1992, pages 76-77,**
- **JOURNAL OF ORGANIC CHEMISTRY, vol. 57, no. 17, 1992, EASTON US, pages 4669-75, XP002002497 K. MIZUNO ET AL.: "Insertion of nitrogen oxide and nitrosonium ion into the cyclopropane ring"**
- **CHEMICAL ABSTRACTS, vol. 80, no. 7, 18 Février 1974 Columbus, Ohio, US; abstract no. 36593, T. V. LEONOVA ET AL.: page 253; XP002002503 & ZH. ORG. KHIM., vol. 9, no. 11, 1973, pages 2251-54,**
- **JOURNAL OF ORGANIC CHEMISTRY, vol. 40, no. 21, 1975, EASTON US, pages 3005-10, XP002002498 J. M. HOFFMANN ET AL.: "Cyclopropane ring opening by photolytically generated bromine atoms"**
- **CHEMICAL ABSTRACTS, vol. 66, no. 19, 8 Mai 1967 Columbus, Ohio, US; abstract no. 85497, YU. S. SHABAROV ET AL.: page 909; XP002002504 & ZH. ORG. KHIM., vol. 2, no. 12, 1966, pages 2154-58,**
- **TETRAHEDRON LETTERS, vol. 27, no. 35, 1986, OXFORD GB, pages 4125-28, XP002002499 R. A. MOSS: "Are Phenylcarbenes ambiphilic?"**
- **CHEMICAL ABSTRACTS, vol. 51, no. 2, 25 Janvier 1960 Columbus, Ohio, US; abstract no. 3519, MASAYUKI HAMADA: XP002002505 & BOTYU-KAYAKU, vol. 21, 1956, pages 22-28,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 805 795 B1

- **CHEMICAL ABSTRACTS, vol. 62, no. 12, 7 Juin 1965 Columbus, Ohio, US; abstract no. 14536c, YU. S. SHABAROV ET AL.: XP002002506 & ZH. OBSHCH. KHIM., vol. 35, no. 2, 1965, pages 243-245,**
- **TETRAHEDRON LETTERS, vol. 28, no. 7, 1987, OXFORD GB, pages 801-802, XP002002500 XIAN HUANG ET AL.: "A nover route for the synthesis of .alpha.,.beta.-unsaturated esters, ketones and nitriles using dibutyl telluride"**
- **CHEMICAL ABSTRACTS, vol. 93, no. 21, 24 Novembre 1980 Columbus, Ohio, US; abstract no. 204404, S. P. DHOUBHADEL ET AL.: page 666; XP002002507 & J. NPA, vol. 12, no. 1, 1979, pages 31-33,**
- **CHEMICAL ABSTRACTS, vol. 120, no. 25, 20 Juin 1994 Columbus, Ohio, US; abstract no. 323477, S. M. DESENKO ET AL.: page 909; XP002002508 & DOPOV. AKAD. NAUK. UKR., no. 8, 1993, pages 122-125,**
- **J. MED. CHEM. (1983), 26(9), 1282-93, XP002002501 DAWSON, MARCIA I. ET AL: "Aromatic retinoic acid analogs. 2. Synthesis and pharmacological activity"**

## Description

La présente invention concerne de nouveaux amides aromatiques, leur procédé de préparation, les compositions les contenant et leur utilisation comme pesticides.

Certains 5-cyclopropyl pentadièneamides sont décrits dans les demandes de brevet européen publiées sous les numéros 0 369 762, 0 524 041 et 0 553 081 comme possédant de telles propriétés.

Il a maintenant été trouvé que les composés répondant à la formule (I) telle que définie ci-après possèdent aussi d'intéressantes propriétés pesticides, ce qui les rend susceptible d'être utilisables pour la protection des plantes, la sauvegarde de l'hygiène des locaux publics ou privés, ainsi que dans la mise en oeuvre de traitement thérapeutique à usage vétérinaire, voir humain.

Les produits de formule (I) sont de plus photostables et/ou peu toxiques pour les mammifères et correspondent ainsi parfaitement aux exigences de l'industrie agrochimique moderne.

La présente invention a donc pour objet sous toutes leurs formes stéréoisomères ou sous forme de mélanges de stéréoisomères, les composés de formule (I) :

$$Q\text{-}(CH_2)_a\text{-}(X_1)_b\text{-}Q_1\text{-}Q_2\text{-}C(X_2)\text{-}N(R_1)(R_2) \qquad (I)$$

dans laquelle :

Q représente un radical aryle ou un radical dérivé d'un hydrocarbure bicyclique condensé comportant un cycle benzénique, qui est lié au groupe adjacent $(CH_2)_a$ par un atome de carbone dudit cycle benzénique, Q comporte de 6 à 12 atomes de carbone et peut être soit non substitué, soit substitué,
a et b sont identiques ou différents et sont indépendamment l'un de l'autre égaux à 0 ou à 1,
$X_1$ représente un radical bivalent oxy, thio, sulfinyle ou sulfonyle,
$X_2$ représente un atome d'oxygène ou un atome de soufre,
$Q_1$ représente un radical cyclopropanediyle, qui est soit non substitué, soit substitué,
$Q_2$ représente un radical arylène ou un radical dérivé d'un hydrocarbure bicyclique condensé comportant un cycle benzénique, qui est lié aux groupes adjacents $Q_1$ et $C(X_2)$ par deux atomes de carbone dudit cycle benzénique, $Q_2$ comporte de 6 à 12 atomes de carbone et peut indépendamment de Q, être soit non substitué, soit substitué
$R_1$ et $R_2$ représentent indépendamment l'un de l'autre :

**soit** un atome d'hydrogène,
**soit** un radical dérivé d'un hydrocarbure cyclique ou acyclique, aromatique ou non aromatique, linéaire ou ramifié, saturé ou insaturé, et comportant de 1 à 12 atomes de carbone, lui-même pouvant être soit non substitué, soit substitué,
**soit** un radical dérivé d'une structure linéaire ou ramifiée comportant de 1 à 20 atomes de carbone et de 1 à 6 hétéroatomes choisis parmi les atomes d'oxygène, d'azote ou de soufre, lui-même pouvant être soit non substitué, soit substitué,
**soit** un radical dérivé d'un hétérocycle aromatique ou non aromatique comportant de 5 à 9 atomes de carbone et de 1 à 4 hétéroatomes choisis parmi les atomes d'oxygène, d'azote ou de soufre, ce radical pouvant être non substitué ou substitué,
**soit** un groupe (A) :

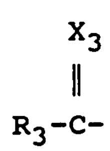

dans lequel $X_3$ représente un atome d'oxygène ou un atome de soufre, et $R_3$ représente un atome d'hydrogène ou un groupe $R_4\text{-}(T_1)_i$ dans lequel i est égal à 0 ou à 1, $T_1$ représente un groupe bivalent oxy, carbonyle ou oxycarbonyle et $R_4$ représente un atome d'hydrogène ou un radical dérivé d'un hydrocarbure cyclique ou acyclique, aromatique ou non aromatique, linéaire ou ramifié, saturé ou insaturé, et comportant de 1 à 20 atomes de carbone, lui-même pouvant être soit non substitué, soit substitué,
**soit** un groupe (B) :

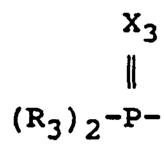

dans lequel $R_3$ et $X_3$ sont tels que définis plus haut,

**soit** un groupe (C) :

$$-T_2-R_5$$

dans lequel $T_2$ représente un radical bivalent thio, sulfinyle, sulfonyle ou sulfonyloxy et $R_5$ représente un atome d'hydrogène ou un radical dérivé d'un hydrocarbure cyclique ou acyclique, aromatique ou non aromatique, linéaire ou ramifié, saturé ou insaturé, et comportant de 1 à 8 atomes de carbone, lui-même pouvant être soit non substitué, soit substitué,

**soit** un groupe (D) :

$$[S(O)_j]_k-N(R_6)(R_7)$$

dans lequel j est égal à 0, 1 ou 2, k est égal à 0 ou 1, $R_6$ représente un atome d'hydrogène ou un radical dérivé d'un hydrocarbure cyclique ou acyclique, aromatique ou non aromatique, linéaire ou ramifié, saturé ou insaturé, et comportant de 1 à 8 atomes de carbone, lui-même pouvant être soit non substitué, soit substitué, et $R_7$ représente un groupe carboxy, fluorocarbonyle, alcoxycarbonyle comportant de 2 à 5 atomes de carbone, ou un radical acyle comportant de 1 à 5 atomes de carbone, ou un radical alkyle comportant de 1 à 4 atomes de carbone substitué par un groupe cyano ou par un radical alcoxycarbonyle comportant de 2 à 5 atomes de carbone, ou acyle comportant de 1 à 5 atomes de carbone, ou par un radical aryle comportant de 6 à 10 atomes de carbone, non substitué ou substitué par un ou plusieurs groupes choisis parmi les groupes halo, cyano, nitro, trifluorométhyle, trifluorométhoxy, trifluorométhylthio ou les radicaux alkyle comportant de 1 à 4 atomes de carbone ou alkoxy comportant de 1 à 4 atomes de carbone ou $R_6$ et $R_7$ forment ensemble avec l'atome d'azote auquel ils sont liés un hétérocycle azoté comportant de 3 à 10 chaînons renfermant éventuellement un ou plusieurs autres hétéroatomes cycliques choisis parmi l'oxygène, l'azote et le soufre et éventuellement substitué,

**soit** $R_1$ et $R_2$ représentent ensemble avec l'atome d'azote auquel ils sont liés un hétérocycle azoté comportant de 3 à 10 chaînons renfermant éventuellement un ou plusieurs autres hétéroatomes cycliques choisis parmi l'oxygène, l'azote et le soufre et éventuellement substitué étant entendu que dans ladite formule (I) lorsque Q représente un radical 2-(phénylaminocarbonyl) phényle, Q1 un radical 1,2-cyclopropanediyle, $Q_2$ un radical ortho-phénylène, $X_2$ un atome d'oxygène, $R_2$ un atome d'hydrogène et que a et b sont égaux à 0, $R_1$ ne représente pas un radical phényle.

Par composé de formule (I) on désigne tous les isomères géométriques et les stéréoisomères possibles pris individuellement ou en mélange.

Par radical dérivé d'un hydrocarbure bicyclique condensé comportant un cycle benzénique, on désigne notamment pour la définition de Q les radicaux indanyle, indényle, naphtyle, dihydronaphtyle ou tétrahydronaphtyle et pour la définition de $Q_2$ notamment les radicaux ortho-phénylène, méta-phénylène, para-phénylène, 1,2-naphtylène, 1,3-naphtylène, 1,4-naphthylène, 4,5-indènylène, 4,6-indènylène, 4,7-indènylène, 4,5-indanylène, 4,6-indanylène, 4,7-indanylène, 1,2,3,4-tétrahydro 5,6-naphtylène, 1,2,3,4-tétrahydro 5,7-naphtylène, 5,8-tétrahydronaphtylène.

Par radical dérivé d'un hydrocarbure cyclique, aromatique ou non aromatique, saturé ou insaturé, on désigne notamment le radical phényle, les radicaux cycloalkyle tels que les radicaux cyclopropyle, cyclobutyle, cyclopentyle ou cyclohexyle ainsi que les radicaux alkyle substitué par un radical cyclique tels que le radical benzyle ou cyclopropylméthyle.

Par radical dérivé d'un hydrocarbure acyclique, linéaire ou ramifié, saturé ou insaturé, on désigne les radicaux alkyle tels que notamment méthyle, éthyle, propyle, isopropyle, butyle, isobutyle, sec-butyle, tert-butyle, pentyle, isopentyle, néopentyle, tert-pentyle, hexyle ou isohexyle, les radicaux alkényle, tels que notamment les radicaux vinyle, 1-propényle, 2-méthyl 2-propényle, isoprènyle, les radicaux alkynyles tels que notamment les radicaux éthynyle, 1-propynyle, 2-propynyle ainsi que les radicaux comportant plusieurs insaturations tels que les radicaux alcadiényle tels

que 1,3-butadiényle ou le radical pent-2-ène-4-ynyle.

Par radical dérivé d'une structure linéaire ou ramifiée, comportant de 1 à 20 atomes de carbone et de 1 à 6 hétéroatome, on désigne notamment les radicaux dérivés des alcanes dont certains atomes de carbone sont remplacés par des atomes d'oxygène, de soufre ou un groupe -NH-, tels que les radicaux hydroxyméthyle, méthoxyméthyle, éthoxyméthyle, 2-hydroxyéthyle, 2-méthoxyéthyle, 2-(2-méthoxy éthoxy) éthyle, (2-méthoxyéthoxy) méthyle, [2-(2-méthoxy éthoxy) éthoxy] méthyle, éthoxyméthyle, [2-(2-butoxy éthoxy) éthoxy] méthyle, 2-méthylthio éthyle, 2-(méthylamino) éthyle, méthylaminométhyle, butylaminobutyl ou propoxyméthyle.

Par radical dérivé d'un hétérocycle aromatique ou non aromatique, on désigne notamment les radicaux thiényle, furyle, pyrannyle, pyrrolyle, 2H-pyrrolyle, imidazolyle, pyrazolyle, pyridyle, pyrazinyle, pyrimidinyle, pyridazinyle, isothiazolyle, isoxazolyle, furazannyle, thiazolyle, oxazolyle, pyrrolidinyle, pyrrolinyle, imidazolidinyle, pyrazolinyle, pipéridyle, pipérazinyle, morpholinyle, azépinyle, thiazinyle, tétrazinyle, oxathiolannyle ou thiadiazinyle.

Lorsque Q est substitué par un ou plusieurs substituants, ces substituants sont choisis notamment parmi les atomes d'halogène ou les groupes ou méthylènedioxy, difluorométhylènedioxy, tétrafluoro éthylènedioxy, cyano, nitro, cyanato, thiocyanato, pentafluorothio, fluorosulfonyle ou $R-(T)_c-$ dans lequel R représente un atome d'hydrogène ou un radical dérivé d'un hydrocarbure cyclique ou acyclique, aromatique ou non aromatique, linéaire ou ramifié, saturé ou insaturé, et comportant de 1 à 8 atomes de carbone, lui-même pouvant être soit non substitué, soit substitué par un ou plusieurs atomes d'halogène, c est égal à 0 ou à 1 et T représente un groupe bivalent oxy, carbonyle, carbonyloxy, oxycarbonyle, thio, sulfinyle, sulfonyle, ou sulfonyloxy, $-(CO)_d-N(R')-(CO)_e-(O)_f-$ ou $-N(R'')-S(O)_g-$ dans lesquels R' et R" représentent indépendamment de R un atome d'hydrogène, ou un radical dérivé d'un hydrocarbure cyclique ou acyclique, aromatique ou non aromatique, linéaire ou ramifié, saturé ou insaturé, et comportant de 1 à 8 atomes de carbone, lui-même pouvant être soit non substitué, soit substitué par un ou plusieurs atomes d'halogène, d, e et f sont égaux à 0 ou à 1, f est égal à 0 quand e est égal à 0, la somme e + d est égale à 0 ou à 1, et g est égal à 0, à 1 ou à 2,

Lorsque $Q_1$ est substitué, les substituants sont choisis notamment parmi les atomes d'halogène, les radicaux cyano, azido, ou aliphatiques, saturés ou insaturés comportant de 1 à 4 atomes de carbone non substitués ou substitués par un ou plusieurs atomes d'halogène, tel que les radicaux méthyle, vinyle ou éthynyle.

Lorsque $Q_2$ est substitué, ces substituants sont choisis notamment parmi les atomes d'halogène ou les groupes méthylènedioxy, difluorométhylènedioxy, tétrafluoro éthylènedioxy, cyano, nitro, amino, alkylamino, alkénylamino, cyanato, thiocyanato, pentafluorothio, fluorosulfonyle ou $R-(T)_c-$ dans lequel R représente un atome d'hydrogène ou un radical dérivé d'un hydrocarbure cyclique ou acyclique, aromatique ou non aromatique, linéaire ou ramifié, saturé ou insaturé, et comportant de 1 à 8 atomes de carbone, lui-même pouvant être soit non substitué, soit substitué par un ou plusieurs atomes d'halogène, c est égal à 0 ou à 1 et T représente un groupe bivalent oxy, carbonyle, carbonyloxy, oxycarbonyle, thio, sulfinyle, sulfonyle, ou sulfonyloxy, $-(CO)_d-N(R')-(CO)_e-(O)_f-$ ou $-N(R'')-S(O)_g-$ dans lesquels R', R", d, e, f et g sont tels que définis précédemment.

Lorsque dans ce qui précède ou dans ce qui suit, il est indiqué qu'un radical peut être substitué par un ou plusieurs atomes d'halogène, ces atomes d'halogène peuvent être des atomes de fluor, de chlore, de brome ou d'iode. Lorsqu'il s'agit d'un radical polysubstitué par des atomes de fluor, on désigne notamment les radicaux perfluorés.

Lorsque dans ce qui précède, un radical dérivé d'un hydrocarbure cyclique ou acyclique, aromatique ou non aromatique, linéaire ou ramifié, saturé ou insaturé est substitué, ces substituants sont choisis notamment parmi les atomes d'halogène ou les groupes ou méthylènedioxy, difluorométhylènedioxy, tétrafluoro éthylènedioxy, cyano, nitro, cyanato, thiocyanato, pentafluorothio, fluorosulfonyle ou $R-(T)_c-$ dans lequel R représente un atome d'hydrogène ou un radical dérivé d'un hydrocarbure cyclique ou acyclique, aromatique ou non aromatique, linéaire ou ramifié, saturé ou insaturé, et comportant de 1 à 8 atomes de carbone et éventuellement 1 ou plusieurs hétéroatomes, lui-même pouvant être soit non substitué, soit substitué par un ou plusieurs atomes d'halogène, c est égal à 0 ou à 1 et T représente un groupe bivalent oxy, carbonyle, carbonyloxy, oxycarbonyle, thio, sulfinyle, sulfonyle, ou sulfonyloxy, $-(CO)_d-N(R')-(CO)_e-(O)_f-$ ou $-N(R'')-S(O)_g-$ dans lesquels R', R", d, e, f et g sont tels que définis précédemment.

Les composés de formule (I) dans laquelle Q, $Q_1$, $Q_2$ sont non substitués ou substitués comme décrit dans les paragraphes précédents, constituent une première variante préférée de la présente invention.

Dans une deuxième variante préférée de la présente invention, Q représente un radical phényle ou un radical naphtyle, non substitué ou substitué par 1 à 3 substituants et notamment un radical choisi parmi les radicaux phényle, 2-chloro phényle, 3-chloro phényle, 3-bromophényle, 3-(trifluorométhyl) phényle, 4-chloro phényle, 4-bromo phényle, 4-iodo phényle, 4-(trifluorométhyl) phényle, 4-nitrophényle, 4-méthoxy phényle, 4-(difluorométhoxy) phényle, 4-(trifluorométhoxy) phényle, 3-bromo 4-(difluorométhoxy) phényle, 4-(2,2-dibromo éthényl) phényle, 4-éthynyl phényle, 4-benzyl phényle, 3,4-dibromo phényle, 2,4-dichlorophényle, 3,4-dichloro phényle, 3,4-difluorophényle, 3-chloro 4-iodo phényle, 4-bromo 3-chloro phényle, 4-bromo 2-fluoro phényle, 4-bromo 3-fluoro phényle, 4-bromo 3-(trifluorométhyl) phényle, 4-chloro 3-(trifluorométhyl) phényle, 3,5-bis(trifluorométhyl) phényle, 3,4,5-trichloro phényle, 4-bromo 3,5-dichloro phényle, 3-phénoxyphényle, 4-(fluoro 3-phénoxy) phényle, 3-bromo 4-(trifluorométhylsulfonyloxy) phényle, 3,4-bis (trifluorométhylsulfonyloxy) phényle, 2-naphtyle ou 5-bromo 2-naphtyle ou 6-bromo 1-napthyle.

Dans une troisième variante préférée de la présente invention, la configuration stérique de $Q_1$ est telle que

le groupe $Q-(CH_2)_a-(X_1)_b-$ est en position trans par rapport au groupe $Q_2-C(X_2)-N(R_1)(R_2)$.

Dans une quatrième variante préférée de la présente invention, $Q_1$ est non substitué ou substitué par 1 ou 2 atomes d'halogène ou 1 ou 2 radicaux méthyle et notamment un radical choisi parmi les radicaux 1,2-cyclopropanediyle, 1-fluoro 1,2-cyclopropanediyle, 1-chloro 1,2-cyclopropanediyle, 1-bromo 1,2-cyclopropanediyle, 1-méthyle 1,2-cyclopropanediyle, 3,3-dibromo 1,2-cyclopropanediyle et 1,2-difluoro 1,2-cyclopropanediyle.

Dans une cinquième variante préférée de la présente invention, $Q_2$ représente un des radicaux ortho-phénylène, méta-phénylène, para-phénylène, 1,2-naphtylène, 1,3-naphtylène, 1,4-naphtylène, 1,5-naphtylène, 2,5-naphtylène, 4,5-indènylène, 4,6-indènylène, 4,7-indènylène, 4,5-indanylène, 4,6-indanylène, 4,7-indanylène, 1,2,3,4-tétrahydro 5,6-naphtylène, 1,2,3,4-tétrahydro 5,7-naphtylène, 1,2,3,4-tétrahydro 5,8-naphtylène, non substitué ou substitué par 1 à 4 substituants, et notamment les radicaux 2-méthyl 1,4-phénylène, 2,3,5,6-tétrafluoro 1,4-phénylène, 3-méthyl 1,4-phénylène, 2-chloro 1,4-phénylène, 2-acétylamino 5-chloro 1,4-phénylène, 2-amino 5-chloro 1,4-phénylène, 2-diméthylamino 5-chloro 1,4-phénylène.

Dans une sixième variante préférée de la présente invention, $R_1$ représente un radical alkyle ramifié comportant de 1 à 6 atomes de carbone tel que le radical isopropyle, isobutyle, 1,2-diméthyl propyle, 1,1,2-triméthyl propyle ou 2,2-diméthyl propyle, 1-méthylpropyle, 2-méthylbutyle, 2,2-diméthyl 1-méthylpropyle, 3-chloropropyle, cyclobutyle, cyclohexyle, cyclopropylméthyle, un radical (2-méthyl 1,3-dioxolan 2-yl) méthyle, ou un radical alkényle ramifié comportant de 2 à 5 atomes de carbone tel que le radical 2-méthyl 2-propényle, 2-chloro 2-propényle, 2-bromo 2-propényle ou encore un radical phényle non substitué ou un radical phényle substitué tel que le radical 2-fluoro phényle, 2-chloro phényle, 2-bromo phényle, 2-iodo phényle, 2-nitro phényle, 2-hydroxy phényle, 2-méthoxyphényle, 2-cyano phényle, 2-amino phényle, 2-(diméthylamino) phényle, 2-(trifluorométhoxy) phényle, 2-phénoxy phényle, 2-méthoxycarbonyl phényle, 2-éthoxycarbonyl phényle, 2-phénylcarbonylphényle, 2-formyl phényle, 2-acétyl phényle, 2-(méthylthio) phényle, 2-fluorosulfonyl phényle, 2-éthynyl phényle, 2-(1-méthyl 2-propényl) phényle, 2-(hydroxyméthyl) phényle, 2-méthyl phényle, 2-(terbutyl) phényle, 2-(fluorométhyl) phényle, 2-(difluorométhyl) phényle, 2-(trifluorométhyl) phényle, 2-éthyl phényle, 2-propyl phényle, 2-isopropyl phényle, 2-(pyridin-1-yl) phényle, 3-chloro phényle, 3-bromophényle, 3-méthoxy phényle, 3-fluoro phényle, 3-méthyl phényle, 3-phénoxyphényle, 4-fluoro phényle, 4-chloro phényle, 4-cyano phényle, 4-(cyanométhyl) phényle, 4-méthoxy phényle, 4-méthyl phényle, 4-(terbutyl) phényle), 4-(trifluorométhyl) phényle, 4-phénoxyphényle, 4-benzyloxyphényle, 4-méthoxycarbonylphényle, 4-cyclohexylphényle, 2,4-dichlorophényle, 2,5-dichlorophényle, 2,6-dichlorophényle, 3,4-dichlorophényle, 3,5-dichlorophényle, 2,3-diméthylphényle, 2,4-diméthylphényle, 2,5-diméthylphényle, 2,6-diméthylphényle, 3,5-diméthylphényle, 2,4,6-triméthylphényle, 2,5-diméthoxyphényle, 3,4-diméthoxyphényle, 3,5-diméthoxyphényle, 2-méthoxy 5-méthylphényle, 2-méthoxy 5-trifluorométhyle, 3-méthoxy 5-trifluorométhylphényle, 2-méthoxy 5-nitrophényle, 2-nitro 4-méthoxyphényle, 2-méthyl 4-méthoxyphényle, 3,5-dichloro 4-méthoxyphényle, 4,5-dichloro 2-méthoxyphényle, 4-bromo 3,5-dichlorophényle, 2-(1-méthyléthyl) phényle, 3-chloro 2-méthyl phényle, 3-fluoro 2-méthyl phényle, 3-bromo 2-méthyl phényle, 2,3-dichloro phényle, 2,3-dibromo phényle, 3,4-dibromophényle, 4-fluoro 2-méthyl phényle, 4-chloro 2-fluoro phényle, 4-fluoro 2-(trifluorométhyl) phényle, 2,4-difluoro phényle, 2-chloro 4-méthylphényle, 2-chloro 5-méthyl phényle, 5-chloro 2-méthyl phényle, 5-fluoro 2-méthyl phényle, 5-iodo 2-méthyl phényle, 2,6-difluoro phényle, 2-fluoro 4-méthylphényle, 2-fluoro 6-méthyl phényle, 2-chloro 6-méthyl phényle ou bien un radical dérivé d'un hétérocycle tel que le radical 2-chloro 3-pyridyle, 3-pyridyle, 2-pyridyle, 4-pyridyle, 2-chloro 3-pyridyle, 3-méthyl pyridyle, 5-méthyl isoxazol-3-yle, 1,3,4-thiadiazol-2-yle, 4-pyrimidinyle, 3-pyrazolyle, 4-(trifluorométhyl) thiazol-2-yle, 5-chloro 4-(trifluorométhyl) thiazol-2-yle, 1-pipéridinyle, 2,6-diméthyl 1-pipéridinyle, 4-morpholinyle ou 1-perhydroazépinyle, ou enfin $R_1$ et $R_2$ représentent ensemble avec l'atome d'azote auquel ils sont liés un hétérocycle tel que le radical 4-thiomorpholinyle, 4-morpholinyle, 1-aziridinyle, 1-pipéridinyle, 1-pyrrolidinyle, 2-méthylpipéridin-1-yle, 2-hydroxyméthylpyrrolidin-1-yle.

Dans une septième variante préférée de la présente invention, $R_2$ représente un atome d'hydrogène, un radical alkyle comportant de 1 à 4 atomes de carbone, notamment le radical éthyle, un radical benzyle, un radical éthoxyméthyle, un radical propoxyméthyle, un groupe (A) dans lequel $X_3$ représente un atome d'oxygène et $R_3$ représente un radical alkyle comportant de 1 à 11 atomes de carbone, un radical alcoxycarbonyle comportant de 2 à 5 atomes de carbone ou phénoxycarbonyle, et $R_2$ représente notamment dans ce cas, le radical acétyle hexanoyle, décanoyle, 2-méthoxy 2-oxo acétyle, 2-éthoxy 2-oxo acétyle, 2-phénoxy 2-oxo acétyle, un groupe (B) dans lequel $R_3$ représente un radical alkoxy comportant de 1 à 4 atomes de carbone et $R_2$ représente notamment dans ce cas le radical diéthoxyphosphonyle, un groupe (C) dans lequel $T_2$ représente un radical bivalent thio et $R_5$ un radical trifluorométhyle ou pentafluoroéthyle ou un radical phényle non substitué ou substitué par un ou plusieurs atomes d'halogène ou par un ou plusieurs radicaux alkyle comportant de 1 à 4 atomes de carbone, et dans ce cas $R_2$ représente notamment le radical trifluorométhylthio, pentafluoroéthylthio, ou phénylthio, (4-chloro phényl) thio, (2-méthyl phényl) thio, (4-méthyl phényl) thio ou (4-tertbutyl phényl) thio ou un groupe (D) dans lequel j est égal à O et $R_2$ représente notamment dans ce cas le radical (N-formyl N-méthyl amino) thio ou (N-méthoxycarbonyl N-méthyl amino) thio.

Dans une huitième variante préférée, a et b sont égaux à 0.

L'invention a aussi pour objet un procédé de préparation des composés de formule (I), caractérisé en ce que l'acide ou le dérivé d'acide correspondant de formule (II) :

$$Q\text{-}(CH_2)_a\text{-}(X_1)_b\text{-}Q_1\text{-}Q_2\text{-}C(O)\text{-}Z \qquad (II)$$

dans laquelle Q, a, b, $Q_1$ et $Q_2$ sont tels que définis précédemment et Z représente un groupe hydroxy, un groupe halogène, un radical alkoxy comportant de 1 à 4 atomes de carbone ou un groupe -P(O) (OΦ)-NHΦ, dans lequel Φ représente un groupe phényle,
est mis à réagir avec une amine de formule (III) :

$$HN(R_1)(R_2) \qquad (III)$$

dans laquelle $R_1$ et $R_2$ sont tels que définis précédemment, pour obtenir un produit de formule (I) correspondant, dans laquelle $X_2$ représente un atome d'oxygène, qui si nécessaire, est converti en un produit de formule (I) dans laquelle ($X_2$) représente un atome de soufre.

Les produits de formule (I) obtenus selon le procédé ci-dessus peuvent être le cas échéant séparés en leurs isomères optiquement actifs.

La séparation des isomères peut être effectué selon les méthodes connues de l'homme du métier par exemple par cristallisation ou par chromatographie.

Selon des conditions préférentielles de mise en oeuvre du procédé de préparation des composés de formule (I) telle qu'indiqué ci-dessus, la réaction d'amidification est en général effectuée à une température comprise entre -25° et +150°C dans un solvant anhydre et aprotique tel que l'éther, le dichlorométhane, le toluène ou le benzène.

Les conditions réactionnelles précises dépendent aussi de la nature du groupe Z ; par exemple quand Z représente un groupe alkoxy, la réaction est à température élevée par exemple entre 25° et 125°C, notamment à 50°C et de préférence en présence d'un trialkylaluminium, tel que le triméthylaluminium, qui forme un complexe avec l'amine de formule (III).

Quand Z représente un groupe halogéné, ou phosphoroimidate, la réaction s'effectue entre 0° et 30°C, notamment à température ordinaire et de préférence en présence d'une amine tertiaire, telle que la triéthylamine. La conversion d'un amide en thioamide se fait selon des méthodes connues de l'homme du métier, telle que la réaction de l'amide avec du pentasulfure de phosphore, du sulfure d'hydrogène, du trisulfure de bore ou du bromure de thiophosphoryle, ou le réactif de LAWESSON.

Lorsque le dérivé de l'acide de formule (II) est un halogénure d'acide, par exemple le chlorure d'acide, il est préparé à partir de l'acide par réaction avec le réactif approprié comme le chlorure d'oxalyle ou le chlorure de thionyle. Quand Z représente un groupe -P(O)(OΦ)-NHΦ, le dérivé correspondant est préparé à partir de l'acide par réaction avec Cl-P(O)(OΦ)-NHΦ. L'acide de formule (II) dans laquelle Z représente un groupe hydroxy peut être préparé par hydrolyse d'un ester.

La préparation du composé de formule (II) dans laquelle Z représente un radical alkoxy peut être faite notamment :

a) par cyclopropanation d'un composé de formule (IV) :

$$Q\text{-}(CH_2)_a\text{-}(X_1)_b\text{-}C(R_9)\text{=}C(R_{10})\text{-}Q_2\text{-}C(O)\text{-}Z \qquad (IV)$$

dans laquelle Q, a, $X_1$, b, $Q_2$ et Z sont tels que définis précédemment et $R_9$ et $R_{10}$ représentent indépendamment l'un de l'autre un atome d'hydrogène, un atome d'halogène ou un radical alkyle, avec un générateur de carbène, tel que le diazométhane, le dibromométhane ou le diiodométhane en présence de diéthylzinc ;

b) par cyclocondensation d'un dérivé de formule (V) :

$$Q\text{-}(CH_2)_a\text{-}(X_1)_b\text{-}C(R_9)\text{=}CH(R_{10}) \qquad (V)$$

dans laquelle Q, a, $X_1$, b, $R_9$ et $R_{10}$ sont tels que définis précédemment avec un composé de formule (VI) :

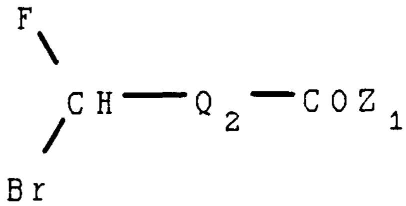

(VI)

dans laquelle $Z_1$ représente un radical alcoxy, pour conduire au composé de formule (II) dans laquelle $Q_1$ représente un radical cyclopropanediyle substitué en position 1 par un atome de fluor.

L'acide de formule (II) dans laquelle Z représente un radical hydroxy peut aussi être préparé par élimination d'azote à partir d'un composé de formule (VII) :

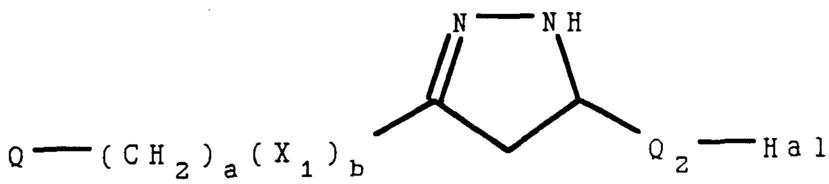

(VII)

pour conduire au composé de formule (VIII) :

$$Q\text{-}(CH_2)_a(X_1)_b\text{-}Q_1\text{-}Q_2\text{-}Hal \quad (VIII)$$

dans laquelle Q, a, $X_1$, b, $Q_2$ sont tels que définis précédemment, Hal représente un atome de brome ou d'iode et $Q_1$ représente un radical cyclopropanediyle non substitué, produit de formule (VIII) qui est transformé en dérivé cyano de formule (IX) :

$$Q\text{-}(CH_2)_a(X_1)_b\text{-}Q_1\text{-}Q_2\text{-}C{\equiv}N \quad (IX)$$

lequel est ensuite converti en acide correspondant de formule (II).

Le composé de formule (VII) est préparé par réaction de l'hydrazine avec le composé de formule (X) :

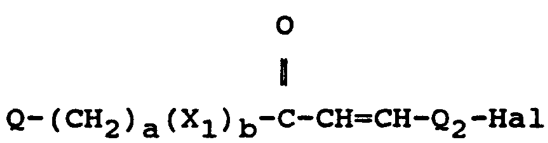

(X)

le composé de formule (X) se prépare par condensation en milieu basique d'un composé de formule (XI) :

$$Q\text{-}(CH_2)_a(X_1)_b\text{-}CO\text{-}CH_3 \quad (XI)$$

avec un aldéhyde aromatique de formule (XII) :

$$Hal\text{-}Q_2\text{-}CHO \quad (XII)$$

produits de formules (XI) et (XII) qui sont soit commerciaux, soit aisément synthétisables par l'homme du métier.

Le composé de formule (VI) est préparé par bromation d'un composé de formule (XIII) :

$$F\text{-}CH_2\text{-}Q_2\text{-}COZ_1 \qquad (XIII)$$

qui lui-même se prépare à partir d'un composé de formule (XIV) :

$$HO\text{-}CH_2\text{-}Q_2\text{-}COZ_1 \qquad (XIV)$$

qui est commercial ou aisément synthétisable par l'homme du métier.

Les composés de formule (IV) et (V) sont connus ou peuvent être obtenus selon les méthodes usuelles connues de l'homme du métier.

Les conditions expérimentales sont illustrées par les exemples décrits dans la présente demande.

L'invention a aussi pour objet les composés de formules (II), (VI), (VII), (VIII), (IX) et (X) à l'exception des produits dont les noms suivent :

- le 5-(2-phényl cyclopropyl) 2-acétoxy benzoate de méthyle
- le 2-(2-phényl cyclopropyl) benzoate de méthyle
- le 1,2-diphényl cyclopropane 4,4'di carbonitrile
- le 4-[2-(1-naphtyl) cyclopropyl] benzonitrile et
- le 3-(4-bromo phényl) 1-phényl propénone

et à l'exception des produits de formule (VIII) dans laquelle $X_1$ représente un groupe sulfonyle.

Les composés de formule (I) peuvent être utilisés pour lutter contre des organismes nuisibles tels que les arthropodes, par exemple les insectes et acariens, et les helminthes, par exemple les nématodes, ou les mollusques, par exemple les limaces. La présente invention a donc pour objet un procédé pour la lutte contre les arthropodes et/ou helminthes et/ou mollusques, qui comprend l'administration aux arthropodes et/ou helminthes et/ou mollusques ou à leur environnement d'une quantité d'un composé de formule (I) suffisante pour détruire l'organisme nuisible. La présente invention a aussi pour objet un procédé pour lutter contre et/ou éradiquer les infestations par les arthropodes et/ou helminthes et/ou mollusques chez les animaux (y compris l'être humain) et/ou les plantes (y compris les arbres) et/ou les produits stockés, qui comprend l'administration à l'animal ou au site d'une quantité efficace d'un composé de formule (I). L'invention a aussi pour objet les composés de formule (I) à utiliser en médecine humaine et vétérinaire, en santé publique et/ou en agriculture pour la lutte contre les arthropodes et/ou helminthes nuisibles.

Les composés de formule (I) sont d'une valeur particulière pour la protection des récoltes sur pied, du fourrage, des plantations, des cultures en serres, des vergers et des vignobles, des plantes ornementales et des arbres en plantation et en forêt, par exemple des céréales (comme le maïs, le froment, le riz et le sorgho), du coton, du tabac, des légumes et salades (comme les haricots, les choux, les curcubitacées, les laitues, les oignons, les tomates et les poivrons), des cultures vivrières (par exemple la pomme de terre, la betterave sucrière, l'arachide, le soja et le colza), de la canne à sucre, les prairies et cultures de fourrage (comme le maïs, le sorgho et la luzerne), des plantations (comme celles produisant le thé, le café, le cacao, la banane, l'huile de palme, la noix de coco, le caoutchouc et les épices), des vergers et bois plantés (comme ceux produisant les fruits à noyaux et pépins, les agrumes, les kiwis, les avocats, les mangues, les olives et les noix), des vignobles, des plantes ornementales, des fleurs et buissons en serre et dans les jardins et parcs, des arbres forestiers (à feuillage tant caduc que persistant) dans les forêts, plantations et pépinières.

Ils sont précieux aussi pour la protection du bois d'oeuvre (sur pied, abattu, transformé, stocké ou dans les bâtiments) contre l'attaque par les sirex (par exemple Urocerus), les coléoptères (par exemple les scolytidés, les platypodidés, les lyctidés, la bostrychidés, les cérambucidés et les anobiidés) et les termites.

Ils trouvent leur application dans la protection des produits stockés, comme les grains, les fruits, les noix, les épices et le tabac, à l'état entier, moulu ou transformé en produits, contre l'attaque par les mites, coléoptères et charançons. Ils protègent également les produits animaux stockés, comme les peaux, les poils, la laine et les plumes, sous forme naturelle ou convertie (par exemple en tapis ou matières textiles), contre l'attaque par les mites et coléoptères, de même que la viande et le poisson contre l'attaque par les coléoptères et les mouches.

Les composés de formule générale (I) sont d'une utilité particulière pour la lutte contre les arthropodes, les helminthes ou les mollusques qui sont nuisibles pour l'homme et les animaux domestiques ou sont les propagateurs ou vecteurs de maladies frappant ces derniers, par exemple celles décrites ci-dessus, plus spécialement dans le domaine de la lutte contre les tiques, la gale, les poux, les puces, les moucherons et les mouches, causes de morsures et de nuisances.

L'invention concerne notamment l'utilisation des composés de formule (I) telle que définie précédemment,

comme pesticide notamment comme insecticides, acaricide et nématicide dans la protection des cultures notamment les cultures de riz et de coton, ou pour le traitement des lieux de stockage des produits desdites cultures et notamment comme insecticide ou acaricide dans les locaux domestiques ou publics.

Les composés de formule (I) peuvent être utilisés à de telles fins par application des composés tels quels ou sous forme diluée de façon connue à l'état de bain, de spray, de brouillard, de vernis, de mousse, de poudre, de poudre à poudrer, de suspension aqueuse, de pâte, de gel, de shampooing, d'onguent, de solide combustible, de mat de vaporisation, de spirale combustible, d'appât, de supplément alimentaire, de poudre mouillable, de granules, d'aérosol, de concentrés émulsifiables, de suspensions huileuses, de solutions huileuses, de bombe sous pression, d'article imprégné, de lotion ou d'autres compositions standard bien connues de l'homme de métier. Les concentrés pour bains ne sont pas appliqués tels quels, mais dilués avec de l'eau et les animaux sont immergés dans une cuve contenant le bain. Les sprays peuvent être appliqués à la main ou à l'aide d'une lance ou d'un arceau de pulvérisation. L'animal, la terre, la plante ou la surface peut être saturé du spray par application en grand volume ou être revêtu superficiellement du spray par application en petit ou ultrapetit volume. Les suspensions aqueuses peuvent être appliquées sur l'animal de la même façon que les sprays et les bains. Les poudres à poudrer peuvent être distribuées au moyen d'un applicateur de poudre ou, dans le cas des animaux, être incorporées à des sacs perforés attachés à des arbres ou à des poteaux. Les pâtes, shampooings et onguents peuvent être appliqués à la main ou répartis sur la surface d'une matière inerte contre laquelle les animaux se frottent et transfèrent ainsi la matière à leur peau. Les lotions sont distribuées sous la forme de dose de liquide de petit volume sur le dos des animaux, de façon que la totalité ou la majeure partie du liquide reste sur les animaux.

Les composés de formule (I) peuvent être présentés à l'état de compositions prêtes à l'usage sur les plantes, les animaux et les surfaces ou à l'état de compositions qui doivent être diluées avant l'application, mais les compositions des deux types comprennent un composé de formule (I) en mélange intime avec un ou plusieurs excipients ou diluants. Les excipients peuvent être liquides, solides ou gazeux ou peuvent comprendre des mélanges de telles substances et le composé de formule (I) peut être présent en une concentration de 99 à 0,025% p/v, suivant le fait que la composition doit ou non être davantage diluée.

Les poudres à poudrer, poudres et granules comprennent le composé de formule (I) en mélange intime avec un excipient inerte solide pulvérulent, par exemple des argiles appropriées, le kaolin, la bentonite, l'attapulgite, le noir de carbone adsorbant, le talc, le mica, la craie, le gypse, le phosphate tricalcique, le liège en poudre, le silicate de magnésium, les excipients végétaux, l'amidon et les terres de diatomées. Ces compositions solides sont généralement préparées en imprégnant les diluants solides avec des solutions du composé de formule (I) dans des solvants volatils, en évaporant les solvants et, si la chose est souhaitée, en broyant les produits pour obtenir des poudres et, si la chose est souhaitée, en granulant, en pressant ou en encapsulant les produits.

Les sprays d'un composé de formule (I) peuvent comprendre une solution dans un solvant organique (par exemple ceux énumérés ci-dessous) ou une émulsion dans de l'eau (bain ou douche) préparée sur le terrain à partir d'un concentré émulsifiable (appelé aussi huile miscible à l'eau), qui peut servir aussi pour l'immersion. Le concentré comprend de préférence un mélange du constituant actif, avec ou sans solvant organique et un ou plusieurs émulsifiants. Les solvants peuvent être présents entre de larges limites, mais de préférence en une quantité de 0 à 90 % p/v de la composition et peuvent être choisis parmi le kérosène, les cétones, les alcools, le xylène, le naphta aromatique et d'autres solvants connus pour la mise en composition. La concentration des émulsifiants peut varier entre de larges limites, mais se situe de préférence dans l'intervalle de 5 à 25 % p/v et les émulsifiants sont avantageusement des tensioactifs non ioniques, notamment des esters polyoxy- alcoyléniques d'alcoylphénols et dérivés polyoxyéthyléniques d'anhydrides d'hexitols, ou bien des tensioactifs anioniques, notamment le laurylsulfate de sodium, des sulfates d'éthers d'alcools gras, des sels de sodium et calcium d'alcoylarylsulfonates et d'alcoylsulfosuccinates.

Des émulsifiants cationiques sont notamment le chlorure de benzalkonium et les éthosulfates d'ammonium quaternaire.

Des émulsifiants amphotères sont notamment l'imidazoline oléique carboxyméthylée et les alcoyl diméthylbétaïnes.

Les mats de vaporisation comprennent normalement un mélange de coton et de cellulose pressé en une plaque d'environ 32 mm sur 22 mm sur 3 mm traitée au moyen d'une quantité atteignant 0,3 ml d'un concentré qui contient le constituant actif dans un solvant organique et facultativement un antioxydant, un colorant et un parfum.

L'insecticide est vaporisé par une source de chaleur, comme un appareil de chauffage électrique pour mats.

Les solides combustibles comprennent normalement de la sciure de bois et un liant en mélange avec le constituant actif et à l'état façonné en rubans (habituellement en hélice). Un colorant et un fongicide peuvent être ajoutés aussi.

Les poudres mouillables comprennent un excipient solide inerte, un ou plusieurs tensioactifs et facultativement des stabilisants et/ou antioxydants.

Les concentrés émulsifiables comprennent des émulsifiants et souvent un solvant organique tel que kérosène, des cétones, des alcools, des xylènes, du naphta aromatique et d'autres solvants connus.

Les poudres mouillables et concentrés émulsifiables contiennent normalement 5 à 95 % en poids du constituant actif et sont dilués avant usage, par exemple avec de l'eau.

Les vernis comprennent une solution du constituant actif dans un solvant organique, conjointement avec une résine et facultativement un plastifiant.

Les bains peuvent être préparés non seulement à partir de concentrés émulsifiables, mais aussi à partir de poudres mouillables, de bains à base de savon et de suspensions aqueuses comprenant un composé de formule (I) en mélange intime avec un dispersant et un ou plusieurs tensioactifs.

Les suspensions aqueuses d'un composé de formule (I) peuvent comprendre une suspension dans de l'eau conjointement avec un agent de mise en suspension, de stabilisation ou autre. Les suspensions ou solutions peuvent être appliquées telles quelles ou sous une forme diluée de façon connue.

Les onguents (ou graisses) peuvent être préparés à partir d'huiles végétales, d'esters synthétiques d'acides gras ou de lanoline, conjointement avec un base inerte telle que la paraffine molle. Un composé de formule (I) est de préférence réparti uniformément dans le mélange en solution ou suspension. Les onguents peuvent aussi être obtenus à partir de concentrés émulsifiables par dilution de ces derniers dans une base d'onguent.

Les pâtes et shampooings sont aussi des compositions semi-solides dans lesquelles un composé de formule (I) peut être présent à l'état de dispersion uniforme dans une base appropriée, comme de la paraffine liquide ou molle, ou bien dans une base non grasse avec du glycérol, de la colle ou un savon convenable. Du fait que les onguents, shampooings et pâtes sont habituellement appliqués sans autre dilution, ils doivent contenir le pourcentage approprié du composé de formule (I) que requiert le traitement.

Les sprays en aérosol peuvent être préparés à l'état de simple solution du constituant actif dans le propulseur d'aérosol et un cosolvant tel qu'un alcane halogéné et les solvants mentionnés ci-dessus, respectivement. Les compositions en lotions peuvent être présentées à l'état de solution ou suspension d'un composé de formule (I) dans un milieu liquide. Un oiseau ou mammifère hôte peut aussi être protégé contre l'infestation par les acariens ectoparasites en portant un produit manufacturé façonné en matière plastique convenablement moulée qui est imprégné d'un composé de formule (I). Ces produits manufacturés comprennent les colliers, marques d'oreilles, bandes, toiles et rubans convenablement attachés sur la partie appropriée du corps. La matière plastique est avantageusement un poly(chlorure de vinyle).

L'invention a donc notamment pour objet une composition comprenant :

a) un composé de formule (I) telle que définie précédemment,
b) des excipients inertes appropriés à l'utilisation comme pesticides dudit produit de formule (I),

une composition comprenant :

a) un composé de formule (I) telle que définie précédemment,
b) des excipients inertes appropriés à l'utilisation dans le domaine vétérinaire dudit produit de formule (I),

et un composé de formule (I) telle que définie précédemment, pour la mise en oeuvre d'une méthode de traitement du corps humain ou animal caractérisé en ce qu'une formulation pharmaceutiquement acceptable dudit composé est appliquée sur ledit corps.

Les composés de formule (I) sont à utiliser dans la protection et le traitement des espèces végétales, auquel cas une quantité insecticide, acaricide, molluscide, nématocide efficace du constituant actif est appliquée. La dose d'application varie avec le composé choisi, la nature de la composition, le mode d'application, l'espèce végétale, la densité de plantation, l'infestation probable et différents autres facteurs, mais en général une dose convenant pour l'agriculture se situe dans l'intervalle de 0,001 à 3 kg par hectare et de préférence entre 0,01 et 1 kg par hectare. Les compositions typiques à usage agricole contiennent entre 0,0001 % et 50 % d'un composé de formule (I) et avantageusement entre 0,1 et 15 % en poids d'un composé de formule (I).

La concentration du composé de formule (I) pour une application sur un animal, des locaux ou des endroits extérieurs varie avec le composé choisi, l'intervalle entre les traitements, la nature de la composition et l'infestation probable, mais en général le composé doit être contenu dans la composition appliquée en quantité de 0,001 à 20,0 % p/v, de préférence 0,01 à 10 % p/v. La quantité du composé déposé sur un animal varie avec le mode d'application, la taille de l'animal, la concentration du composé dans la composition appliquée, le facteur de dilution de la composition et la nature de la composition, mais se situe généralement dans l'intervalle de 0,0001 % à 0,5 % p/p, sauf pour les compositions non diluées, comme les compositions en lotion qui sont généralement déposées à une concentration de l'intervalle de 0,1 à 20,0 % et de préférence de 0,1 à 10 %. La quantité du composé à appliquer sur les produits stockés se situe généralement dans l'intervalle de 0,1 à 20 ppm. Les pulvérisations dans les espaces peuvent être réalisées pour arriver à une concentration initiale moyenne de 0,001 à 1 mg du composé de formule (I) par m3 d'espace traité.

Les onguents, les graisses, les pâtes et les aérosols sont habituellement appliqués au hasard comme décrit

ci-dessus et des concentrations de 0,001 à 20 % p/v d'un composé de formule (I) dans la composition appliquée peuvent être utilisées.

Les composés de formule (I) se sont révélés avoir de l'activité contre la mouche ordinaire (Musca domestica). De surcroît, certains composés de formule (I) ont de l'activité contre d'autres arthropodes nuisibles, notamment Myzus persicae, Tetranychus urticae, Spodoptera littoralis, Heliotuis virescens, Plutella xylostella, Culex spp., Tribolium castaneum, Sitophilus granarius, Periplaneta americana et Blattella germanica. Les composés de formule (I) sont donc utiles pour la lutte contre les arthropodes, par exemple les insectes et acariens, dans tout milieu où ils constituent une nuisance, par exemple en agriculture, en élevage, en santé publique et en milieu domestique.

Des insectes nuisibles sont notamment des membres des ordres de coléoptères (par exemple Anobium, Ceuthorrhynchus, Rhynchophorus, Cosmopolites, Lissorhoptrus, Meligethes, Hypothenemus, Hylesinus, Acalymma, Lema, Psylliodes, Leptinotarsa, Gonocephalum, Agriotes, Dermolepida, Heteronychus, Phaedon, Tribolium, Sitophilus, Diabrotica, Anthonomus ou Anthrenus spp.), des lépidoptères (par exemple Ephestia, Mamestra, Earias, Pectinophora, Ostrinia, Trichloplusia, Pieris, Laphygma, Agrotis, Amathes, Wiseana, Tryporyza, Diatraea, Sparganothis, Cydia, Archips, Plutella, Chilo, Heliothis, Spodoptera littoralis, Helrotuis virescens, Spodoptera ou Tineola spp.), des diptères (par exemple Musca, Aedes, Anopheles, Culex, Glossina, Simulium, Stomoxys, Haematobia, Tabanus, Hydrotaea, Lucilia, Chrysomyia, Callitroga, Dermatobia, Gasterophilus, Hypoderma, Hylemyia, Atherigona, Chlorops, Phytomyza, Ceratitis, Liriomyza et Melophagus spp.), des phtiraptères (Mallophaga, par exemple Damalina spp., et Anoplura, par exemple Linognathus et Haematopinus spp.), des hémiptères (par exemple Aphis, Bemisia, Phorodon, Aeneolamia, Empoasca, Parkinsiella, Pyrilla, Aonidiella, Coccus, Pseudococcus, Helopeltis, Lygus, Dysdercus, Oxycarenus, Nezara, Aleyrodes, Triatoma, Psylla, Myzus, Megoura, Phylloxera, Adelges, Nilaparvata, Nephotettix ou Cimex spp.), des orthoptères (par exemple Locusta, Gryllus, Schistocerca ou Acheta spp.), des dictyoptères (par exemple Blattella, Periplaneta ou Blatta spp.), des hyménoptères (par exemple Athalia, Cephus, Atta, Solenopsis ou Monomorium spp.), des isoptères (par exemple Odontotermes et Reticulitermes spp.), des siphonaptères (par exemple Ctenocephalides ou Pulex spp.), des thysanures (par exemple Lepisma spp.), des dermaptères (par exemple Forficula spp.) et des psocoptères (par exemple Peripsocus spp.) et des thysanoptères (par exemple Thrips tabaci).

Des acariens nuisibles sont notamment les tiques, par exemple les membres des genres Boophilus, Ornithodorus, Rhipicephalus, Amblyomma, Hyalomma, Ixodes, Haemaphysalis, Dermacentor et Anocentor, et les mites et les gales telles que Acarus, Tetranychus, Psoroptes, Notoednes, Sarcoptes, Psorergates, Chorioptes, Eutrombicula, Demodex, Panonychus, Bryobia, Eriophyes, Blaniulus, Polyphagotarsonemus, Scutigerella et Oniscus spp.

Les nématodes qui attaquent les plantes et les arbres importants en agriculture, sylviculture et horticulture, tant directement qu'en propageant des maladies bactériennes, virales, mycoplasmiques ou fongiques des plantes, sont notamment les nématodes des gales dès racines, comme Meloidogyne spp. (par exemple M. incognita); les nématodes des racines, comme Globodera spp. (par exemple G. rostochiensis); Heterodera spp. (par exemple H. avenae); Radopholus spp. (par exemple R. similis); les nématodes des prairies, comme Pratylenchus spp. (par exemple P. pratensis); Belonolaimus spp (par exemple B. gracilis); Tylenchulus spp. (par exemple T. semipenetrans); Rotylenchulus spp. (par exemple R. reniformis); Rotylenchus spp. (par exemple R. robustus); Helicotylenchus spp. (par exemple H. multicinctus); Hemicycliophora spp. (par exemple H. gracilis); Criconemoides spp. (par exemple C. similis); Trichodorus spp. (par exemple T. primitivus); les nématodes xiphophores, comme Xiphinema spp. (par exemple X. diversicaudatum), Longidorus spp. (par exemple L. elongatus); Hoplolaimus spp. (par exemple H. coronatus); Aphelenchoides spp. (par exemple A. ritzemabosi, A. besseyi); et les nématodes des bulbes, comme Ditylenchus spp. (par exemple D dipsaci).

Les composés de l'invention peuvent être combinés avec un ou plusieurs autres constituants pesticides actifs (par exemple des pyréthroïdes, des carbamates et des organophosphates) et/ou avec des attractifs, des répulsifs, des bactéricides, des fongicides, des nématocides, des anthelminthiques et ainsi de suite. De surcroît, il a été observé que l'activité des composés de l'invention peut être accrue par l'addition d'un agent de synergie ou de potentialisation, par exemple un agent de synergie de la classe des inhibiteurs de l'oxydase, comme le pipéronylbutoxyde ou le 2-propynylphénylphosphonate de propyle, par l'addition d'un deuxième composé de l'invention ou d'un pyréthroïde pesticide. Lorsqu'un agent de synergie inhibiteur de l'oxydase est présent dans une composition de l'invention, le rapport de l'agent de synergie au composé de formule (I) se situe dans l'intervalle de 25:1 à 1:25, par exemple à environ 10:1.

Des stabilisants pour empêcher toute dégradation chimique que peuvent subir les composés de l'invention sont notamment, par exemple, des antioxydants (comme les tocophérols, le butylhydroxyanisole, le butylhydroxytoluène), la vitamine C (acide ascorbique) et des capteurs d'oxygène (comme l'épichlorhydrine), de même que des bases organiques et inorganiques, par exemple des trialcoylamines telles que la triéthylamine, qui peuvent agir comme stabilisants basiques et capteurs.

Les composés de la présente invention ont des propriétés pesticides et/ou une photostabilité accrues et/ou une toxicité réduite pour les mammifères.

Les exemples suivants illustrent de façon non limitative des aspects préférés de l'invention.

**<u>Préparation 1</u> : Acide 4-(1-fluoro 2-phényl cyclopropyl) benzoïque, isomère cis racémique (A) + mélange cis racémique + trans racémiques (50/50) (B)**

<u>Stade A</u> : 4-formyl benzoate de tert-butyle

**a) chlorure de l'acide 4-formyl benzoïque**

On mélange à 0°C sous gaz inerte, 15 g d'acide 4-formyl benzoïque commercial, 200 $cm^3$ de dichlorométhane, puis ajoute 9 $cm^3$ de chlorure d'oxalyle commercial et 0,2 $cm^3$ de diméthylformamide, laisse 0,5 heure à 0°C puis 20 heures à température ambiante, amène à sec le chlorure d'acide obtenu.

**b) 4-formyl benzoate de tert-butyle**

On mélange à 6°C sous gaz inerte, 20 $cm^3$ de tert-butanol et 100 $cm^3$ de pyridine, puis introduit une solution du résidu obtenu au stade a) dans 200 $cm^3$ de diméthylformamide, après 1 heure à température ambiante, on chauffe 4 heures au reflux puis laisse revenir à température ambiante, chasse le solvant, reprend le résidu à l'acétate d'éthyle, lave et chromatographie sur silice. On obtient ainsi l'ester tert-butylique avec un rendement de 48,5 %.
<u>CCM</u> : Rf = 0,3 (éluant : heptane-acétate d'éthyle (8-2)).

<u>Stade B</u> : 4-(hydroxyméthyl) benzoate de tert-butyle

5,23 g de 4-formyl benzoate de tert-butyle obtenu selon le stade A sont mélangés à 100 $cm^3$ de tétrahydrofuranne et refroidis à 0°C ; on ajoute ensuite 1 g de borohydrure de sodium, laisse remonter la température à 20-25°C, agite 3 heures, puis hydrolyse avec 100 $cm^3$ d'une solution aqueuse à 10 % de chlorure d'ammonium, extrait à l'acétate d'éthyle, lave, sèche, amène à sec et chromatographie sur silice (éluant heptane - acétate d'éthyle (1-1)).
On isole ainsi 5 g du dérivé hydroxyméthyle attendu.
<u>CCM</u> : Rf = 0,3 (éluant : heptane-acétate d'éthyle (1-1)).
<u>Microanalyse</u> : $C_{12}H_{16}O_3$ = 208,3

| | C % | H % |
|---|---|---|
| Calculés | 69,2 | 7,7 |
| Trouvés | 68,9 | 7,8 |

<u>RMN</u> du proton dans $CDCl_3$ à 250 MHz (en ppm)
1,59 (s) : $CO_2tBu$ ; 2,01 (t) : $\Phi$-$CH_2$-O<u>H</u> ; 4,75 (d) : $\Phi$-C<u>$H_2$</u>-OH 7,40 et 7,96 (AA'BB') : H aromatiques.
<u>IR</u> dans $CHCl_3$ en $cm^{-1}$
3610 : OH ; 1708 : C=O ester ; 1613-1576-1505 : aromatiques.

<u>Stade C</u> : 4-(fluorométhyl) benzoate de tert-butyle

4,5 g de 4-(hydroxyméthyl) benzoate de tert-butyle obtenu selon le stade B sont mélangés à 100 $cm^3$ de dichlorométhane sous gaz inerte et refroidis à 0°C. On ajoute ensuite 3,56 g de trifluorure de diéthylaminosoufre $(C_2H_5)_2NSF_3$ commercial et connu sous l'abréviation DAST, agite 1 heure à 0°+5°C, puis 1 heure à 20-25°C, verse sur une solution aqueuse à 10 % de bicarbonate de sodium, extrait au dichlorométhane, sèche, amène à sec et chromatographie sur silice (éluant chlorure de méthylène). On isole 3 g du dérivé fluorométhylé attendu.
<u>CCM</u> : Rf = 0,37 (éluant : heptane-acétate d'éthyle (8-2)).
<u>Microanalyse</u> pour $C_{12}H_{15}FO_2$ = 210,25

| | C % | H % | F % |
|---|---|---|---|
| Calculés | 68,6 | 7,2 | 9,0 |
| Trouvés | 68,3 | 7,3 | 9,1 |

<u>RMN</u> du proton dans $CDCl_3$ à 250 MHz (en ppm)
1,60 (s) : $CO_2tBu$ ; 5,43 (d,J = 47) : C<u>$H_2$</u>-F ; 7,40 et 8,01 (AA'BB') : H aromatiques.
<u>IR</u> dans $CHCl_3$ en $cm^{-1}$
1712 : C=O ester ; 1618-1582-1512 : aromatiques.

Stade D : 4-(bromofluoro méthyl) benzoate de tert-butyle

2,3 g de 4-(fluorométhyl) benzoate de tert-butyle obtenu selon le stade C, sont mélangés à 30 $cm^3$ de tétrachlorométhane. On y ajoute 0,1 g de 2,2'-azobisisobutyronitrile, commercial et connu sous l'abréviation AIBN, 2 g de N-bromo succinimide, puis porte au reflux et irradie 4 heures, ajoute de nouveau 0,2 g d'AIBN, irradie de nouveau 4 heures, puis laisse au repos 48 heures à température ambiante. Après filtration et chromatographie sur silice, on obtient 1,7 g du dérivé bromofluoré attendu.
Fusion : 39,4°C
CCM : Rf = 0,32 (éluant : heptane-dichlorométhane-tert-butyle méthyle éther (90-5-5)).
Microanalyse pour $C_{12}H_{14}BrFO_2$ = 289,15

| | C % | H % | Br % | F % |
|---|---|---|---|---|
| Calculés | 49,85 | 4,9 | 27,6 | 6,6 |
| Trouvés | 50,0 | 4,9 | 27,4 | 6,9 |

RMN du proton dans $CDCl_3$ à 250 MHz (en ppm)
1,6 (s) : $CO_2$tBu ; 7,43 (d,J = 49,5) : CHBrF ; 7,53-8,04 (AA'BB') : H aromatiques.
IR dans $CHCl_3$ en $cm^{-1}$
1711 : C=O ester ; 1613-1580-1506 : aromatiques.

Stade E : (±) 4-(1-fluoro 2-phényl cyclopropyl) benzoate de tert-butyle (isomère cis)

On mélange sous gaz inerte, 30 $cm^3$ de tétrahydrofuranne, 2,2 g de tert-butylate de potassium et 4 $cm^3$ de styrène commercial puis en 0,5 heures une solution de 3 g du 4-(bromo fluorométhyl) benzoate de tert-butyle, obtenu selon le stade D, dans 30 $cm^3$ de tétrahydrofuranne ; 0,25 h plus tard le mélange est versé sur une solution aqueuse à 10 % de chlorure d'ammonium, le tout est extrait à l'acétate d'éthyle, lavé, séché, amené à sec et chromatographié. On obtient alors 0,25 g du produit attendu.
Fusion : 57,4°C
CCM : Rf = 0,15 (éluant : heptane-toluène (50-50))
Microanalyse pour $C_{20}H_{21}FO_2$ = 312,4

| | C % | H % | F % |
|---|---|---|---|
| Calculés | 76,9 | 6,8 | 6,1 |
| Trouvés | 76,8 | 6,7 | 5,9 |

RMN du proton dans $CDCl_3$ à 250 MHz (en ppm)
1,6 (s) : $CO_2$tBu ; 1,5 et 2,0 (m) $CH_2$ en 3 du cyclopropane ; 2,51 (m) : CH en 2 du cyclopropane (en position trans/F) ; 7,31 (m), 8,0 (dl) : H du phénylène ; 7,31 (m) : H du phényle.
IR dans $CHCl_3$ en $cm^{-1}$
1370 $cm^{-1}$ ; 1707 $cm^{-1}$ : $CO_2$tBu ; 1614, 1606, 1575, 1511, 1500 : Φ.
SM : $M^+ = 312^+$
En opérant de manière analogue, on a aussi obtenu le mélange d'isomères composés suivant :

- **le 4-(1-fluoro 2-phényl cyclopropyl) benzoate de tert-butyle (isomères cis + trans ; cis/trans = 70/30), racémique.**

Stade F : Acide 4-(1-fluoro 2-phényl cyclopropyl) benzoïque (isomères cis + trans ; cis/trans = 70/30), racémiques

1 g de 4-(1-fluoro 2-phényl cyclopropyl) benzoate de tert-butyle cis/trans = 70/30 racémique obtenu selon le stade E est mélangé à 80 $cm^3$ de toluène ; on y ajoute 0,09 mg du monohydrate de l'acide para-toluènesulfonique, on chauffe 6 heures à 100°C puis laisse au repos une nuit. Après lavage, extraction à l'acétate d'éthyle, séchage et évaporation à sec, on obtient 1 g de solide blanc (isomère cis/trans = 70/30, racémique).

Stade G : Acide 4-(1-fluoro 2-phényl cyclopropyl) benzoïque, isomère cis racémique (A) + mélange cis racémique + trans racémiques (50/50) (B)

Le gramme du solide blanc obtenu ci-dessus est recristallisé dans l'éther isopropylique. On obtient 0,39 g de l'isomère cis pur racémique (A) qui précipite et 0,5 g du mélange cis/trans (50/50) (B) après avoir amené à sec les liqueurs mères.
Analyse de l'isomère (A)
Fusion : 169,2°C
Microanalyse pour $C_{16}H_{13}FO_2$ = 256,3

| | C % | H % | F % |
|---|---|---|---|
| Calculés | 75,0 | 5,1 | 7,4 |
| Trouvés | 74,7 | 5,0 | 7,3 |

RMN du proton dans $CDCl_3$ à 250 MHz (en ppm)
1,83 (m) et 2,04 (m) : C$\underline{H}_2$ en 3 du cyclopropane ; 2,57 (m) : C$\underline{H}$ en 2 du cyclopropane ; 7,39 et 8,14 (AA',BB') : $\underline{H}$ du groupe phénylène ; 7,35 à 7,4 (m) : H du groupe phényle.
IR dans $CHCl_3$ en $cm^{-1}$
1725 et 1694 : C=O ; 1615, 1575, 1512 et 1500 : Φ.
SM : $M^+ = 312^+$

**EXEMPLE 1 : 4-(1-fluoro 2-phényl cyclopropyl) N-isobutyl benzènamide (isomères cis+trans, cis/trans = 3/2)**

Stade A : préparation du chlorure de l'acide 4-(1-fluoro 2-phényl cyclopropyl) benzoïque

On mélange sous gaz inerte 0,35 g de l'acide 4-(1-fluoro 2-phényl cyclopropyl) benzoïque (mélange (B)), obtenu selon la préparation 1, et 20 $cm^3$ de dichlorométhane, on refroidit à 0°C, on ajoute au mélange 0,207 g de chlorure d'oxalyle commercial et 0,01 $cm^3$ de diméthylformamide, agite 3 heures à 20-25°C et amène à sec pour recueillir le chlorure d'acide.

Stade B : Amidification

On mélange sous gaz inerte, 10 $cm^3$ de pyridine et 0,2 $cm^3$ d'isobutylamine commercial, refroidit à 0°C puis ajoute une solution du chlorure d'acide obtenu au stade A dans 4 $cm^3$ de diméthylformamide ; on agite 20 heures à 20-25°C, amène à sec, purifie et recristallise dans l'éther isopropylique pour isoler 0,3 g de l'amide attendu.
Fusion : 100°C
CCM : Rf = 0,2-0,25 (éluant : heptane-dichlorométhane-tert-butyl méthyl éther (5-4-1)).
Microanalyse pour $C_{20}H_{22}FNO$ = 311,14

| | C % | H % | F % | N % |
|---|---|---|---|---|
| Calculés | 77,1 | 7,1 | 6,1 | 4,5 |
| Trouvés | 77,0 | 7,1 | 6,2 | 4,5 |

RMN du proton dans $CDCl_3$ (en ppm)
mélange d'isomères cis (A) et trans (B)

(A)
0,95 et 0,99 (d) : C$\underline{H}_3$ de l'isobutyle ; 1,68 à 2,10 : C$\underline{H}$ de l'isobutyle et C$\underline{H}_2$ en 3 du cyclopropyle ; 2,51 (m) : C$\underline{H}$ en 2 du cyclopropyle ; 3,23 : C$\underline{H}_2$ de l'isobutyle ; 6,07 : N$\underline{H}$ ; 6,95 à 7,36 : $\underline{H}$ du groupe phényle ; 7,58 (d) et 7,79 (d) : $\underline{H}$ du groupe phénylène.
(B)
3,04 (d,d,d, J = 8,5-11,5 et 20) : C$\underline{H}$ en 2 du cyclopropyle ; 3,30 : C$\underline{H}_2$ de l'isobutyle ; 6,18 : N$\underline{H}$.

IR dans $CHCl_3$ en $cm^{-1}$
3465 : =C-NH ; 1656 : C=O ; 1616, 1572, 1530 et 1502 : Φ.
SM : $M^+$=$311^+$

**<u>EXEMPLE 2</u> : 4-(1-fluoro 2-phényl cyclopropyl) N-(2-méthyl phényl) benzènamide (isomères cis+trans, cis/trans = 3/1)**

<u>Stade A</u> : Préparation du chlorure d'acide intermédiaire

On mélange sous gaz inerte, 0,15 g du mélange d'isomères (B) et 0,20 g de l'isomère cis (A) de l'acide 4-(1-fluoro 2-phényl cyclopropyl) benzoïque, obtenus selon la préparation 1, ainsi que 20 $cm^3$ de dichlorométhane, refroidit à 0°C, puis on ajoute au mélange 0,15 $cm^3$ de chlorure d'oxalyle et 0,01 $cm^3$ de diméthylformamide, agite 3 heures à température ambiante et amène à sec sous vide pour recueillir le chlorure d'acide.

<u>Stade B</u> : Amidification

On mélange sous gaz inerte 10 $cm^3$ de pyridine, 0,15 $cm^3$ d'ortho-toluidine commerciale et refroidit à 0°C et ajoute une solution du chlorure d'acide obtenu au stade A dans 6 $cm^3$ de diméthylformamide ; on agite 24 heures à température ambiante, amène à sec et chromatographie sur silice pour obtenir 0,43 g de l'amide attendu.
<u>Fusion</u> : 138,2°C
<u>CCM</u> : Rf = 0,3-0,35 (éluant : heptane-dichlorométhane-tert-butyl méthyl éther (5-4-1)).
<u>Microanalyse</u> pour $C_{23}H_{20}FNO$ = 345,4

| | C % | H % | F % | N % |
|---|---|---|---|---|
| Calculés | 80,0 | 5,8 | 5,5 | 4,1 |
| Trouvés | 79,9 | 5,9 | 5,4 | 4,0 |

<u>RMN</u> du proton dans $CDCl_3$ à 250 MHz (en ppm)
mélange d'isomères cis (A) 75 % et trans (B) 25 %
Pour les 2 isomères 1,70 à 1,87 et 1,93 à 2,10 : C<u>H</u>$_2$ en 3 du cyclopropane, 6,95 à 8,00 : <u>H</u> aromatiques et N<u>H</u>.
pour (A) :
2,35 (s) : C<u>H</u>$_3$ du Φ-$CH_3$ ; 2,55 (m) : C<u>H</u> en 2 du cyclopropane, pour (B) :
2,30 (s) : C<u>H</u>$_3$ du Φ-$CH_3$ ; 3,08 (d,d,d ; J=8, 5, 11 et 20) : C<u>H</u> en 2 du cyclopropane,
<u>IR</u> dans $CHCl_3$ en $cm^{-1}$
3440 : =C-NH ; 1677 : $\rangle$C=O ; 1614, 1605, 1589, 1571, 1526, 1510 et 1500 : Φ.
<u>SM</u> : $MH^+$=$346^+$

**<u>Préparation 3</u> : acide 4-[2-(4-chloro phényl) cyclopropyl] benzoïque (isomère trans)**

<u>Stade A</u> : 3-(4-bromo phényl) 1-(4-chloro phényl) propénone

On chauffe à 35°C, un mélange préparé à partir de 15,46 g de 4-chloroacétophénone commercial, 18,5 g de 4-bromo benzaldéhyde et 75 $cm^3$ d'éthanol à 100 %, puis après 0,25 h ajoute une solution de 7,41 g d'hydroxyde de sodium dans 37 $cm^3$ d'eau, et agite une nuit. On filtre, rince et obtient 33 g du produit attendu.
<u>CCM</u> : Rf = 0,65 (éluant : hexane-acétate d'éthyle (1-1))

<u>Stade B</u> : 5-(4-bromo phényl) 3-(4-chloro phényl) 4,5-dihydro pyrazole

On mélange 25 g de la 3-(4-bromo phényl) 1-(4-chloro phényl) propènone, obtenu selon le stade A, 50 $cm^3$ d'hydrate d'hydrazine, 8 $cm^3$ d'eau, porte au reflux pendant 1,5 heures puis refroidit à température ambiante, ajoute 200 $cm^3$ d'eau et lave le précipité obtenu.

<u>Stade C</u> : 1-(4-bromo phényl) 2-(4-chloro phényl) cyclopropane (isomère trans)

Au produit brut obtenu selon le stade B, on ajoute 50 $cm^3$ de diéthylène glycol et 2,72 g d'hydroxyde de potassium, chauffe à 240°C pendant 1,5 h, puis refroidit et verse dans l'eau, extrait à l'éther diéthylique, sèche et amène à sec et chromatographie, pour obtenir après recristallisation 7,5 g du produit attendu.

<u>Stade D</u> : 4-[2-(4-chloro phényl) cyclopropyl] benzonitrile

On mélange 2,0 g du dérivé bromé obtenu selon le stade C, 15 $cm^3$ de N-méthyl pyrrolidine commercial et

1,17 g de chlorure cuivreux, porte au reflux pendant 24 heures, refroidit à 20°C, extrait à l'éther éthylique, sèche, amène à sec et chromatographie sur silice. On récupère 0,8 g du produit attendu.
CCM : Rf = 0,53 (éluant : éther éthylique-hexane (1-1))
SM : $M^+=254^+$

Stade E : Acide 4-[2-(4-chloro phényl) cyclopropyl] benzoïque (isomère trans)

On mélange 0,4 g du dérivé cyané obtenu selon le stade D, 7,5 $cm^3$ d'éthanol et 7,5 $cm^3$ d'une solution aqueuse 10M d'hydroxyde de sodium, porte au reflux pendant 12 heures verse dans l'eau, lave, acidifie à l'acide chlorhydrique, extrait à l'éther éthylique, sèche, amène à sec et obtient 0,3 g de l'acide attendu.
CCM : Rf = 0,37 (éluant : éther éthylique)

**EXEMPLE 3 : 4-[2-(4-chloro phényl) cyclopropyl] N-isobutyl benzènamide (isomère trans)**

On mélange 0,3 g du produit obtenu selon la préparation 3, avec 5 $cm^3$ de dichlorométhane, puis on ajoute 0,1 $cm^3$ de chlorure d'oxalyle, une goutte de diméthylformamide, agite 2 heures, amène à sec, ajoute 5 $cm^3$ de dichlorométhane, refroidit à -10°C et ajoute 0,1 $cm^3$ de pyridine, 0,1 $cm^3$ d'isobutylamine, 5 mg de diméthylaminopyridine et 2 $cm^3$ de dichlorométhane, et agite 1 heure à température ambiante. On verse ensuite dans l'eau, lave, sèche et filtre puis amène à sec et purifie.
On obtient 0,23 g du produit attendu.
Fusion : 136-138°C
CCM : Rf = 0,46 (éluant : éther éthylique).
RMN du proton dans $CDCl_3$ à 250 MHz
0,96 (d) : $C\underline{H}_3$ de l'isobutyle ; 1,52 (m) et 2,16 (m) : $C\underline{H}$ en 1 et 2 et $C\underline{H}_2$ en 3 du cyclopropyle ; 1,80-2,08 (m) : $C\underline{H}$ de l'isobutyle ; 3,24 (t) : $C\underline{H}_2$ de l'isobutyle ; 6,15 : $N\underline{H}$ ; 7,06 (d), 7,18 (d), 7,25 (d), 7,71 (d) : $\underline{H}$ aromatiques.
SM : $M^+=328^+$

**Préparation 4 : acide 4-[2-(4-chloro phényl) 1-fluoro cyclopropyl] benzoïque (isomère cis)**

Stade A : 4-[2-(4-chloro phényl) 1-fluoro cyclopropyl] benzoate de tert-butyle (isomères cis + trans ; cis/trans = 37/63), racémique,

En opérant comme au stade E de la préparation 1, en utilisant le 4-chloro styrène commercial, on obtient le produit recherché.
Fusion : 110°C produit cis
CCM :
produit cis 0,5-0,27 (éluant : hexane-toluène (50-50)) produit trans 0,27-0,30 (éluant : hexane-toluène (50-50))
RMN du proton à 250 MHz (en ppm)
produit cis 2,4-2,45 : $C\underline{H}$ en position 2 du cyclopropane
produit trans 3,0-3,05 : $C\underline{H}$ en position 2 du cyclopropane

Stade B : acide 4-[2-(4-chloro phényl) 1-fluoro cyclopropyl] benzoïque (isomère cis)

En opérant de manière analogue à partir de l'isomère cis du 4-[2-(4-chloro phényl) 1-fluoro cyclopropyl] benzoate de tert-butyle, on obtient le produit recherché avec un rendement de 80 %.
Fusion : 204°C.

**EXEMPLE 4 : 4-[2-(4-chloro phényl) 1-fluoro cyclopropyl] N-éthyl N-(2-méthyl phényl) benzènamide (isomère cis)**

En opérant comme à l'exemple 1, à partir de l'acide préparé selon la préparation 4 et de N-éthyl ortho-toluidine commercial, on obtient le produit recherché.
Fusion : 133°C
RMN du proton dans $CDCl_3$ à 250 MHz (en ppm)
1,20-1,23-1,26 : $N-CH_2-C\underline{H}_3$ ; 1,68 à 1,86 : $C\underline{H}_2$ en 3 du cyclopropyle ; 2,18 (s) : $C\underline{H}_3$ du $\Phi-CH_3$ ; 2,30-2,32-2,34 : $C\underline{H}$ en 2 du cyclopropyle ; 3,62 à 3,69 et 4,10 à 4,20 : $N-C\underline{H}_2-CH_3$ ; 7,01 à 7,51 : 12H aromatiques.
SM : $MH^+=408^+$

**<u>EXEMPLE 5</u> : 4-[2-(4-chloro phényl) 1-fluoro cyclopropyl] N-isobutyl benzènamide (isomère cis)**

En opérant comme à l'exemple 1, à partir de l'acide préparé selon la préparation 4 et de N-isobutyl amine commercial, on obtient le produit recherché.
<u>Fusion</u> : 140°C
<u>RMN</u> du proton dans $CDCl_3$ à 250 MHz (en ppm)
0,99 (d) : $C\underline{H}_3$ de l'isobutyle ; 1,70 à 1,98 (m) : $C\underline{H}_2$ en 3 du cyclopropyle et CH de l'isobutyle ; 2,47 (m) : $C\underline{H}$ en 2 du cyclopropyle (trans/F) ; 3,30 (m) : $C\underline{H}_2$ de l'isobutyle ; 6,202 (tl) : $N\underline{H}$ ; 7,20 à 7,35 : 6H aromatiques ; 7,80 (dl) : 2H aromatiques.

**<u>EXEMPLE 6</u> : 4-[2-(4-chloro phényl) 1-fluoro cyclopropyl] N-(2-méthyl phényl) benzènamide (isomère cis)**

En opérant comme à l'exemple 1, à partir de l'acide préparé selon la préparation 4 et d'ortho-toluidine commercial, on obtient le produit recherché.
<u>Fusion</u> : 172°C
<u>CCM</u> : Rf = 0,14 (éluant : hexane-acétate d'éthyle (8-2)).
<u>Microanalyse</u> pour $C_{23}H_{19}ClFNO$ = 379,866

| | C % | H % | F % | Cl % | N % |
|---|---|---|---|---|---|
| Calculés | 72,73 | 5,04 | 5,0 | 9,3 | 3,69 |
| Trouvés | 72,5 | 4,9 | 4,8 | 9,5 | 3,6 |

<u>RMN</u> du proton dans $CDCl_3$ à 250 MHz (en ppm)
1,803 à 2,025 (2H) : $C\underline{H}_2$ en 3 du cyclopropyle ; 2,35 (s) et 2,45 0 2,57 (m) (4H) : $\Phi$-$C\underline{H}_3$ et $C\underline{H}$ en 2 du cyclopropyle (trans/F) ; 7,13 à 7,98 (13H) : <u>H</u> aromatiques et $N\underline{H}$
<u>IR</u> dans $CHCl_3$ en $cm^{-1}$
3444 : =C-NH ; 1677 : O=C< ; 1615, 1589, 1570, 1526, 1510 et 1496 : $\Phi$ + amide secondaire.

**<u>Préparation 7</u> : acide 4-[2-(3,4-dibromo phényl) 1-fluoro cyclopropyl] benzoïque (isomère cis)**

<u>Stade A</u> : 4-[2-(3,4-dibromo phényl) 1-fluoro cyclopropyl] benzoate de tert-butyle (isomères cis)

En opérant comme au stade E de la préparation 1, en utilisant le 3,4-bromo styrène, on obtient le produit recherché.
<u>RMN</u> du proton
1,60 (s) : $CO_2tBu$
7,28 à 8,03 : 7H aromatiques

<u>Stade B</u> : acide 4-[2-(3,4-dibromo phényl) 1-fluoro cyclopropyl] benzoïque (isomère cis)

En opérant de la même manière qu'au stade F de la préparation 1, on obtient l'acide recherché.

**<u>EXEMPLE 7</u> : 4-[2-(3,4-dibromo phényl) 1-fluoro cyclopropyl] N-(2-méthyl phényl) benzènamide (isomère cis) et ses isomères optiquement actifs.**

En opérant comme à l'exemple 1, à partir de l'acide préparé selon la préparation 7 et d'ortho-toluidine commercial, on obtient le produit recherché.
<u>Fusion</u> : 158°C
<u>RMN</u> du proton dans $CDCl_3$ à 250 MHz (en ppm)
1,77 à 2,10 : $C\underline{H}_2$ en 3 du cyclopropyle ; 2,35 (s) : $C\underline{H}_3$ du $\Phi$-$CH_3$ ; 2,46 (d,d,d) : $C\underline{H}$ en 2 du cyclopropyle (trans/F) ; 7,63 (s) : $N\underline{H}$ ; 7,12 à 7,92 : 11H aromatiques.

- Séparation des isomères optiquement actifs.

2 g de produit obtenu à l'exemple 7 sont injectés par lots de 500 mg sur 260 g de phase CHIRALPAK AD®, 20 $\mu$ avec le mélange éluant heptane-éthanol-méthanol 50-30-20 et à un débit de 100 ml/mn. On récupère après distillation des bonnes fractions, l'énantiomère A dextrogyre 830 mg (pureté 99%) et l'énantiomère B le vogyre 850

mg (pureté 96,6%). L'énantiomère B est réinjecté par lots de 425 mg dans les mêmes conditions pour aboutir à 780 mg (pureté 99,4%).

Contrôles :

Les puretés sont déterminées par HPLC analytique sur une colonne chiralpak AD® 25 cm x 0,46 cm à un débit de 1ml/mn avec le même mélange éluant. La détection se fait à 254 nm et les facteurs de capacité sont respectivement de 1,44 et 2,35 pour les énantiomères dextrogyre et lévogyre.
Enantiomère A : αD (0,6% dans $CHCl_3$) = +261°.
Enantiomère B : αD (0,6% dans $CHCl_3$) = -257°.

**EXEMPLE 8 : 4-[2-(3,4-dibromo phényl) 1-fluoro cyclopropyl] N-isobutyl benzènamide (isomère cis)**

En opérant comme à l'exemple 1, à partir de l'acide préparé selon la préparation 7 et de N-isobutyl amine commercial, on obtient le produit recherché.
Fusion : 132°C
RMN du proton dans $CDCl_3$ à 250 MHz (en ppm)
0,97 (d), 1,00 (d) : $C\underline{H}_3$ de l'isobutyle ; 1,63 à 1,98 (3H) : $C\underline{H}_2$ en 3 du cyclopropyle et C<u>H</u> de l'isobutyle ; 2,45 (1H) : C<u>H</u> en 2 du cyclopropyle (trans/F) ; 3,25 (t) (2H) : $C\underline{H}_2$ de l'isobutyle ; 6,05 (f) : N<u>H</u> ; 7,06 à 7,82 (7H) : H aromatiques.

A partir des isomères trans des acides préparés selon les préparations 1 à 7, on obtient les composés suivants :

**EXEMPLE 9 : 4-[2-(4-chloro phényl) 1-fluoro cyclopropyl] N-isobutyl benzènamide (isomère trans)**

Fusion : 157°C
RMN du proton dans $CDCl_3$ à 250 MHz (en ppm)
0,96 (d) : $C\underline{H}_3$ de l'isobutyle ; 1,65 à 2,08 (m) : $C\underline{H}_2$ en 3 du cyclopropyle et C<u>H</u> de l'isobutyle ; 2,99 (d,d,d J=8,5, 11,5 et 20) : C<u>H</u> en 2 du cyclopropyle (cis/F) ; 3,24 (t) : $C\underline{H}_2$ de l'isobutyle ; 6,13 (tl) : N<u>H</u> ; 6,88 (d) et 7,11 (d) : <u>H</u> du 4-chloro phényle ; 7,16 (d) et 7,61 (d) : <u>H</u> du para-phénylène.

**EXEMPLE 10 : 4-[2-(4-chloro phényl) 1-fluoro cyclopropyl] N-(2-méthyl phényl) benzènamide (isomère trans)**

CCM : Rf = 0,16 (éluant : hexane-acétate d'éthyle (8-2)). IR dans $CHCl_3$ en $cm^{-1}$
3450 : C-NH ; 1676 : O=C ; 1615, 1589, 1572, 1526, 1510 et 1495 : Φ + amide secondaire.

En mettant en oeuvre l'une des méthodes exposées aux pages précédentes, on a préparé les produits suivants.

Dans les tableaux suivants :

- l'astérique devant le numéro de l'exemple indique que l'on a isolé l'isomère cis pur et le double astérique l'isomère trans,
- TfO représente le radical trifluorométhyl sulfonyloxy,
- $Z_2$ représente le radical 2-(pyrrolydin-1-yl) phényle,
- THP signifie tétrahydropyranyloxy.

## TABLEAU A

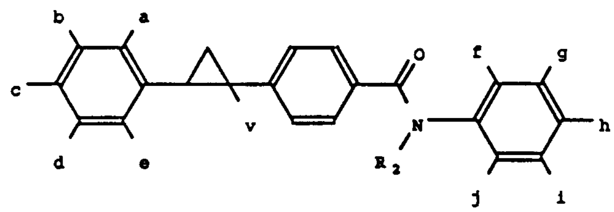

| Ex N°: | a | b | c | d | e | v | f | g | h | i | j | $R_2$ |
|---|---|---|---|---|---|---|---|---|---|---|---|---|
| 2 | H | H | H | H | H | F | Me | H | H | H | H | H |
| 4* | H | H | Cl | H | H | F | Me | H | H | H | H | Et |
| 6* | H | H | Cl | H | H | F | Me | H | H | H | H | H |
| 7* | H | Br | Br | H | H | F | Me | H | H | H | H | H |
| 7A | H | Br | Br | H | H | F | Me | H | H | H | H | H |
| 7B | H | Br | Br | H | H | F | Me | H | H | H | H | H |
| 10** | H | H | Cl | H | H | F | Me | H | H | H | H | H |
| 12* | H | Cl | Cl | H | H | F | Me | H | H | H | H | H |
| 13* | H | Cl | Cl | H | H | F | Me | H | H | H | H | Et |
| 14** | H | Cl | Cl | H | H | H | Me | H | H | H | H | H |
| 15* | H | Br | Br | H | H | F | Me | H | H | H | H | Et |
| 16* | H | H | MeO | H | H | F | Me | H | H | H | H | Et |
| 17* | H | Cl | Cl | Cl | H | F | Me | H | H | H | H | H |
| 18* | H | $CF_3$ | Br | H | H | F | Me | H | H | H | H | H |
| 19* | Cl | H | Cl | H | H | F | Me | H | H | H | H | H |
| 20 | Cl | H | Cl | H | H | F | Me | H | H | H | H | H |
| 21** | Cl | H | Cl | H | H | F | Me | H | H | H | H | H |
| 22* | H | H | Br | H | H | F | Me | H | H | H | H | H |
| 23* | H | Br | H | H | H | F | Me | H | H | H | H | H |
| 24* | Cl | H | H | H | H | F | Me | H | H | H | H | H |
| 25** | Cl | H | H | H | H | F | Me | H | H | H | H | H |
| 26* | H | PhO | H | H | H | F | Me | H | H | H | H | H |
| 27* | H | PhO | H | H | H | F | Me | H | H | H | H | Et |
| 29* | H | H | Me | H | H | F | Me | H | H | H | H | H |

TABLEAU A (suite)

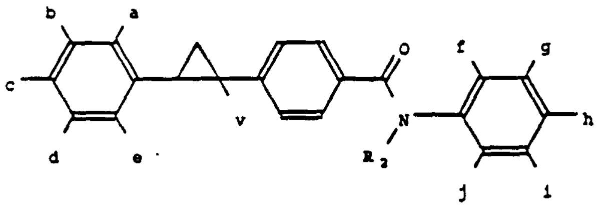

| Ex N°: | a | b | c | d | e | v | f | g | h | i | j | $R_2$ |
|---|---|---|---|---|---|---|---|---|---|---|---|---|
| 31* | Cl | H | H | H | H | F | Me | H | H | H | H | Et |
| 32* | H | $CF_3$ | Br | H | H | F | Me | H | H | H | H | Et |
| 33* | H | H | $CF_3$ | H | H | F | Me | H | H | H | H | H |
| 34** | H | H | $CF_3$ | H | H | F | Me | H | H | H | H | H |
| 35* | Cl | Cl | H | H | H | F | Me | H | H | H | H | H |
| 36* | Cl | Cl | H | H | H | F | Me | H | H | H | H | Et |
| 37** | H | Br | Br | H | H | F | Me | H | H | H | H | Et |
| 38* | H | H | $CF_3$ | H | H | F | Me | H | H | H | H | Et |
| 39** | H | H | $CF_3$ | H | H | F | Me | H | H | H | H | Et |
| 40* | H | TfO | TfO | H | H | F | Me | H | H | H | H | H |
| 41** | H | Br | Br | H | H | F | Me | H | H | H | H | H |
| 42** | H | H | F | H | H | F | Me | H | H | H | H | H |
| 43* | H | H | F | H | H | F | Me | H | H | H | H | H |
| 44* | H | Br | Br | H | H | F | Me | H | H | H | H | Pr |
| 45* | H | Br | Br | H | H | F | Me | H | H | H | H | Me |
| 46* | Cl | H | H | H | Cl | F | Me | H | H | H | H | H |
| 47* | H | Br | Br | H | H | F | Me | H | H | H | H | Bn |
| 48* | H | Br | Br | H | H | F | Me | H | H | H | H | Bu |
| 49* | H | H | $CF_3O$ | H | H | F | Me | H | H | H | H | H |
| 50* | H | H | $CHF_2O$ | H | H | F | Me | H | H | H | H | H |
| 51* | H | PhO | F | H | H | F | Me | H | H | H | H | H |
| 52* | H | H | $NO_2$ | H | H | F | Me | H | H | H | H | H |
| 53* | H | F | F | H | H | F | Me | H | H | H | Me | H |

TABLEAU A (suite)

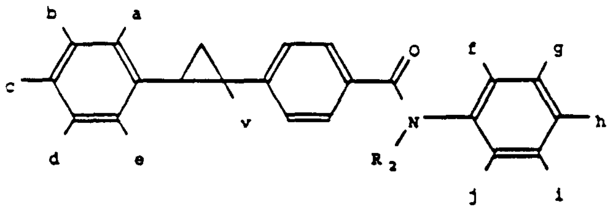

| Ex N°: | a | b | c | d | e | v | f | g | h | i | j | $R_2$ |
|---|---|---|---|---|---|---|---|---|---|---|---|---|
| 56* | H | Br | Br | H | H | F | H | H | tBu | H | H | H |
| 57* | H | Br | Br | H | H | F | $CH_2=C(CH_3)$ | H | H | H | H | H |
| 58* | H | Br | Br | H | H | F | MeS | H | H | H | H | H |
| 59* | H | Br | Br | H | H | F | MeO | H | H | H | H | H |
| 60* | H | Br | Br | H | H | F | H | H | cyclohexyl | H | H | H |
| 61* | H | Br | Br | H | H | F | Ph | H | H | H | H | H |
| 62* | H | Br | Br | H | H | F | H | Br | H | H | H | H |
| 63* | H | Br | Br | H | H | F | Cl | H | H | H | H | H |
| 64* | H | Br | Br | H | H | F | H | H | H | H | H | H |
| 65* | H | Br | Br | H | H | F | Et | H | H | H | H | H |
| 66* | H | Br | Br | H | H | F | N≡C | H | H | H | H | H |
| 67* | H | Br | Br | H | H | F | $CF_3O$ | H | H | H | H | H |
| 68* | H | Br | Br | H | H | F | PhO | H | H | H | H | H |
| 69* | H | Br | Br | H | H | F | Br | H | H | H | H | H |
| 70* | H | Br | Br | H | H | F | H | Cl | MeO | Cl | H | H |
| 71* | H | Br | Br | H | H | F | MeO | H | Cl | Cl | H | H |
| 72* | H | Br | Br | H | H | F | H | H | F | H | H | H |
| 73* | H | Br | Br | H | H | F | H | F | H | H | H | H |
| 74* | H | Br | Br | H | H | F | H | Cl | Br | Cl | H | H |
| 75* | H | Br | Br | H | H | F | $NO_2$ | H | MeO | H | H | H |
| 76* | H | Br | Br | H | H | F | H | Cl | Cl | H | H | H |
| 77* | H | Br | Br | H | H | F | MeO | H | H | Me | H | H |
| 78* | H | Br | Br | H | H | F | H | MeO | H | MeO | H | H |

TABLEAU A (suite)

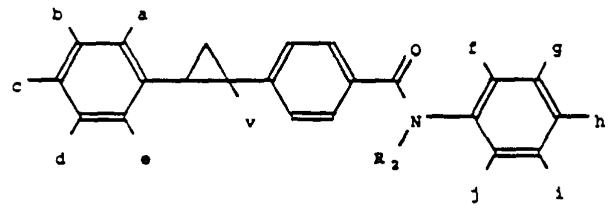

| Ex N°: | a | b | c | d | e | v | f | g | h | i | j | $R_2$ |
|---|---|---|---|---|---|---|---|---|---|---|---|---|
| 80* | H | Br | Br | H | H | F | H | MeO | MeO | H | H | H |
| 81* | H | Br | Br | H | H | F | Me | H | H | H | Me | Me |
| 82* | H | Br | Br | H | H | F | Me | H | MeO | H | H | H |
| 83* | H | Br | Br | H | H | F | F | H | H | H | F | H |
| 84* | H | Br | Br | H | H | F | H | H | $CO_2Me$ | H | H | H |
| 85* | H | Br | Br | H | H | F | H | H | BnO | H | H | H |
| 86* | H | Br | Br | H | H | F | Me | H | H | H | Me | H |
| 87* | H | Br | Br | H | H | F | Cl | H | H | H | Cl | H |
| 88* | H | Br | Br | H | H | F | MeO | H | H | $CF_3$ | H | H |
| 89* | H | Br | Br | H | H | F | Me | Me | H | H | H | H |
| 90* | H | Br | Br | H | H | F | Me | H | Me | H | H | H |
| 91* | H | Br | Br | H | H | F | H | MeO | H | $CF_3$ | H | H |
| 92* | H | Br | Br | H | H | F | Cl | H | H | Me | H | H |
| 93* | H | Br | Br | H | H | F | H | H | MeO | H | H | H |
| 94 | H | Br | Br | H | H | F | H | H | PhO | H | H | H |
| 95* | H | Br | Br | H | H | F | F | H | Me | H | H | H |
| 96* | H | Br | Br | H | H | F | H | Cl | Cl | H | H | Me |
| 97* | H | Br | Br | H | H | F | MeO | H | H | MeO | H | H |
| 98* | H | Br | Br | H | H | F | Me | Cl | H | H | H | H |
| 99* | H | Br | Br | H | H | F | H | PhO | H | H | H | H |
| 100* | H | Br | Br | H | H | F | MeO | H | H | $NO_2$ | H | H |
| 101* | H | Br | Br | H | H | F | H | Br | Br | H | H | H |
| 102* | H | Br | Br | H | H | F | PhCO | H | H | H | H | H |
| 103* | H | Br | Br | H | H | F | Cl | H | H | Cl | H | H |

TABLEAU A (suite)

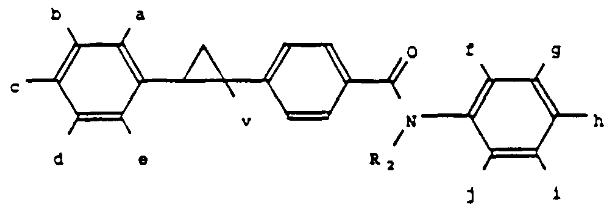

| Ex N°: | a | b | c | d | e | v | f | g | h | i | j | $R_2$ |
|---|---|---|---|---|---|---|---|---|---|---|---|---|
| 104* | H | Br | Br | H | H | F | H | H | $NCCH_2$ | H | H | H |
| 105* | H | Br | Br | H | H | F | Me | H | Me | H | Me | H |
| 106* | H | Br | Br | H | H | F | Cl | H | Cl | H | H | H |
| 107* | H | Br | Br | H | H | F | H | Cl | H | Cl | H | H |
| 108* | H | Br | Br | H | H | F | H | Me | H | Me | H | H |
| 109* | H | Br | Br | H | H | F | Me | H | H | Cl | H | H |
| 110* | H | Br | Br | H | H | F | $z_2$ | H | H | H | H | H |
| 111* | H | Br | Br | H | H | F | $CO_2Me$ | H | H | Cl | H | H |
| 112* | H | Br | Br | H | H | F | tBu | H | H | H | H | H |
| 113** | H | Cl | Cl | H | H | H | Me | H | H | H | H | H |
| 114** | H | Cl | Cl | H | H | H | Me | H | H | H | H | Et |
| 115* | H | Cl | Cl | H | H | H | Me | H | H | H | H | H |
| 116 | H | H | Br | H | H | H | Me | H | H | H | H | Et |
| 117** | H | Br | Br | H | H | H | Me | H | H | H | H | H |
| 118** | H | Br | Br | H | H | H | Me | H | H | H | Me | H |
| 119** | H | Cl | Cl | H | H | H | Me | H | H | H | Me | H |
| 120* | H | Cl | Cl | H | H | Br | Me | H | H | H | H | H |
| 121* | H | Cl | Cl | H | H | Cl | Me | H | H | H | H | H |
| 268* | H | Br | Br | H | H | F | $CHF_2O$ | H | H | H | H | H |
| 269* | H | F | F | H | H | F | Me | H | H | H | H | H |
| 270* | H | Br | TfO | Br | H | F | Me | H | H | H | H | H |
| 271* | H | Br | TfO | H | H | F | Me | H | H | H | H | H |
| 272* | H | Br | TfO | H | H | F | Me | H | H | H | Me | H |

TABLEAU A (suite)

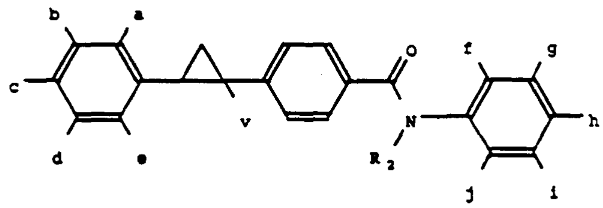

| Ex N°: | a | b | c | d | e | v | f | g | h | i | j | $R_2$ |
|---|---|---|---|---|---|---|---|---|---|---|---|---|
| 274* | H | Br | $CHF_2O$ | Br | H | F | Me | H | H | H | Me | H |
| 275* | H | Br | $CHF_2O$ | Br | H | F | Me | H | H | H | H | H |
| 276* | H | Br | Br | H | H | F | Cl | H | Me | H | H | H |
| 282* | H | Br | Br | H | H | F | OH | H | H | H | H | H |
| 283* | H | Cl | Cl | H | H | F | $CH_2OH$ | H | H | H | H | H |
| 284* | H | Cl | Cl | H | H | F | $CH_2F$ | H | H | H | H | H |
| 285* | H | Cl | Cl | H | H | F | CHO | H | H | H | H | H |
| 286* | H | Cl | Cl | H | H | F | Me | H | H | H | Me | H |
| 287* | H | Cl | Cl | H | H | F | Me | H | Me | H | H | H |
| 288* | H | Cl | Cl | H | H | F | F | H | H | H | H | H |
| 289* | H | Cl | Cl | H | H | F | Cl | H | Me | H | H | H |
| 290* | H | Cl | Cl | H | H | F | CN | H | H | H | H | H |
| 291* | H | Cl | Cl | H | H | F | OMe | H | H | $CF_3$ | H | H |
| 308* | H | Cl | Cl | H | H | F | H | H | PhO | H | H | H |
| 317** | H | Br | Br | H | H | H | Me | H | Me | H | H | H |
| 318** | H | Br | Br | H | H | H | F | H | H | H | H | H |
| 319** | H | Br | Br | H | H | H | Cl | H | Me | H | H | H |
| 320** | H | Br | Br | H | H | H | H | H | PhO | H | H | H |
| 321** | H | Br | Br | H | H | H | H | H | CN | H | H | H |
| 322** | H | Br | Br | H | H | H | OMe | H | H | $CF_3$ | H | H |
| 323** | H | Br | Br | H | H | H | $CH_2OH$ | H | H | H | H | H |
| 324** | H | Br | Br | H | H | H | $CH_2F$ | H | H | H | H | H |

# TABLEAU A (fin)

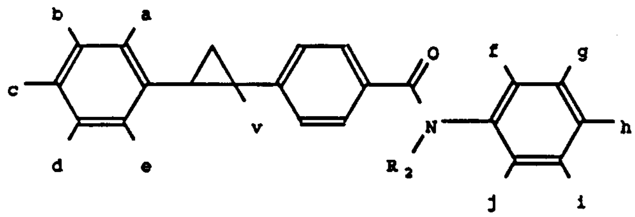

| Ex N°: | a | b | c | d | e | v | f | g | h | i | j | $R_2$ |
|---|---|---|---|---|---|---|---|---|---|---|---|---|
| 30* | H | H | MeO | H | H | F | Me | H | H | H | H | H |
| 54* | H | Br | Br | H | H | F | F | H | H | H | H | H |
| 55* | H | Br | Br | H | H | F | H | H | Me | H | H | H |
| 79* | H | Br | Br | H | H | F | MeOC(O) | H | H | H | H | H |
| 273* | H | Br | $CHF_2O$ | H | H | F | Me | H | H | H | H | H |
| 344* | H | H | TfO | H | H | F | Me | H | H | H | H | H |
| 345* | H | Cl | Cl | H | H | F | $CHF_2$ | H | H | H | H | H |
| 346* | H | Cl | Cl | H | H | F | Me | H | H | H | Cl | H |
| 266* | H | Cl | H | Cl | H | F | Me | H | H | H | H | H |
| 267* | H | Br | Br | H | H | F | H | Me | H | H | H | H |

TABLEAU B

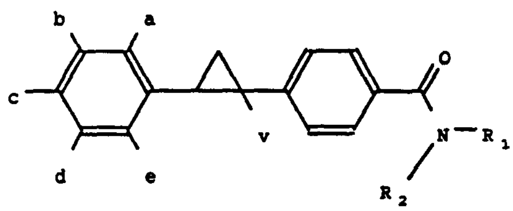

| Ex N°: | a | b | c | d | e | v | $R_1$ | $R_2$ |
|---|---|---|---|---|---|---|---|---|
| 1 | H | H | H | H | H | F | $(CH_3)_2CHCH_2$ | H |
| 3** | H | H | Cl | H | H | H | $(CH_3)_2CHCH_2$ | H |
| 5* | H | H | Cl | H | H | F | $(CH_3)_2CHCH_2$ | H |
| 8* | H | Br | Br | H | H | F | $(CH_3)_2CHCH_2$ | H |
| 9** | H | H | Cl | H | H | F | $(CH_3)_2CHCH_2$ | H |
| 122** | H | H | F | H | H | F | $(CH_3)_2CHCH_2$ | H |
| 123* | H | H | F | H | H | F | $(CH_3)_2CHCH_2$ | H |
| 124* | Cl | H | H | H | Cl | F | $(CH_3)_2CHCH_2$ | H |
| 125 | Cl | Cl | H | H | H | F | $(CH_3)_2CHCH_2$ | H |
| 126* | H | F | F | H | H | F | $(CH_3)_2CHCH_2$ | H |
| 127* | H | H | $CHF_2O$ | H | H | F | $(CH_3)_2CHCH_2$ | H |
| 128* | H | H | $CF_3O$ | H | H | F | $(CH_3)_2CCH_2$ | H |
| 129* | H | Br | Br | H | H | F | $(CH_3)_2CHCH(CH_3)$ | $CH_3CH_2$ |
| 130* | H | Br | Br | H | H | F | $CH_3(CH_2)_2$ | H |
| 131* | H | Br | Br | H | H | F | $(CH_3)_3CH_2C(CH_3)_2$ | H |
| 132* | H | Br | Br | H | H | F | $CH_3CH_2$ | $CH_3CH_2$ |
| 133* | H | Br | Br | H | H | F | $CH_3(CH_2)_3$ | H |
| 134* | H | Br | Br | H | H | F | $(CH_3)_2CHCH(CH_3)$ | H |
| 135* | H | Br | Br | H | H | F | $(CH_3)_2CHCH_2CH(CH_3)$ | H |
| 136* | H | Br | Br | H | H | F | $(CH_3)_2CHCH_2CH_2$ | H |
| 137* | H | Br | Br | H | H | F | $CH_3(CH_2)_2$ | $CH_3$ |
| 138* | H | Br | Br | H | H | F | $CH_3(CH_2)_5$ | H |
| 139* | H | Br | Br | H | H | F | $(CH_3)_3CCH(CH_3)$ | H |

## TABLEAU B (suite)

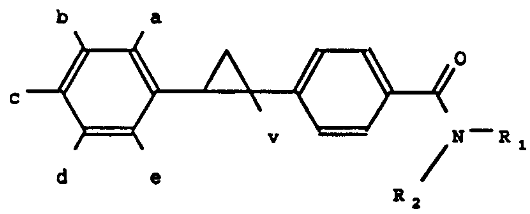

| Ex N° | a | b | c | d | e | v | $R_1$ | $R_2$ |
|---|---|---|---|---|---|---|---|---|
| 140* | H | Br | Br | H | H | F | $(CH_3)_3C$ | H |
| 141* | H | Br | Br | H | H | F | $(CH_3)_3CCH_2$ | H |
| 142* | H | Br | Br | H | H | F | $CH_3CH_2CH(CH_3)$ | H |
| 143* | H | Br | Br | H | H | F | $CH_3CH_2CH(CH_3)CH_2$ | H |
| 144* | H | Br | Br | H | H | F | $CH_3$ | $CH_3$ |
| 145* | H | Br | Br | H | H | F | $(CH_3)_2CH$ | H |
| 146* | H | Br | Br | H | H | F | $CH_3CH_2$ | H |
| 147* | H | Br | Br | H | H | F | $CH_3CH_2CH(C_2H_5)$ | H |
| 148* | H | Br | Br | H | H | F | $(C_2H_5)CH(CH_3)CH_2CH(CH_3)$ | H |
| 149* | H | Br | Br | H | H | F | $(CH_3)_2CH(CH_2)_3CH(CH_3)$ | H |
| 150* | H | Br | Br | H | H | F | $(CH_3)_3C(CH_2)_2$ | H |
| 151* | H | Br | Br | H | H | F | $(CH_3)_2CHCH_2$ | $CH_3$ |
| 152* | H | Br | Br | H | H | F | $(CH_3)_3C$ | $CH_3$ |
| 153* | H | Br | Br | H | H | F | $CH_3CH_2C(CH_3)_2$ | H |
| 154 | H | Br | Br | H | H | F | $CH_3(CH_2)_2CH(CH_3)$ | H |
| 155* | H | Br | Br | H | H | F | $(CH_3)_2CH$ | $(CH_3)_2CH$ |
| 156* | H | Br | Br | H | H | F | $(CH_3)_2CH$ | Et |
| 157** | H | H | Br | H | H | H | $(CH_3)_2CHCH_2$ | H |
| 158 | H | Br | Br | H | H | H | $(CH_3)_2CHCH_2$ | H |
| 159 | H | Cl | Cl | H | H | H | $(CH_3)_2CHCH(CH_3)$ | H |
| 161* | H | Cl | Cl | H | H | Cl | $(CH_3)_2CHCH_2$ | H |
| 162* | H | Cl | Cl | H | H | Cl | $(CH_3)_2CHCH(CH_3)$ | H |

TABLEAU B (suite)

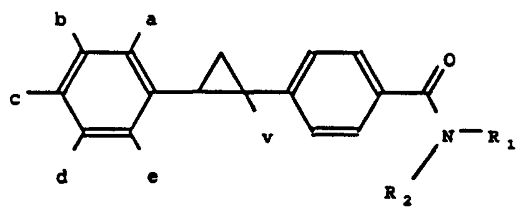

| Ex N°: | a | b | c | d | e | v | $R_1$ | $R_2$ |
|---|---|---|---|---|---|---|---|---|
| 163* | H | Cl | Cl | H | H | Br | $(CH_3)_2CHCH_2$ | H |
| 164* | H | Br | Br | H | H | F | $CH_2=C(CH_3)CH_2$ | H |
| 165* | H | Br | Br | H | H | F | $CH_2=CHCH_2$ | H |
| 166* | H | Br | Br | H | H | F | $CH\equiv CCH_2$ | H |
| 167* | H | Br | Br | H | H | F | $CH_2=C(Cl)CH_2$ | H |
| 168* | H | PhO | F | H | H | F | $(CH_3)_2CHCH_2$ | H |
| 169* | H | F | F | H | H | F | $C_6H_5$-$CH(CH_3)$ (R) | H |
| 170* | H | Br | Br | H | H | F | (2-Me $C_6H_4)CH_2$ | H |
| 171* | H | Br | Br | H | H | F | (4-Cl $C_6H_4)CH_2$ | H |
| 172* | H | Br | Br | H | H | F | $(C_6H_5)CH_2$ | H |
| 173* | H | Br | Br | H | H | F | $C_6H_5$-$CH(CH_3)$ | H |
| 174* | H | Br | Br | H | H | F | cyclobutyl | H |
| 175* | H | Br | Br | H | H | F | cyclohexyl | H |
| 176* | H | Br | Br | H | H | F | cyclopentyl | H |
| 177* | H | Br | Br | H | H | F | cyclopropyl | H |
| 178* | H | Br | Br | H | H | F | (cyclopropyl) méthyl | H |
| 179* | H | Br | Br | H | H | F | $C_6H_5$-$CH_2(CH_2)_3$ | H |
| 180 | H | Br | Br | H | H | F | (3-$CF_3$ $C_6H_4)CH_2CH(CH_3)$ | H |
| 181* | H | Br | Br | H | H | F | (cyclohexyl) méthyl | H |
| 182 | H | Br | Br | H | H | F | adamant-1-yl | H |
| 183* | H | Br | Br | H | H | F | 2-méthyl cyclohexyl | H |
| 184* | H | Br | Br | H | H | F | $CH\equiv CC(CH_3)_2$ | H |
| 185* | H | Br | Br | H | H | F | 2,3-dihydro 1-indènyl | H |

TABLEAU B (suite)

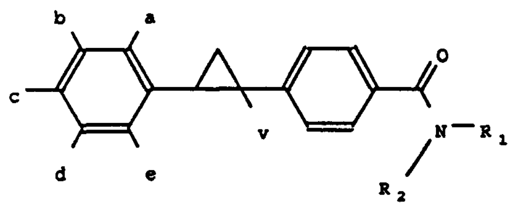

| Ex N°: | a | b | c | d | e | v | $R_1$ | $R_2$ |
|---|---|---|---|---|---|---|---|---|
| 186* | H | Br | Br | H | H | F | 1-naphthyl | H18 |
| 187* | H | Br | Br | H | H | F | $N\equiv CCH_2$ | H |
| 188 | H | Br | Br | H | H | F | (2-thiényl) méthyl | H |
| 190* | H | Br | Br | H | H | F | 4-tBu cyclohexyl (cis) | HH |
| 192* | H | Br | Br | H | H | F | $tBuOC(=O)CH_2$ | H |
| 193* | H | Br | Br | H | H | F | $MeSCH_2CH_2$ | H |
| 194* | H | Br | Br | H | H | F | $ClCH_2(CH_2)_2$ | H |
| 195 | H | Br | Br | H | H | F | 2-(4-morpholinyl) éthyl | H |
| 196* | H | Br | Br | H | H | F | $CF_3CH_2$ | H |
| 197* | H | Br | Br | H | H | F | $(C_7F_{15})CH_2$ | H |
| 198* | H | Br | Br | H | H | F | (tétrahydrofuran-2-yl) méthyl | H |
| 199* | H | Br | Br | H | H | F | 4-Me thiazol-2-yl | H |
| 200* | H | Br | Br | H | H | F | $(C_6H_5)_2CH$ | H |
| 201* | H | Br | Br | H | H | F | $(C_6H_5)CH_2CH(C_6H_5)$ | H |
| 202* | H | Br | Br | H | H | F | $(C_6H_5)_2CHCH_2$ | H |
| 203* | H | Br | Br | H | H | F | $(MeO)_2CHCH_2$ | H |
| 204* | H | Br | Br | H | H | F | $MeOCH_2CH_2$ | H |
| 205* | H | Br | Br | H | H | F | 3-(2-oxo pyrrolidino) propyl | H |
| 206* | H | Br | Br | H | H | F | $CH_2=C(Br)CH_2$ | H |
| 207* | H | Br | Br | H | H | F | $FCH_2CH_2$ | H |
| 208* | H | Br | Br | H | H | F | $[MeOC(=O)](C_6H_5)CH$ | H |
| 209* | H | Br | Br | H | H | F | (1-Et pyrrolidin-2yl) méthyl | H |
| 210* | H | Br | Br | H | H | F | 5-Me isoxazol-3-yl | H |
| 211* | H | Br | Br | H | H | F | $CH_2(OH)CH(CH_3)$ | H |

TABLEAU B (suite)

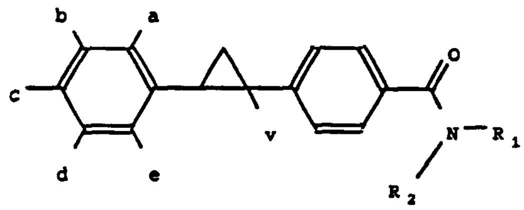

| Ex N°: | a | b | c | d | e | v | $R_1$ | $R_2$ |
|---|---|---|---|---|---|---|---|---|
| 212 | H | Cl | Cl | H | H | H | cyclobutyl | H |
| 213* | H | Br | Br | H | H | F | (4-Me thiazol-5-yl) méthyl | Me |
| 214* | Br | Br | H | H | H | F | $(CH_3)_2NCH_2CH_2$ | Me |
| 215* | H | Br | Br | H | H | F | $(CH_3)_2NCH_2CH(CH_3)$ | H |
| 216* | H | Br | Br | H | H | F | $(CH_3)_2NCH_2CH_2$ | H |
| 219* | H | Br | Br | H | H | F | $N(R_1)(R_2)$: 4-thiomorpholinyl | |
| 220* | H | Br | Br | H | H | F | $N(R_1)(R_2)$: aziridin-1-yl | |
| 221* | H | Br | Br | H | H | F | $N(R_1)(R_2)$: pipéridin-1-yl | |
| 222* | H | Br | Br | H | H | F | $N(R_1)(R_2)$: pyrrolidin-1-yl | |
| 223* | H | Br | Br | H | H | F | $N(R_1)(R_2)$: 4-morpholinyl | |
| 224* | H | Br | Br | H | H | F | $N(R_1)(R_2)$: 2-Me pipéridin-1-yl | |
| 225* | H | Br | Br | H | H | F | pyrrolidin-1-yl | H |
| 226* | H | Br | Br | H | H | F | 4-morpholinyl | H |
| 227* | H | Br | Br | H | H | F | (3,5-diCl $C_6H_3$)NH | H |
| 228* | H | Br | Br | H | H | F | pipéridin-1-yl | H |
| 229* | H | Br | Br | H | H | F | $(CH_3)_2N$ | H |
| 277* | H | Br | Br | H | H | F | (2,6-diMe)pipéridinyl-1-yl | H |
| 278* | H | Br | TfO | Br | H | F | $CH_3CH(CH_3)CH_2$ | H |
| 279* | H | Br | $CHF_2O$ | Br | H | F | $CH_3CH(CH_3)CH_2$ | H |
| 280* | H | Br | Br | H | H | F | pyridin-3-yl | H |
| 281* | H | Br | Br | H | H | F | $N(R_1)(R_2)$:2-$CH_2OH$ pyrrolidin-1-yl | |
| 293* | H | Br | Br | H | H | F | 2-Cl 3-pyridinyl | H |
| 294* | H | Cl | Cl | H | H | F | benzazétidin-1-yl | H |

TABLEAU B (suite)

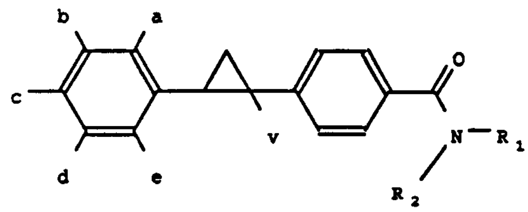

| Ex N°: | a | b | c | d | e | v | $R_1$ | $R_2$ |
|---|---|---|---|---|---|---|---|---|
| 295* | H | Cl | Cl | H | H | F | $(CH_3)_3CCH_2C(CH_3)_2$ | H |
| 296* | H | Cl | Cl | H | H | F | $C_6H_5-CH_2CH_2CH_2CH_2$ | H |
| 297* | H | Cl | Cl | H | H | F | $N(R_1)(R_2)$: 4-morpholinyl | |
| 298* | H | Cl | Cl | H | H | F | $(CH_3)_2CHCH_2$ | H |
| 299* | H | Cl | Cl | H | H | F | $(CH_3)_2CHCH(CH_3)$ | H |
| 300* | H | Cl | Cl | H | H | F | cyclopentyl | H |
| 301* | H | Cl | Cl | H | H | F | 4-morpholinyl | H |
| 302* | H | Cl | Cl | H | H | F | H | H |
| 303* | H | Cl | Cl | H | H | F | $(CH_3)_2C{=}CHCH_2$ | H |
| 304* | H | Cl | Cl | H | H | F | $CH_2{=}C(CH_3)CH_2$ | H |
| 305* | H | Cl | Cl | H | H | F | $Cl-CH_2CH_2CH_2$ | H |
| 306* | H | Cl | Cl | H | H | F | $(CH_3)_2CHCH_2CH_2$ | H |
| 307* | H | Cl | Cl | H | H | F | $CH_3$ | H |
| 309* | H | Cl | Cl | H | H | F | $CH_2{=}C(Br)CH_2$ | H |
| 310* | H | Cl | Cl | H | H | F | $CH_3-S-CH_2CH_2$ | H |
| 311* | H | Cl | Cl | H | H | F | $HC{\equiv}CCH_2$ | H |
| 312* | H | Cl | Cl | H | H | F | $C_6H_5-CH(CH_3)$ | H |
| 313* | H | Cl | Cl | H | H | F | $(CH_3)_2NCH_2CH_2$ | $CH_3$ |
| 314* | H | Cl | Cl | H | H | F | 2-THP | H |
| 315* | H | CL | Cl | H | H | F | cyclohex-3-èn-1-yl | H |
| 316* | H | Cl | Cl | H | H | F | tétrazol-5-yl | H |
| 325** | H | Br | Br | H | H | H | $(CH_3)_3CCH_2C(CH_3)_2$ | H |
| 326** | H | Br | Br | H | H | H | $C_6H_5-CH_2CH_2CH_2CH_2$ | H |
| 327** | H | Br | Br | H | H | H | $N(R_1)(R_2)$: 4-morpholinyl | |
| 328** | H | Br | Br | H | H | H | $(CH_3)_2CHCH_2$ | H |
| 329** | H | Br | Br | H | H | H | $(CH_3)_2CHCH(CH_3)$ | H |

TABLEAU B (fin)

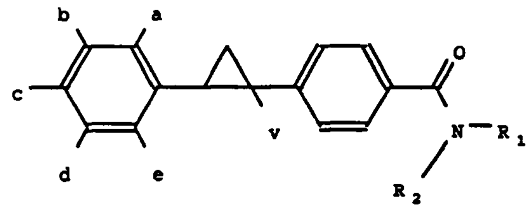

| Ex N°: | a | b | c | d | e | v | $R_1$ | $R_2$ |
|---|---|---|---|---|---|---|---|---|
| 330** | H | Br | Br | H | H | H | cyclopentyl | H |
| 331** | H | Br | Br | H | H | H | 4-morpholinyl | H |
| 332** | H | Br | Br | H | H | H | $CH_2=C(CH_3)CH_2$ | H |
| 333** | H | Br | Br | H | H | H | $(CH_3)_2CHCH_2CH_2$ | H |
| 334** | H | Br | Br | H | H | H | $CH_3$ | H |
| 335** | H | Br | Br | H | H | H | $CH_2=C(Br)CH_2$ | H |
| 336** | H | Br | Br | H | H | H | $CH_3SCH_2CH_2$ | H |
| 337** | H | Br | Br | H | H | H | $CH{\equiv}CCH_2$ | H |
| 338** | H | Br | Br | H | H | H | $C_6H_5-CH(CH_3)$ | H |
| 339** | H | Br | Br | H | H | H | $(CH_3)_2NCH_2CH_2$ | $CH_3$ |
| 340* | H | H | TfO | H | H | F | cyclobutyl | H |
| 191* | H | Br | BR | H | H | F | 4-tBu cyclohexyl (trans) | H |
| 341* | H | H | TfO | H | H | F | $(CH_3)_2CHCH(CH_3)$ | H |

TABLEAU C

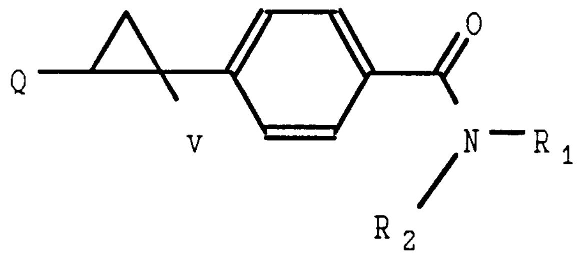

| Ex N°: | Q | V | $R_1$ | $R_2$ |
|---|---|---|---|---|
| 230** | 5-bromo 2-naphthyl | F | 2-méthyl phényl | éthyl |
| 231* | 5-bromo 2-naphthyl | F | 2-méthyl phényl | éthyl |
| 232* | 5-bromo 2-naphthyl | F | 2-méthyl phényl | H |
| 233** | 5-bromo 2-naphthyl | F | 2-méthyl phényl | H |
| 234* | 2-naphthyl | F | 2-méthyl phényl | H |
| 235* | 2-naphthyl | F | 2-méthyl propyl | H |
| 236* | 6-bromo 1-naphthyl | F | 2-méthyl phényl | H |
| 237 | 6-bromo 1-naphthyl | F | 2-méthyl propyl | H |
| 238 | 6-bromo 1-naphthyl | F | 1,2-diméthyl propyl | H |
| 239 | 7-bromo 1-naphthyl | F | 2-méthyl propyl | H |

TABLEAU D

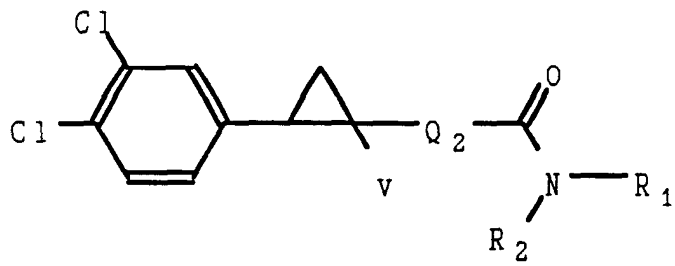

(Dans le tableau qui suit les radicaux phénylène sont liés à la fonction amide par le carbone en position 1, et les radicaux naphthylène par les carbones en positions 1 ou 2)

| Ex N°: | $Q_2$ | V | $R_1$ | $R_2$ |
|---|---|---|---|---|
| 240* | 1,3-phénylène | F | 2-méthyl phényl | H |
| 160** | 1,3-phénylène | F | 2-méthyl propyl | H |
| 241** | 1,3-phénylène | F | 2-méthyl phényl | $CH_3CH_2$ |
| 242* | 1,3-phénylène | F | 2-méthyl phényl | $CH_3CH_2$ |
| 243* | 1,3-phénylène | F | 2-méthyl propyl | H |
| 244* | 2-méthyl 1,4-phénylène | F | 2-méthyl phényl | H |
| 245* | 2-méthyl 1,4-phénylène | F | 2-méthyl propyl | H |
| 246 | 2-méthyl 1,4-phénylène | Me | 2-méthyl phényl | H |
| 247** | 2,3,5,6-tétrafluoro 1,4-phénylène | F | 2-méthyl phényl | H |
| 248* | 3-méthyl 1,4-phénylène | F | 2-méthyl phényl | H |
| 249* | 3-méthyl 1,4-phénylène | F | 2-méthyl propyl | H |
| 250* | 2-chloro 1,4-phénylène | F | 2-méthyl phényl | H |
| 251* | 2,5-naphthylène | F | 2-méthyl phényl | H |
| 252* | 2,5-naphthylène | F | 2-méthyl propyl | H |
| 253* | 1,5-naphthylène | F | 2-méthyl phényl | H |
| 254* | 1,5-naphthylène | F | 2-méthyl propyl | H |
| 255* | 2-acétylamino 5-chloro 1,4-phénylène | F | 2-méthyl phényl | H |
| 256* | 2-amino 5-chloro 1,4-phénylène | F | 2-méthyl phényl | H |
| 257** | 2,3,5,6-tétrafluoro 1,4-phénylène | H | 2-méthyl phényl | $CH_3CH_2$ |
| 258** | 2-chloro 1,4-phénylène | H | 2-méthyl phényl | H |

TABLEAU D (suite)

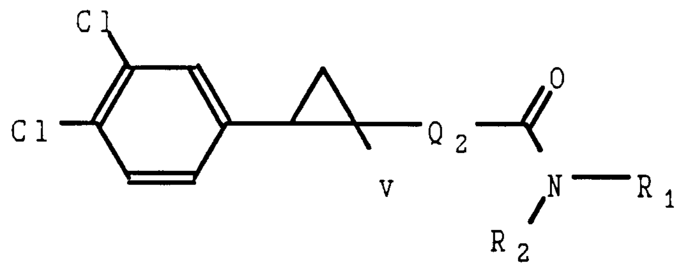

| Ex N°: | $Q_2$ | v | $R_1$ | $R_2$ |
|---|---|---|---|---|
| 259** | 2-chloro 1,4-phénylène | H | 2-méthyl propyl | H |
| 260* | 3-fluoro 1,4-phénylène | H | 2-méthyl phényl | H |
| 261* | 3-fluoro 1,4-phénylène | H | 2-méthyl propyl | H |
| 342 | 1,4-naphtylène | H | 2-méthyl phényl | H |
| 343 | 5-Cl 2(1-méthyléthylidène)amino 1,4-phénylène | F | 2-méthyl phényl | H |

Tableau E

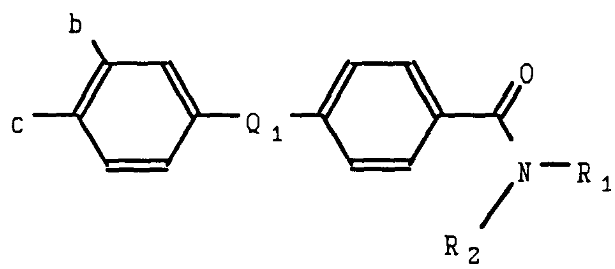

(Dans le tableau qui suit, la position 1 de $Q_1$ est liée au groupe anilide)

| Ex N°: | b | c | $Q_1$ | $R_1$ | $R_2$ |
|---|---|---|---|---|---|
| 262 | Br | Br | 3,3-dibromo 1,2-cyclopropanediyl | 2-méthyl phényl | H |
| 263 | H | Cl | 1,2-difluoro 1,2-cyclopropanediyl | 2-méthyl phényl | H |

TABLEAU F

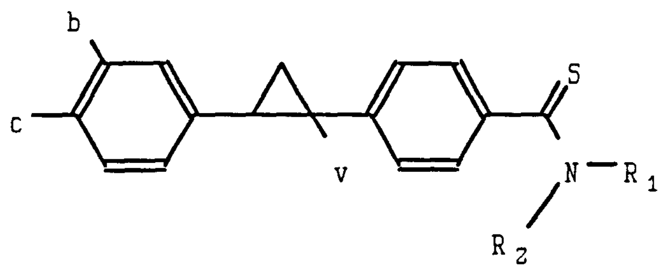

| Ex N°: | b | c | v | $R_1$ | $R_2$ |
|---|---|---|---|---|---|
| 264 | Br | Br | F | 2-méthyl phényl | H |
| 292 | Br | Br | F | 2-méthyl phényl | Et |
| 265 | Br | Br | F | 1,2-diméthyl propyl | H |

Dans le tableau ci-dessous, figurent des résultats d'analyses physiques de produits de formule (I).

| Produits des exemples | F°C | Rf |
|---|---|---|
| 4 | 133°C | 0,19 (hexane/AcOEt 7/3) |
| 6 | 172°C | 0,14 (hexane/AcOEt 8/2) |
| 7 | 158°C | 0,2 (hexane/AcOEt 7/3) |
| 12 | 148°C | 0,2 (hexane/AcOEt 7/3) |
| 13 | 126,8°C | 0,18 (hexane/AcOEt 7/3) |
| 15 | 130,1°C | 0,07 (éther isopropylique/ heptane/1/1) |
| 17 | 195°C | 0,25 (heptane/AcOEt 7/3) |
| 20 | - | 0,1 (heptane/AcOEt 85/15) |
| 23 | 128°C | 0,16 (heptane/AcOEt 7/3) |
| 32 | - | 0,12 (heptane/AcOEt 7/3) |
| 33 | - | 0,25 (heptane/AcOEt 7/3) |
| 38 | - | 0,13 (heptane/AcOEt 7/3) |
| 45 | 147°C | 0,18 (hexane/AcOEt 7/3) |
| 47 | 107,8°C | 0,18 (hexane/AcOEt 8/2) |
| 49 | 137°C | 0,11 (hexane/AcOEt 8/2) |
| 50 | 124°C | 0,45 (heptane/AcOEt 7/3) |
| 54 | 138,1°C | 0,25 (heptane/AcOEt 7/3) |
| 72 | - | 0,22 (heptane/AcOEt 7/3) |
| 73 | - | 0,25 (heptane/AcOEt 7/3) |
| 82 | - | 0,08 (heptane/AcOEt 7/3) |
| 86 | - | 0,24 (hexane/AcOEt 7/3) |
| 105 | - | 0,48 (hexane/AcOEt 7/3) |
| 113 | 156,6°C | 0,3 (heptane/AcOEt 1/1) |
| 114 | 122,3°C | 0,25 (heptane/AcOEt 1/1) |
| 117 | 163,6°C | 0,32 (hepatne/AcOEt 1/1) |

(suite)

| Produits des exemples | F°C | Rf |
|---|---|---|
| 121 | 141°C | 0,1 (heptane/AcOEt 8/2) |
| 284 | 164°C | 0,15 (heptane/Etheriso 1/1) |
| 292 | - | 0,5 (heptane/AcOEt 1/1) |
| 8 | 132°C | 0,17 (hexane/AcOEt 7/3) |
| 128 | 139°C | 0,14 (hexane/AcOEt 7/3) |
| 167 | 136°C | 0,15 (hexane/AcOEt 7/3) |
| 174 | 189,9°C | 0,23 (heptane/AcOEt 1/1) |
| 194 | - | 0,2 ($CH_2Cl_2$) |
| 226 | - | 0,02 (heptane/AcOEt 7/3) |
| 293 | - | 0,25 ($CH_2Cl_2$/TEA 99/1) |
| 231 | - | 0,2 (heptane/AcOEt 7/3) |
| 232 | - | 0,25 (heptane/AcOEt 7/3) |
| 236 | 184°C | 0,32 (heptane/dioxanne 6/4) |
| 244 | 159°C | 0,24 (heptane/AcOEt 7/3) |
| 264 | - | 0,42 (heptane/AcOEt 1/1) |
| 265 | - | 0,28 (hexane/AcOEt 7/3) |
| 273 | 132°C | 0,45 (heptane/AcOEt 1/1) |
| 299 | - | 0,25 (heptane/AcOEt 7/3) |

**Préparations de compositions.**

Dans les exemples de compositions ci-après, les signes suivants signifient :

* Tensioactif.

# Réagit en formant les parois de polyurée des microcapsules.

| 1. | Concentré émulsifiable. | |
|---|---|---|
| | Principe actif | 10,00 |
| | Alcoylphénol éthoxylé * | 7,50 |
| | Alcoylarylsulfonate * | 2,50 |
| | Solvant aromatique en C8-13 | 80,00 |
| | | 100,00 |

| 2. | Concentré émulsifiable. | |
|---|---|---|
| | Principe actif | 10,00 |
| | Alcoylphénol éthoxylé * | 2,50 |
| | Alcoylarylsulfonate * | 2,50 |
| | Solvant cétonique | 64,00 |
| | Solvant aromatique en C8-13 | 18,00 |
| | Antioxydant | 3,00 |
| | | 100,00 |

| 3. | Poudre mouillable. | |
|---|---|---|
| | Principe actif | 5,00 |
| | Solvant aromatique en C8-13 | 7,00 |
| | Solvant aromatique en C18 | 28,00 |
| | Kaolin | 10,00 |
| | Alcoylarylsulfonate * | 1,00 |

(suite)

| 3. | Poudre mouillable. | |
|---|---|---|
| | Acide naphtalènesulfonique * | 3,00 |
| | Terre de diatomées | 46,00 |
| | | 100,00 |

| 4. | Poudre à poudrer. | |
|---|---|---|
| | Principe actif | 0,50 |
| | Talc | 99,50 |
| | | 100,00 |

| 5. | Appât. | |
|---|---|---|
| | Principe actif | 0,5 |
| | Sucre | 79,5 |
| | Cire de paraffine | 20,0 |
| | | 100,00 |

| 6. | Concentré en émulsion. | |
|---|---|---|
| | Principe actif | 5,00 |
| | Solvant aromatique en C8-13 | 32,00 |
| | Alcool cétylique | 3,00 |
| | Monooléate de polyoxyéthylèneglycérol * | 0,75 |
| | Esters de polyoxyéthylènesorbitan * | 0,25 |
| | Solution de silicone | 0,1 |
| | Eau | 58,9 |
| | | 100,00 |

| 7. | Concentré en suspension. | |
|---|---|---|
| | Principe actif | 10,00 |
| | Alcoylphénol éthoxylé * | 3,00 |
| | Solution de silicone | 0,1 |
| | Alcanediol | 5,0 |
| | Fumée de silice | 0,50 |
| | Gomme de xanthane | 0,20 |
| | Eau | 80,0 |
| | Agent tampon | 1,2 |
| | | 100,00 |

| 8. | Microémulsion. | |
|---|---|---|
| | Principe actif | 10,00 |
| | Monooléate de polyoxyéthylèneglycérol * | 10,00 |
| | Alcanediol | 4,00 |
| | Eau | 76,00 |
| | | 100,00 |

| 9. | Granules dispersables dans l'eau. | |
|---|---|---|
| | Principe actif | 70,00 |
| | Polyvinylpyrrolidine | 2,50 |
| | Alcoylphénol éthoxylé | 1,25 |
| | Alcoylarylsulfonate | 1,25 |
| | Kaolin | 25,00 |
| | | 100,00 |

| 10. | Granules. | |
|---|---|---|
| | Principe actif | 2,00 |
| | Alcoylphénol éthoxylé * | 5,00 |
| | Alcoylarylsulfonate * | 3,00 |
| | Solvant aromatique C8-13 | 20,00 |
| | Granules de kieselguhr | 70,00 |
| | | 100,00 |

| 11. | Aérosol (bombe). | |
|---|---|---|
| | Principe actif | 0,3 |
| | Pipéronylbutoxyde | 1,5 |
| | Solvant hydrocarboné saturé en C8-13 | 58,2 |
| | Butane | 40,0 |
| | | 100,00 |

| 12. | Aérosol (bombe). | |
|---|---|---|
| | Principe actif | 0,3 |
| | Solvant hydrocarboné saturé en C8-13 | 10,0 |
| | Monooléate de sorbitan * | 1,0 |
| | Eau | 40,0 |
| | Butane | 48,7 |
| | | 100,00 |

| 13. | Aérosol (bombe). | |
|---|---|---|
| | Principe actif | 1,00 |
| | CO2 | 3,00 |
| | Monooléate de polyoxyéthylèneglycérol * | 1,40 |
| | Propanone | 38,00 |
| | Eau | 56,60 |
| | | 100,00 |

| 14. | Vernis. | |
|---|---|---|
| | Principe actif | 2,50 |
| | Résine | 5,00 |
| | Antioxydant | 0,50 |

(suite)

| | | |
|---|---|---|
| 14. | Vernis. | |
| | White spirit très aromatique | 92,0<br>100,00 |

| | | |
|---|---|---|
| 15. | Spray (prêt à l'usage). | |
| | Principe actif<br>Antioxydant<br>Kérosène inodore | 0,10<br>0,10<br>99,8<br>100,00 |

| | | |
|---|---|---|
| 16. | Spray potentialisé (prêt à l'usage). | |
| | Principe actif<br>Pipéronylbutoxyde<br>Antioxydant<br>Kérosène inodore | 0,10<br>0,50<br>0,10<br>99,30<br>100,00 |

| | | |
|---|---|---|
| 17. | Microcapsules. | |
| | Principe actif<br>Solvant aromatique en C8-13<br>Diisocyanate aromatique #<br>Alcoylphénol éthoxylé *<br>Alcoyldiamine #<br>Diéthylènetriamine<br>Acide chlorhydrique concentré<br>Gomme de xanthane<br>Fumée de silice<br>Eau | 10,0<br>10,0<br>4,5<br>6,0<br>1,0<br>1,0<br>2,2<br>0,2<br>0,5<br>64,6<br>100,00 |

| | | |
|---|---|---|
| 18. | Concentré dispersable. | |
| | Principe actif<br>N-méthylpyrrolidinone<br>N-alcoylpyrrolidinone<br>Solvant aromatique en C8-13<br>Phosphate d'éther polyoxyéthylénique de nonylphénol<br>Alcoylphénol éthoxylé<br>Alcoylarylsulfonate<br>Ether polyalcoylèneglycolique | 5,0<br>15,00<br>53,00<br>16,00<br>6,00<br>3,50<br>1,30<br>0,20<br>100,00 |

| | | |
|---|---|---|
| 19. | Concentré soluble | |
| | On effectue un mélange homogène de :<br>Principe actif<br>Butoxyde pipéronyle | <br>0,25<br>1,00 |

(suite)

| | | |
|---|---|---|
| 19. | Concentré soluble | |
| | Tween 80<br>Topanol A<br>Eau | 0,25<br>0,1<br>98,40 |

| | | |
|---|---|---|
| 20. | Concentré émulsifiable | |
| | On mélange intimement :<br>Principe actif<br>Butoxyde pipéronyle<br>Topanol A<br>Tween 80<br>Xylène | <br>0,015<br>0,5<br>0,1<br>3,5<br>95,885 |

## ETUDE BIOLOGIQUE

### A) Etude sur Phaedon cochleariae

Le produit est dissous à la concentration voulue dans un mélange acétone-eau (50-50). Des disques foliaires de chou chinois (Brassica pekinensis) sont immergés cinq secondes dans la solution, puis laissés à sécher pendant une heure. Dix adultes (mâles et femelles) en mélange) sont ajoutés dans une boîte de Pétri contenant chacune un disque foliaire. Celles-ci sont conservées à une température de 25°C, avec une photopériode de douze heures. Après sept jours, la mortalité des insectes est contrôlée, et la surface foliaire consommée est évaluée.
Les produits suivants présentent une bonne activité dès la dose de 30 ppm : produits des exemples 5, 7, 7B, 8, 12, 13, 17, 18, 231, 232, 45, 128, 226, 86, 87, 141, 145, 194, 105, 284, 264, 183, 218, 286, 298, 301, 346.

### B) Etude sur spodoptera littoralis

Le produit est dissous à la concentration voulue dans un mélange acétone-eau (50-50). Des feuilles de Haricot (Phaseolus vulgaris, var. Delinel) sont immergées cinq secondes dans la solution, puis laissées à sécher dans une boîte de Petri pendant une heure. Dix larves de Spodoptera littoralis sont ensuite ajoutées dans chaque boîte. Celles-ci sont conservées à une température de 25°C, avec une photopériode de douze heures. Après sept jours, la mortalité des larves est contrôlée, et la surface foliaire consommée est évaluée.
Les produits suivants présentent une bonne activité dès la dose de 30 ppm : produits des exemples 4, 6, 7, 7B, 8, 12, 13, 15, 17, 18, 20, 22, 23, 32, 33, 38, 44, 45, 47, 48, 49, 50, 128, 129, 344, 236, 345, 54, 174, 175, 178, 63, 346, 176, 65, 66, 134, 69, 72, 73, 226, 82, 83, 86, 87, 89, 90, 141, 142, 228, 143, 145, 293, 206, 105, 167, 284, 113, 114, 117, 159, 212, 264, 292, 121, 265, 290, 309, 288, 300, 266, 315, 299, 269, 267, 119, 95, 98, 317, 273, 286, 184, 329, 154, 283, 183, 153, 275, 285, 298, 324.

### C) Etude sur Heliothis virescens

Le produit est dissous à la concentration voulue dans un mélange acétone-eau (50-50). 50 µl de solution sont déposés à la surface d'un petit puits contenant environ 2 grammes de milieu artificiel à base végétale. Une larve néonate de Heliothis virescens est ensuite introduite dans chaque puits, qui est fermé avec une feuille de cellophane. Les essais sont conservés à une température de 25°C, avec une photopériode de douze heures. La mortalité des larves est contrôlée après sept jours.
Les produits suivants présentent une bonne activité dès la dose de 30 ppm : produits des exemples 7, 7B, 12, 22, 32, 33, 38, 49, 50, 54, 175, 63, 65, 69, 72, 83, 86, 89, 90, 105, 284, 117, 244, 292, 324, 299, 275, 286, 271, 273, 315, 346, 95.

### D) Etude de l'activité sur Diabrotica

Les insectes tests sont des larves de dernier stade de Diabrotica undécimpunctata.
On traite une rondelle de papier filtre de 9 cm de diamètre, déposée au fond d'une boîte de Petri, à l'aide de

1 $cm^3$ de solution acétonique. Après séchage, on dépose 15 larves par dose et on effectue le contrôle de mortalité 24 heures après le traitement.

Les produits suivants présentent une bonne activité dès la dose de 100 ppm : produits des exemples 134, 81, 86, 286, 298, 299, 346.

## Revendications

**1.** Sous toutes leurs formes stéréoisomères ou sous forme de mélanges de ces stéréoisomères, les composés de formule (I) :

$$Q\text{-}(CH_2)_a\text{-}(X_1)_b\text{-}Q_1\text{-}Q_2\text{-}C(X_2)\text{-}N(R_1)(R_2) \qquad (I)$$

dans laquelle :

Q représente un radical aryle ou un radical dérivé d'un hydrocarbure bicyclique condensé comportant un cycle benzénique, qui est lié au groupe adjacent $(CH_2)_a$ par un atome de carbone dudit cycle benzénique, Q comporte de 6 à 12 atomes de carbone et peut être soit non substitué, soit substitué,
a et b sont identiques ou différents et sont indépendamment l'un de l'autre égaux à 0 ou à 1,
$X_1$ représente un radical bivalent oxy, thio, sulfinyle ou sulfonyle,
$X_2$ représente un atome d'oxygène ou un atome de soufre,
$Q_1$ représente un radical cyclopropanediyle, qui est soit non substitué, soit substitué,
$Q_2$ représente un radical arylène ou un radical dérivé d'un hydrocarbure bicyclique condensé comportant un cycle benzénique, qui est lié aux groupes adjacents $Q_1$ et $C(X_2)$ par deux atomes de carbone dudit cycle benzénique, $Q_2$ comporte de 6 à 12 atomes de carbone et peut indépendamment de Q, être soit non substitué, soit substitué
$R_1$ et $R_2$ représentent indépendamment l'un de l'autre :

**soit** un atome d'hydrogène,
**soit** un radical dérivé d'un hydrocarbure cyclique ou acyclique, aromatique ou non aromatique, linéaire ou ramifié, saturé ou insaturé, et comportant de 1 à 12 atomes de carbone, lui-même pouvant être soit non substitué, soit substitué,
**soit** un radical dérivé d'une structure linéaire ou ramifiée comportant de 1 à 20 atomes de carbone et de 1 à 6 hétéroatomes choisis parmi les atomes d'oxygène, d'azote ou de soufre, lui-même pouvant être soit non substitué, soit substitué,
**soit** un radical dérivé d'un hétérocycle aromatique ou non aromatique comportant de 5 à 9 atomes de carbone et de 1 à 4 hétéroatomes choisis parmi les atomes d'oxygène, d'azote ou de soufre, ce radical pouvant être non substitué ou

substitué,

**soit** un groupe (A) :

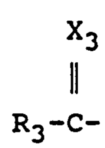

dans lequel $X_3$ représente un atome d'oxygène ou un atome de soufre, et $R_3$ représente un atome d'hydrogène ou un groupe $R_4\text{-}(T_1)_i$ dans lequel i est égal à 0 ou à 1, $T_1$ représente un groupe bivalent oxy, carbonyle ou oxycarbonyle et $R_4$ représente un atome d'hydrogène ou un radical dérivé d'un hydrocarbure cyclique ou acyclique, aromatique ou non aromatique, linéaire ou ramifié, saturé ou insaturé, et comportant de 1 à 20 atomes de carbone, lui-même pouvant être soit non substitué, soit substitué,
**soit** un groupe (B) :

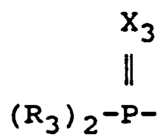

dans lequel $R_3$ et $X_3$ sont tels que définis plus haut,
**soit** un groupe (C) :

$$-T_2-R_5$$

dans lequel $T_2$ représente un radical bivalent thio, sulfinyle, sulfonyle ou sulfonyloxy et $R_5$ représente un atome d'hydrogène ou un radical dérivé d'un hydrocarbure cyclique ou acyclique, aromatique ou non aromatique, linéaire ou ramifié, saturé ou insaturé, et comportant de 1 à 8 atomes de carbone, lui-même pouvant être soit non substitué, soit substitué,
**soit** un groupe (D) :

$$[\,S(O)_j]_k-N(R_6)(R_7)$$

dans lequel j est égal à 0, 1 ou 2, k est égal à 0 ou 1, $R_6$ représente un atome d'hydrogène ou un radical dérivé d'un hydrocarbure cyclique ou acyclique, aromatique ou non aromatique, linéaire ou ramifié, saturé ou insaturé, et comportant de 1 à 8 atomes de carbone, lui-même pouvant être soit non substitué, soit substitué, et $R_7$ représente un groupe carboxy, fluorocarbonyle, alcoxycarbonyle comportant de 2 à 5 atomes de carbone, ou un radical acyle comportant de 1 à 5 atomes de carbone, ou un radical alkyle comportant de 1 à 4 atomes de carbone substitué par un groupe cyano ou par un radical alcoxycarbonyle comportant de 2 à 5 atomes de carbone, ou par un acyle comportant de 1 à 5 atomes de carbone, ou par un radical aryle comportant de 6 à 10 atomes de carbone, non substitué ou substitué par un ou plusieurs groupes choisis parmi les groupes halo, cyano, nitro, trifluorométhyle, trifluorométhoxy, trifluorométhylthio ou les radicaux alkyle comportant de 1 à 4 atomes de carbone ou alkoxy comportant de 1 à 4 atomes de carbone ou $R_6$ et $R_7$ forment ensemble avec l'atome d'azote auquel ils sont liés un hétérocycle azoté comportant de 3 à 10 chaînons renfermant éventuellement un ou plusieurs autres hétéroatomes cycliques choisis parmi l'oxygène, l'azote et le soufre et éventuellement substitué,
**soit** $R_1$ et $R_2$ représentent ensemble avec l'atome d'azote auquel ils sont liés un hétérocycle azoté comportant de 3 à 10 chaînons renfermant éventuellement un ou plusieurs autres hétéroatomes cycliques choisis parmi l'oxygène, l'azote et le soufre et éventuellement substitué étant entendu que dans ladite formule (I) lorsque Q représente un radical 2-(phénylaminocarbonyl) phényle, $Q_1$ un radical 1,2-cyclopropanediyle, $Q_2$ un radical ortho-phénylène, $X_2$ un atome d'oxygène, $R_2$ un atome d'hydrogène et que a et b sont égaux à 0, $R_1$ ne représente pas un radical phényle.

**2.** Composé de formule (I) telle que définie à la revendication 1 dans laquelle Q est non substitué ou substitué par un ou plusieurs substituants choisis parmi les atomes d'halogène ou les groupes méthylènedioxy, difluorométhylènedioxy, tétrafluoro éthylènedioxy, cyano, nitro, cyanato, thiocyanato, pentafluorothio, fluorosulfonyle, ou $R-(T)_c-$ dans lequel R représente un atome d'hydrogène ou un radical dérivé d'un hydrocarbure cyclique ou acyclique, aromatique ou non aromatique linéaire ou ramifié, saturé ou insaturé, et comportant de 1 à 8 atomes de carbone, lui-même pouvant être soit non substitué, soit substitué par un ou plusieurs atomes d'halogène, c est égal à 0 ou à 1 et T représente un groupe bivalent oxy, carbonyle, carbonyloxy, oxycarbonyle, thio, sulfinyle, sulfonyle, sulfonyloxy, $-(CO)_d-N(R')-(CO)_e-(O)_f-$ ou $-N(R'')-S(O)_g-$ dans lesquels R' et R'' représentent indépendamment de R un atome d'hydrogène ou un radical dérivé d'un hydrocarbure cyclique ou acyclique, aromatique ou non aromatique, linéaire ou ramifié, saturé ou insaturé, et comportant de 1 à 8 atomes de carbone, lui-même pouvant être soit non substitué, soit substitué par un ou plusieurs atomes d'halogène, d, e et f sont égaux à 0 ou à 1, f est égal à 0 quand e est égal à 0, la somme e + d est égale à 0 ou à 1, et g est égal à 0, à 1 ou à 2,
$Q_1$ est non substitué ou substitué par un ou plusieurs substituants choisis parmi les atomes d'halogène, les radicaux cyano, azido, ou aliphatiques, saturés ou insaturés comportant de 1 à 4 atomes de carbone non substitués ou substitués par un ou plusieurs atomes d'halogène et $Q_2$ est non susbtitué ou substitué par un ou plusieurs substituants choisis parmi les atomes d'halogène ou les groupes méthylènedioxy, difluorométhylènedioxy, tétrafluoro

éthylènedioxy, cyano, nitro, amino, alkylamino, alkénylamino, cyanato, thiocyanato, pentafluorothio, fluorosulfonyle, ou R-$(T)_c$- dans lequel R représente un atome d'hydrogène ou un radical dérivé d'un hydrocarbure cyclique ou acyclique, aromatique ou non aromatique linéaire ou ramifié, saturé ou insaturé, et comportant de 1 à 8 atomes de carbone, lui-même pouvant être soit non substitué, soit substitué par un ou plusieurs atomes d'halogène, c est égal à 0 ou à 1 et T représente un groupe bivalent oxy, carbonyle, carbonyloxy, oxycarbonyle, thio, sulfinyle, sulfonyle, sulfonyloxy, $-(CO)_d-N(R')-(CO)_e-(O)_f-$ ou $-N(R'')-S(O)_g-$ dans lesquels R', R", d, e, f et g sont tels que définis précédemment.

**3.** Composé de formule (I) telle que définie à la revendication 1 ou 2, dans laquelle Q est un radical phényle ou un radical naphtyle, non substitué ou substitué par 1 à 3 substituants.

**4.** Composé de formule (I) telle que définie à la revendication 3 dans laquelle Q représente un radical choisi parmi les radicaux phényle, 2-chloro phényle, 3-chloro phényle, 3-bromophényle, 3-(trifluorométhyl) phényle, 4-chloro phényle, 4-bromo phényle, 4-iodo phényle, 4-(trifluorométhyl) phényle, 4-nitrophényle, 4-méthoxy phényle, 4-(difluorométhoxy) phényle, 4-(trifluorométhoxy) phényle, 3-bromo 4-(difluorométhoxy) phényle, 4-(2,2-dibromo éthényl) phényle, 4-éthynyl phényle, 4-benzyl phényle, 3,4-dibromo phényle, 2,4-dichlorophényle, 3,4-dichloro phényle, 3,4-difluorophényle, 3-chloro 4-iodo phényle, 4-bromo 3-chloro phényle, 4-bromo 2-fluoro phényle, 4-bromo 3-fluoro phényle, 4-bromo 3-(trifluorométhyl) phényle, 4-chloro 3-(trifluorométhyl) phényle, 3,5-bis(trifluorométhyl) phényle, 3,4,5-trichloro phényle, 4-bromo 3,5-dichloro phényle, 3-phénoxyphényle, 4-(fluoro 3-phénoxy) phényle, 3-bromo 4-(trifluorométhylsulfonyloxy) phényle, 3,4-bis (trifluorométhylsulfonyloxy) phényle, 2-naphtyle ou 5-bromo 2-naphtyle ou 6-bromo 1-napthyle.

**5.** Composé de formule (I) telle que définie à l'une quelconque des revendications 1 à 4, dans laquelle la configuration stérique de $Q_1$ est telle que le groupe $Q-(CH_2)_a-(X_1)_b-$ est en position trans par rapport au groupe $Q_2-C(X_2)-N(R_1)(R_2)$.

**6.** Composé de formule (I) telle que définie à l'une quelconque des revendications 1 à 5 dans laquelle $Q_1$ est non substitué ou substitué par 1 ou 2 atomes d'halogène ou 1 ou 2 radicaux méthyle.

**7.** Composé de formule (I) telle que définie à la revendication 6 dans laquelle $Q_1$ représente un radical choisi parmi les radicaux 1,2-cyclopropanediyle, 1-fluoro 1,2-cyclopropanediyle, 1-chloro 1,2-cyclopropanediyle, 1-bromo 1,2-cyclopropanediyle, 1-méthyle 1,2-cyclopropanediyle, 3,3-dibromo 1,2-cyclopropanediyle et 1,2-difluoro 1,2-cyclopropanediyle.

**8.** Composé de formule (I) telle que définie à l'une quelconque des revendications 1 à 7, dans laquelle $Q_2$ représente un des radicaux ortho-phénylène, méta-phénylène, para-phénylène, 1,2-naphtylène, 1,3-naphtylène, 1,4-naphtylène, 1,5-naphtylène, 2,5-naphtylène, 4,5-indènylène, 4,6-indènyléne, 4,7-indènylène, 4,5-indanylène, 4,6-indanylène, 4,7-indanylène, 1,2,3,4-tétrahydro 5,6-naphtylène, 1,2,3,4-tétrahydro 5,7-naphtylène, 1,2,3,4-tétrahydro 5,8-naphtylène, non substitué ou substitué par 1 à 4 substituants.

**9.** Composés de formule (I) telle que définie à la revendication 8 dans laquelle $Q_2$ représente un des radicaux 2-méthyl 1,4-phénylène, 2,3,5,6-tétrafluoro 1,4-phénylène, 3-méthyl 1,4-phénylène, 2-chloro 1,4-phénylène, 2-acétylamino 5-chloro 1,4-phénylène, 2-amino 5-chloro 1,4-phénylène, 2-diméthylamino 5-chloro 1,4-phénylène.

**10.** Composé de formule (I) telle que définie à l'une quelconque des revendications 1 à 9, dans laquelle $R_1$ représente un radical alkyle ramifié comportant de 1 à 6 atomes de carbone tel que le radical isopropyle, isobutyle, 1,2-diméthyl propyle, 1,1,2-triméthyl propyle ou 2,2-diméthyl propyle, 1-méthylpropyle, 2-méthylbutyle, 2,2-diméthyl 1-méthyléthyle, 3-chloropropyle, cyclobutyle, cyclohexyle, cyclopropylméthyle, un radical (2-méthyl 1,3-dioxolan 2-yl) méthyle, ou un radical alkényle ramifié comportant de 2 à 5 atomes de carbone tel que le radical 2-méthyl 2-propényle, 2-chloro 2-propényle, 2-bromo 2-propényle ou encore un radical phényle non substitué ou un radical phényle substitué tel que le radical 2-fluoro phényle, 2-chloro phényle, 2-bromo phényle, 2-iodo phényle, 2-nitro phényle, 2-hydroxy phényle, 2-méthoxyphényle, 2-cyano phényle, 2-amino phényle, 2-(diméthylamino) phényle, 2-(trifluorométhoxy) phényle, 2-phénoxy phényle, 2-méthoxycarbonyl phényle, 2-éthoxycarbonyl phényle, 2-phénylcarbonylphényle, 2-formyl phényle, 2-acétyl phényle, 2-(méthylthio) phényle, 2-fluorosulfonyl phényle, 2-éthynyl phényle, 2-(1-méthyl 2-propényl) phényle, 2-(hydroxyméthyl) phényle, 2-méthyl phényle, 2-(terbutyl) phényle, 2-(fluorométhyl) phényle, 2-(difluorométhyl) phényle, 2-(trifluorométhyl) phényle, 2-éthyl phényle, 2-propyl phényle, 2-isopropyl phényle, 2-(pyridin-1-yl) phényle, 3-chloro phényle, 3-bromophényle, 3-méthoxy phényle, 3-fluoro phényle, 3-méthyl phényle, 3-phénoxyphényle, 4-fluoro phényle, 4-chloro phényle, 4-cyano phényle, 4-(cyanométhyl) phényle, 4-méthoxy phényle, 4-méthyl phényle, 4-(terbutyl) phényle), 4-(trifluorométhyl) phényle, 4-phénoxyphényle, 4-benzyloxyphényle, 4-méthoxycarbonylphényle, 4-cyclohexylphényle, 2,4-dichlorophényle, 2,5-dichlorophényle, 2,6-dichlorophényle, 3,4-dichlorophényle, 3,5-dichlorophényle, 2,3-diméthylphényle, 2,4-diméthylphényle, 2,5-diméthylphényle, 2,6-diméthylphényle, 3,5-diméthylphényle, 2,4,6-triméthylphényle, 2,5-diméthoxyphényle, 3,4-diméthoxyphényle, 3,5-diméthoxyphényle, 2-méthoxy 5-méthylphényle, 2-méthoxy 5-trifluorométhylephényle, 3-méthoxy 5-trifluorométhylphényle, 2-méthoxy 5-nitrophényle, 2-nitro 4-méthoxyphényle, 2-méthyl 4-méthoxyphényle, 3,5-dichloro 4-méthoxyphényle, 4,5-dichloro 2-méthoxyphényle, 4-bromo 3,5-dichlorophényle, 2-(1-méthyléthyl) phényle, 3-chloro 2-méthyl phényle, 3-fluoro 2-méthyl phényle, 3-bromo 2-méthyl phényle, 2,3-dichloro phényle, 2,3-dibromo phényle, 3,4-dibromophényle, 4-fluoro 2-méthyl phényle, 4-chloro 2-fluoro phényle, 4-fluoro 2-(trifluorométhyl) phényle, 2,4-difluoro phényle, 2-chloro 4-méthylphényle, 2-chloro 5-méthyl phényle, 5-chloro 2-méthyl phényle, 5-fluoro 2-méthyl phényle, 5-iodo 2-méthyl phényle, 2,6-difluoro phényle, 2-fluoro 4-méthylphényle, 2-fluoro 6-méthyl phényle, 2-chloro 6-méthyl phényle ou bien un radical dérivé d'un hétérocycle tel que le radical 2-chloro 3-pyridyle, 3-pyridyle, 2-pyridyle, 4-pyridyle, 2-chloro 3-pyridyle, 3-méthyl pyridyle, 5-méthyl isoxazol-3-yle, 1,3,4-thiadiazol-2-yle, 4-pyrimidinyle, 3-pyrazolyle, 4-(trifluorométhyl) thiazol-2-yle, 5-chloro 4-(trifluorométhyl) thiazol-2-yle, 1-pipéridinyle, 2,6-diméthyl 1-pipéridinyle, 1,4-oxazin-4-yle ou 1-perhydroazépinyle, ou enfin $R_1$ et $R_2$ représentent ensemble avec l'atome d'azote auquel ils sont liés un hétérocycle tel que le radical 1,4-thiazin-4-yle, 1,4-oxazin-4-yle, 1-aziridinyle, 1-pipéridinyle, 1-pyrrolidinyle, 2-méthylpipéridin-1-yle, 2-hydroxyméthylpyrrolidin-1-yle.

**11.** Composé de formule (I) telle que définie à l'une quelconque des revendications 1 à 10, dans laquelle $R_2$ représente un atome d'hydrogène, un radical alkyle comportant de 1 à 4 atomes de carbone, notamment le radical éthyle, un radical benzyle, un radical éthoxyméthyle, un radical propoxyméthyle ou un groupe (A) dans lequel $X_3$ représente un atome d'oxygène et $R_3$ représente un radical alkyle comportant de 1 à 11 atomes de carbone, un radical alcoxycarbonyle comportant de 2 à 5 atomes de carbone ou phénoxycarbonyle, et $R_2$ représente notamment dans ce cas, le radical acétyl hexanoyle, décanoyle, 2-méthoxy 2-oxo acétyle, 2-éthoxy 2-oxo acétyle, 2-phénoxy 2-oxo acétyle ou un groupe (B) dans lequel $R_3$ représente un radical alkoxy comportant de 1 à 4 atomes de carbone et $R_2$ représente notamment dans ce cas le radical diéthoxyphosphonyle ou un groupe (C) dans lequel $T_2$ représente un radical bivalent thio et $R_5$ un radical trifluorométhyle ou pentafluoroéthyle ou un radical phényle non substitué ou substitué par un ou plusieurs atomes d'halogène ou un ou plusieurs radicaux alkyle comportant de 1 à 4 atomes de carbone, et dans ce cas $R_2$ représente notamment le radical trifluorométhylthio, pentafluoroéthylthio ou un radical phénylthio, (4-chloro phényl) thio, (2-méthyl phényl) thio, (4-méthyl phényl) thio ou (4-tertbutyl phényl) thio ou un groupe (D) dans lequel j est égal à O et $R_2$ représente notamment dans ce cas le radical (N-formyl N-méthyl amino) thio ou (N-méthoxycarbonyl N-méthyl amino) thio.

**12.** Composé de formule (I) telle que définie à l'une quelconque des revendications 1 à 11 dans laquelle a et b sont égaux à 0.

**13.** Procédé de préparation du composé de formule (I) telle que définie à l'une quelconque des revendications 1 à 12, caractérisé en ce que l'acide ou le dérivé d'acide correspondant de formule (II) :

$$Q\text{-}(CH_2)_a\text{-}(X_1)_b\text{-}Q_1\text{-}Q_2\text{-}C(O)\text{-}Z \qquad (II)$$

dans laquelle Q, $X_1$, a, b, $Q_1$ et $Q_2$ sont tels que définis dans les revendications 1 à 12 et Z représente un groupe hydroxy, un groupe halogéné, un radical alkoxy comportant de 1 à 4 atomes de carbone ou un groupe -P(O)(OΦ)-NHΦ, dans lequel Φ représente un groupe phényle,
est mis à réagir avec une amine de formule (III) :

$$HN(R_1)(R_2) \qquad (III)$$

dans laquelle $R_1$ et $R_2$ sont tels que définis précédemment, pour obtenir un produit de formule (I) correspondant, dans laquelle $X_2$ représente un atome d'oxygène, qui si nécessaire, est converti en un produit de fomule (I) dans laquelle ($X_2$) représente un atome de soufre.

**14.** Utilisation des composés de formule (I) telle que définie à l'une quelconque des revendications 1 à 13 comme pesticide notamment comme insecticide, acaricide et nématicide dans la protection des cultures notamment les

cultures de riz et de coton, ou pour le traitement des lieux de stockage des produits desdites cultures et notamment comme insecticide ou acaricide dans les locaux domestiques ou publics.

**15.** Composition pesticide comprenant :

a) un composé de formule (I) telle que définie à l'une quelconque des revendications 1 à 13,
b) des excipients inertes appropriés à l'utilisation comme pesticide dudit produit de formule (I).

**16.** Composition pesticide comprenant :

a) un composé de formule (I) telle que définie à l'une quelconque des revendications 1 à 13,
b) des excipients inertes appropriés à l'utilisation dans le domaine vétérinaire dudit produit de formule (I).

**17.** Composé de formule (I) telle que définie à l'une des revendications 1 à 13, pour la mise en oeuvre d'une méthode de traitement du corps humain ou animal caractérisé en ce qu'une formulation pharmaceutiquement acceptable dudit composé est appliquée sur ledit corps.

**Patentansprüche**

**1.** In sämtlichen ihrer stereoisomeren Formen oder in Form von Gemischen dieser Stereoisomeren die Verbindungen der Formel (I):

$$Q\text{-}(CH_2)_a\text{-}(X_1)_b\text{-}Q_1\text{-}Q_2\text{-}C(X_2)\text{-}N(R_1)(R_2) \qquad (I)$$

worin

Q einen Arylrest oder einen Rest, abgeleitet von einem kondensierten bicyclischen Kohlenwasserstoff, enthaltend einen Benzolring, der an die benachbarte Gruppe $(CH_2)_a$ über ein Kohlenstoffatom des Benzolrings gebunden ist, bedeutet, Q 6 bis 12 Kohlenstoffatome aufweist und entweder unsubstituiert oder substituiert sein kann,
a und b identisch oder verschieden sind und unabhängig voneinander für 0 oder für 1 stehen,
$X_1$ einen zweiwertigen Oxy-, Thio-, Sulfinyl- oder Sulfonylrest wiedergibt,
$X_2$ ein Sauerstoffatom oder ein Schwefelatom darstellt,
$Q_1$ einen Cyclopropandiylrest wiedergibt, der entweder unsubstituiert oder substituiert ist,
$Q_2$ einen Arylenrest oder einen Rest, abgeleitet von einem kondensierten bicyclischen Kohlenwasserstoff, enthaltend einen Benzolring, der an die benachbarten Gruppen $Q_1$ und $C(X_2)$ über zwei Kohlenstoffatome des Benzolrings gebunden ist, bedeutet, $Q_2$ 6 bis 12 Kohlenstoffatome aufweist und unabhängig von Q entweder unsubstituiert oder substituiert sein kann,
$R_1$ und $R_2$ unabhängig voneinander bedeuten:

entweder ein Wasserstoffatom,

oder einen Rest, abgeleitet von einem cyclischen oder acyclischen, aromatischen oder nichtaromatischen, linearen oder verzweigten, gesättigten oder ungesättigten Kohlenwasserstoff und enthaltend 1 bis 12 Kohlenstoffatome, der seinerseits unsubstituiert oder substituiert sein kann,
oder einen Rest, abgeleitet von einer linearen oder verzweigten Struktur mit 1 bis 20 Kohlenstoffatomen und 1 bis 6 Heteroatomen, ausgewählt unter den Sauerstoff-, Stickstoff- oder Schwefelatomen, der seinerseits entweder unsubstituiert oder substituiert sein kann,
oder einen Rest, abgeleitet von einem aromatischen oder nichtaromatischen Heterocyclus mit 5 bis 9 Kohlenstoffatomen und 1 bis 4 Heteroatomen, ausgewählt unter den Sauerstoff-, Stickstoff- oder Schwefelatomen, wobei dieser Rest unsubstituiert oder substituiert sein kann,
oder eine Gruppe (A):

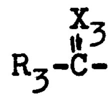

worin $X_3$ ein Sauerstoffatom oder ein Schwefelatom bedeutet und $R_3$ ein Wasserstoffatom oder eine Gruppe $R_4$-$(T_1)_i$ wiedergibt, worin i für 0 oder 1 steht, $T_1$ eine zweiwertige Oxy-, Carbonyl- oder Oxycarbonylgruppe bedeutet und $R_4$ ein Wasserstoffatom oder einen Rest, abgeleitet von einem cyclischen oder acyclischen, aromatischen oder nichtaromatischen, linearen oder verzweigten, gesättigten oder ungesättigten Kohlenwasserstoff und enthaltend 1 bis 20 Kohlenstoffatome, bedeutet, der seinerseits unsubstituiert oder substituiert sein kann,
oder eine Gruppe (B):

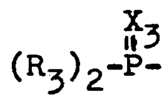

worin $R_3$ und $X_3$ wie vorstehend definiert sind,
oder eine Gruppe (C):

$$-T_2-R_5$$

worin $T_2$ einen zweiwertigen Thio-, Sulfinyl-, Sulfonyl- oder Sulfonyloxyrest bedeutet und $R_5$ ein Wasserstoffatom oder einen Rest, abgeleitet von einem cyclischen oder acyclischen, aromatischen oder nichtaromatischen, linearen oder verzweigten, gesättigten oder ungesättigten Kohlenwasserstoff und enthaltend 1 bis 8 Kohlenstoffatome, bedeutet, der seinerseits entweder unsubstituiert oder substituiert sein kann,
oder eine Gruppe (D):

$$[S(O)_j]_k-N(R_6)(R_7)$$

worin j für 0, 1 oder 2 steht, k für 0 oder 1 steht, $R_6$ ein Wasserstoffatom oder einen Rest, abgeleitet von einem cyclischen oder acyclischen, aromatischen oder nichtaromatischen, linearen oder verzweigten, gesättigten oder ungesättigten Kohlenwasserstoff und enthaltend 1 bis 8 Kohlenstoffatome, bedeutet, der seinerseits unsubstituiert oder substituiert sein kann, und $R_7$ eine Carboxy-, Fluorcarbonyl-, Alkoxycarbonylgruppe mit 2 bis 5 Kohlenstoffatomen oder einen Acylrest mit 1 bis 5 Kohlenstoffatomen oder einen Alkylrest mit 1 bis 4 Kohlenstoffatomen, substituiert durch eine Cyanogruppe oder durch einen Alkoxycarbonylrest mit 2 bis 5 Kohlenstoffatomen, oder Acyl mit 1 bis 5 Kohlenstoffatomen oder durch einen Arylrest mit 6 bis 10 Kohlenstoffatomen, unsubstituiert oder substituiert durch eine oder mehrere Gruppen, ausgewählt unter den Halo-, Cyano-, Nitro-, Trifluormethyl-, Trifluormethoxy-, Trifluormethylthio-Gruppen oder den Alkylresten mit 1 bis 4 Kohlenstoffatomen oder Alkoxyresten mit 1 bis 4 Kohlenstoffatomen, wiedergibt, oder $R_6$ und $R_7$ gemeinsam mit dem Stickstoffatom, an das sie gebunden sind, einen Stickstoffheterocyclus mit 3 bis 10 Gliedern, gegebenenfalls umfassend ein oder mehrere weitere Ringheteroatome, ausgewählt unter Sauerstoff, Stickstoff und Schwefel, und gegebenenfalls substituiert, bilden,
oder $R_1$ und $R_2$ gemeinsam mit dem Stickstoffatom, an das sie gebunden sind, einen Stickstoffheterocyclus mit 3 bis 10 Gliedern, gegebenenfalls umfassend ein oder mehrere weitere Ringheteroatome, ausgewählt unter Sauerstoff, Stickstoff und Schwefel, und gegebenenfalls substituiert, bilden, mit der Maßgabe, daß in besagter Formel (I), wenn Q einen 2-(Phenylaminocarbonyl)-phenylrest, $Q_1$ einen 1,2-Cyclopropandiylrest, $Q_2$ einen ortho-Phenylenrest, $X_2$ ein Sauerstoffatom, $R_2$ ein Wasserstoffatom bedeutet und a und b für O stehen, $R_1$ keinen Phenylrest darstellt.

**2.** Verbindung der Formel (I), wie in Anspruch 1 definiert, worin

Q unsubstituiert ist oder substituiert durch einen oder mehrere Substituenten, ausgewählt unter den Halogenatomen oder den Methylendioxy-, Difluormethylendioxy-, Tetrafluorethylendioxy-, Cyano-, Nitro-, Cyanato-, Thiocyanato-, Pentafluorthio-, Fluorsulfonylgruppen oder R-$(T)_c$-, worin R ein Wasserstoffatom oder einen

Rest, abgeleitet von einem cyclischen oder acyclischen, aromatischen oder nichtaromatischen, linearen oder verzweigten, gesättigten oder ungesättigten Kohlenwasserstoff und enthaltend 1 bis 8 Kohlenstoffatome, der seinerseits unsubstituiert oder durch ein oder mehrere Halogenatome substituiert sein kann, bedeutet, c für 0 oder für 1 steht und T eine zweiwertige Oxy-, Carbonyl-, Carbonyloxy-, Oxycarbonyl-, Thio-, Sulfinyl-, Sulfonyl-, Sulfonyloxygruppe, eine Gruppe $-(CO)_d-N(R')-(CO)_e-(O)_f-$ oder $-N(R'')-S(O)_g-$ bedeutet, worin R' und R" unabhängig von R ein Wasserstoffatom oder einen Rest, abgeleitet von einem cyclischen oder acyclischen, aromatischen oder nichtaromatischen, linearen oder verzweigten, gesättigten oder ungesättigten Kohlenwasserstoff und enthaltend 1 bis 8 Kohlenstoffatome, der seinerseits unsubstituiert oder durch ein oder mehrere Halogenatome substituiert sein kann, wiedergeben, d, e und f für 0 oder für 1 stehen, f für 0 steht, wenn e für 0 steht, die Summe e + d für 0 oder für 1 steht und g für 0, für 1 oder für 2 steht,
$Q_1$ unsubstituiert oder substituiert ist durch einen oder mehrere Substituenten, ausgewählt unter den Halogenatomen, den Cyano-, Azido- oder aliphatischen, gesättigten oder ungesättigten Resten mit 1 bis 4 Kohlenstoffatomen, die unsubstituiert oder durch ein oder mehrere Halogenatome substituiert sind, und $Q_2$ unsubstituiert oder substituiert ist durch einen oder mehrere Substituenten, ausgewählt unter den Halogenatomen und den Methylendioxy-, Difluormethylendioxy-, Tetrafluorethylendioxy-, Cyano-, Nitro-, Amino-, Alkylamino-, Alkenylamino-, Cyanato-, Thiocyanato-, Pentafluorthio-, Fluorsulfonylgruppen oder $R-(T)_c-$, worin R ein Wasserstoffatom oder einen Rest, abgeleitet von einem cyclischen oder acyclischen, aromatischen oder nichtaromatischen, linearen oder verzweigten, gesättigten oder ungesättigten Kohlenwasserstoff und enthaltend 1 bis 8 Kohlenstoffatome, der seinerseits unsubstituiert oder durch ein oder mehrere Halogenatome substituiert sein kann, bedeutet, c für 0 oder für 1 steht und T eine zweiwertige Oxy-, Carbonyl-, Carbonyloxy-, Oxycarbonyl-, Thio-, Sulfinyl-, Sulfonyl-, Sulfonyloxygruppe, eine Gruppe $-(CO)_d-N(R')-(CO)_e-(O)_f-$ oder $-N(R'')-S(O)_g-$ wiedergibt, worin R', R", d, e, f und g wie zuvor definiert sind.

**3.** Verbindung der Formel (I) gemäß Anspruch 1 oder 2, worin Q einen Phenylrest oder einen Naphthylrest, der unsubstituiert oder durch 1 bis 3 Substituenten substituiert ist, bedeutet.

**4.** Verbindung der Formel (I) gemäß Anspruch 3, worin Q einen Rest bedeutet, ausgewählt unter den Phenyl-, 2-Chlorphenyl-, 3-Chlorphenyl-, 3-Bromphenyl-, 3-(Trifluormethyl)-phenyl-, 4-Chlorphenyl-, 4-Bromphenyl-, 4-Jodphenyl-, 4-(Trifluormethyl)-phenyl-, 4-Nitrophenyl-, 4-Methoxyphenyl-, 4-(Difluormethoxy)-phenyl-, 4-(Trifluormethoxy)-phenyl-, 3-Brom-4-(difluormethoxy)-phenyl-, 4-(2,2-Dibromethenyl)-phenyl-, 4-Ethinylphenyl-, 4-Benzylphenyl-, 3,4-Dibromphenyl-, 2,4-Dichlorphenyl-, 3,4-Dichlorphenyl-, 3,4-Difluorphenyl-, 3-Chlor-4-jodphenyl-, 4-Brom-3-chlorphenyl-, 4-Brom-2-fluorphenyl-, 4-Brom-3-fluorphenyl-, 4-Brom-5-(trifluormethyl)-phenyl-, 4-Chlor-3-(trifluormethyl)-phenyl-, 3,5-Bis-(trifluormethyl)-phenyl-, 3,4,5-Trichlorphenyl-, 4-Brom-3,5-dichlorphenyl-, 3-Phenoxyphenyl-, 4-(Fluor-3-phenoxy)-phenyl-, 3-Brom-4-(trifluormethylsulfonyloxy)-phenyl-, 3,4-Bis-(trifluormethylsulfonyloxy)-phenyl-, 2-Naphthyl- oder 5-Brom-2-naphthyl- oder 6-Brom-1-naphthylresten.

**5.** Verbindung der Formel (I), wie in einem der Ansprüche 1 bis 4 definiert, worin die sterische Konfiguration von $Q_1$ derart ist, daß sich die Gruppe $Q-(CH_2)_a-(X_1)_b-$ in trans-Stellung in bezug auf die Gruppe $Q_2-C(X_2)-N(R_1)(R_2)$ befindet.

**6.** Verbindung der Formel (I), wie in einem der Ansprüche 1 bis 5 definiert, worin $Q_1$ unsubstituiert oder durch 1 oder 2 Halogenatome oder 1 oder 2 Methylreste substituiert ist.

**7.** Verbindung der Formel (I), wie in Anspruch 6 definiert, worin
$Q_1$ einen Rest bedeutet, ausgewählt unter den 1,2-Cyclopropandiyl-, 1-Fluor-1,2-cyclopropandiyl-, 1-Chlor-1,2-cyclopropandiyl-, 1-Brom-1,2-cyclopropandiyl-, 1-Methyl-1,2-cyclopropandiyl-, 3,3-Dibrom-1,2-cyclopropandiyl- und 1,2-Difluor-1,2-cyclopropandiylresten.

**8.** Verbindung der Formel (I), wie in einem der Ansprüche 1 bis 7 definiert, worin $Q_2$ einen der ortho-Phenylen-, meta-Phenylen-, para-Phenylen-, 1,2-Naphthylen-, 1,3-Naphthylen-, 1,4-Naphthylen-, 1,5-Naphthylen-, 2,5-Naphthylen-, 4,5-Indenylen-, 4,6-Indenylen-, 4,7-Indenylen-, 4,5-Indanylen-, 4,6-Indanylen-, 4,7-Indanylen-, 1,2,3,4-Tetrahydro-5,6-naphthylen-, 1,2,3,4-Tetrahydro-5,7-naphthylen-, 1,2,3, 4-Tetrahydro-5,8-naphthylen-Reste, unsubstituiert oder durch 1 bis 4 Substituenten substituiert, bedeutet.

**9.** Verbindungen der Formel (I), wie in Anspruch 8 definiert, worin $Q_2$ einen der 2-Methyl-1,4-phenylen-, 2,3,5,6-Tetrafluor-1,4-phenylen-, 3-Methyl-1,4-phenylen-, 2-Chlor-1,4-phenylen-, 2-Acetylamino-5-chlor-1,4-phenylen-, 2-Amino-5-chlor-1,4-phenylen-, 2-Dimethylamino-5-chlor-1,4-phenylenreste wiedergibt.

**10.** Verbindung der Formel (I), wie in einem der Ansprüche 1 bis 9 definiert, worin $R_1$ einen verzweigten Alkylrest mit 1 bis 6 Kohlenstoffatomen, wie den Isopropyl-, Isobutyl-, 1,2-Dimethylpropyl-, 1,1,2-Trimethylpropyl- oder 2,2-Dimethylpropyl-, 1-Methylpropyl-, 2-Methylbutyl-, 2,2-Dimethyl-1-methylethyl-, 3-Chlorpropyl-, Cyclobutyl-, Cyclohexyl-, Cyclopropylmethylrest,oder einen (2-Methyl-1,3-dioxolan-2-yl)-methylrest oder einen verzweigten Alkenylrest mit 2 bis 5 Kohlenstoffatomen, wie den 2-Methyl-2-propenyl-, 2-Chlor-2-propenyl-, 2-Brom-2-propenylrest oder auch einen unsubstituierten Phenylrest oder einen substituierten Phenylrest, wie den 2-Fluorphenyl-, 2-Chlorphenyl-, 2-Bromphenyl-, 2-Jodphenyl-, 2-Nitrophenyl-, 2-Hydroxyphenyl-, 2-Methoxyphenyl-,2-Cyanophenyl-, 2-Aminophenyl-, 2-(Dimethylamino)-phenyl-, 2-(Trifluormethoxy)-phenyl-, 2-Phenoxyphenyl-, 2-Methoxycarbonylphenyl-, 2-Ethoxycarbonylphenyl-, 2-Phenylcarbonylphenyl-, 2-Formylphenyl-, 2-Acetylphenyl-, 2-(Methylthio)-phenyl-, 2-Fluorsulfonylphenyl-, 2-Ethinylphenyl-, 2-(1-Methyl-2-propenyl)-phenyl-, 2-(Hydroxymethyl)-phenyl-, 2-Methylphenyl-, 2-(tert.-Butyl)-phenyl-, 2-(Fluormethyl)-phenyl-, 2-(Difluormethyl)-phenyl-, 2-(Trifluormethyl)-phenyl-, 2-Ethylphenyl-, 2-Propylphenyl-, 2-Isopropylphenyl-, 2-(Pyridin-1-yl)-phenyl-, 3-Chlorphenyl-, 3-Bromphenyl-, 3-Methoxyphenyl-, 3-Fluorphenyl-, 3-Methylphenyl-, 3-Phenoxyphenyl-, 4-Fluorphenyl-, 4-Chlorphenyl-, 4-Cyanophenyl-, 4-(Cyanomethyl)-phenyl-, 4-Methoxyphenyl-, 4-Methylphenyl-, 4-(tert.-Butyl)-phenyl-, 4-(Trifluormethyl)-phenyl-, 4-Phenoxyphenyl-, 4-Benzyloxyphenyl-, 4-Methoxycarbonylphenyl-, 4-Cyclohexylphenyl-, 2,4-Dichlorphenyl-, 2,5-Dichlorphenyl-, 2,6-Dichlorphenyl-, 3,4-Dichlorphenyl-, 3,5-Dichlorphenyl-, 2,3-Dimethylphenyl-, 2,4-Dimethylphenyl-, 2,5-Dimethylphenyl-, 2,6-Dimethylphenyl-, 3,5-Dimethylphenyl-, 2,4,6-Trimethylphenyl-, 2,5-Dimethoxyphenyl-, 3,4-Dimethoxyphenyl-, 3,5-Dimethoxyphenyl-, 2-Methoxy-5-methylphenyl-, 2-Methoxy-5-trifluormethyl-, 3-Methoxy-5-trifluormethylphenyl-, 2-Methoxy-5-nitrophenyl-, 2-Nitro-4-methoxyphenyl-, 2-Methyl-4-methoxyphenyl-, 3,5-Dichlor-4-methoxyphenyl-, 4,5-Dichlor-2-methoxyphenyl-, 4-Brom-3,5-dichlorphenyl-, 2-(1-Methylethyl)-phenyl-, 3-Chlor-2-methylphenyl-, 3-Fluor-2-methylphenyl-, 3-Brom-2-methylphenyl-, 2,3-Dichlorphenyl-, 2,3-Dibromphenyl-, 3,4-Dibromphenyl-, 4-Fluor-2-methylphenyl-, 4-Chlor-2-fluorphenyl-, 4-Fluor-2-(trifluormethyl)-phenyl-, 2,4-Difluorphenyl-, 2-Chlor-4-methylphenyl-, 2-Chlor-5-methylphenyl-, 5-Chlor-2-methylphenyl-, 5-Fluor-2-methylphenyl-, 5-Jod-2-methylphenyl-, 2,6-Difluorphenyl-, 2-Fluor-4-methylphenyl-, 2-Fluor-6-methylphenyl-, 2-Chlor-6-methylphenylresten, oder aber einen Rest, abgeleitet von einem Heterocyclus, wie den 2-Chlor-3-pyridyl-, 3-Pyridyl-, 2-Pyridyl-, 4-Pyridyl-, 2-Chlor-3-pyridyl-, 3-Methylpyridyl-, 5-Methyl-isoxazol-3-yl-, 1,3,4-Thiadiazol-2-yl-, 4-Pyrimidinyl-, 3-Pyrazolyl-, 4-(Trifluormethyl)-thiazol-2-yl-, 5-Chlor-4-(trifluormethyl)-thiazol-2-yl-, 1-Piperidinyl-, 2,6-Dimethyl-1-piperidinyl-, 1,4-Oxazin-4-yl- oder 1-Perhydroazepinylrest, wiedergibt oder schließlich $R_1$ und $R_2$ gemeinsam mit dem Stickstoffatom, an das sie gebunden sind, einen Heterocyclus, wie den 1,4-Thiazin-4-yl-, 1,4-Oxazin-4-yl-, 1-Aziridinyl-, 1-Piperidinyl-, 1-Pyrrolidinyl-, 2-Methylpiperidin-1-yl-, 2-Hydroxymethylpyrrolidin-1-yl-rest, bilden.

**11.** Verbindung der Formel (I), wie in einem der Ansprüche 1 bis 10 definiert, worin $R_2$ ein Wasserstoffatom, einen Alkylrest mit 1 bis 4 Kohlenstoffatomen, insbesondere den Ethylrest, einen Benzylrest, einen Ethoxymethylrest, einen Propoxymethylrest oder eine Gruppe (A) bedeutet, worin $X_3$ ein Sauerstoffatom darstellt und $R_3$ einen Alkylrest mit 1 bis 11 Kohlenstoffatomen, einen Alkoxycarbonylrest mit 2 bis 5 Kohlenstoffatomen oder einen Phenoxycarbonylrest wiedergibt und $R_2$, insbesondere in diesem Fall, den Acetylhexanoyl-, Decanoyl-, 2-Methoxy-2-oxoacetyl-, 2-Ethoxy-2-oxoacetyl-, 2-Phenoxy-2-oxoacetyl-Rest bedeutet oder eine Gruppe (B), worin $R_3$ einen Alkoxyrest mit 1 bis 4 Kohlenstoffatomen bedeutet,und $R_2$, insbesondere in diesem Fall, den Diethoxyphosphonylrest wiedergibt, oder eine Gruppe (C), worin $T_2$ für einen zweiwertigen Thiorest steht und $R_5$ einen Trifluormethyl- oder Pentafluorethylrest oder einen Phenylrest, unsubstituiert oder durch ein oder mehrere Halogenatome oder einen oder mehrere Alkylreste mit 1 bis 4 Kohlenstoffatomen substituiert, bedeutet, und in diesem Fall $R_2$ insbesondere den Trifluormethylthio-, Pentafluorethylthiorest oder einen Phenylthio-, (4-Chlorphenyl)-thio-, (2-Methylphenyl)-thio-, (4-Methylphenyl)-thio- oder (4-tert.-Butylphenyl)-thiorest bedeutet, oder eine Gruppe (D), worin j für O steht und $R_2$, insbesondere in diesem Fall, den (N-Formyl-N-methylamino)-thio- oder (N-Methoxycarbonyl-N-methylamino)-thiorest wiedergibt.

**12.** Verbindung der Formel (I), wie in einem der Ansprüche 1 bis 11 definiert, worin a und b für 0 stehen.

**13.** Verfahren zur Herstellung der Verbindung der Formel (I), wie in einem der Ansprüche 1 bis 12 definiert, dadurch gekennzeichnet, daß die Säure oder das entsprechende Säurederivat der Formel (II)

$$Q\text{-}(CH_2)_a\text{-}(X_1)_b\text{-}Q_1\text{-}Q_2\text{-}C(O)\text{-}Z \quad (II)$$

worin Q, $X_1$, a, b, $Q_1$ und $Q_2$ wie in den Ansprüchen 1 bis 12 definiert sind und Z eine Hydroxygruppe, eine Halogengruppe, eine Alkoxygruppe mit 1 bis 4 Kohlenstoffatomen oder eine Gruppe -P(O)(O$\phi$)-NH$\phi$ bedeutet, worin

ϕ für eine Phenylgruppe steht,
mit einem Amin der Formel (III)

$$HN(R_1)(R_2) \quad (III)$$

worin $R_1$ und $R_2$ wie vorstehend definiert sind, umgesetzt wird, um zu einem entsprechenden Produkt der Formel (I), worin $X_2$ ein Sauerstoffatom bedeutet, zu gelangen, das erforderlichenfalls in ein Produkt der Formel (I) übergeführt wird, worin ($X_2$) für ein Schwefelatom steht.

**14.** Verwendung der Verbindungen der Formel (I), wie in einem der Ansprüche 1 bis 13 definiert, als Pestizid, insbesondere als Insektizid, Akarizid und Nematizid, beim Schutz von Kulturen, insbesondere Reis-und Baumwollkulturen, oder für die Behandlung von Aufbewahrungsorten der Produkte besagter Kulturen und vor allem als Insektizid oder Akarizid an häuslichen oder öffentlichen Plätzen.

**15.** Pestizidzusammensetzung, umfassend

a) eine Verbindung der Formel (I), wie in einem der Ansprüche 1 bis 13 definiert,
b) inerte Exzipienten, die für die Verwendung als Pestizid des besagten Produkts der Formel (I) geeignet sind.

**16.** Pestizide Zusammensetzung, umfassend

a) eine Verbindung der Formel (I), wie in einem der Ansprüche 1 bis 13 definiert,
b) inerte Exzipienten, die für die Verwendung im Veterinärbereich des besagten Produkts der Formel (I) geeignet sind.

**17.** Verbindung der Formel (I), wie in einem der Ansprüche 1 bis 13 definiert, für den Einsatz bei einem Verfahren zur Behandlung des menschlichen oder tierischen Körpers, dadurch gekennzeichnet, daß eine geeignete pharmazeutische Formulierung der besagten Verbindung auf den besagten Körper aufgebracht wird.

## Claims

**1.** In all their stereoisomer forms or in the form of mixtures of these stereoisomers, the compounds of formula (I):

$$Q\text{-}(CH_2)_a\text{-}(X_1)_b\text{-}Q_1\text{-}Q_2\text{-}C(X_2)\text{-}N(R_1)(R_2) \quad (I)$$

in which:

Q represents an aryl radical or a radical derived from a condensed bicyclic hydrocarbon comprising a benzene ring, which is linked to the adjacent $(CH_2)_a$ group by a carbon atom of said benzene ring, Q comprises from 6 to 12 carbon atoms and can be either non substituted, or substituted, a and b are identical or different and independently of one another are equal to 0 or 1,
$X_1$ represents a divalent oxy, thio, sulphinyl or sulphonyl radical,
$X_2$ represents an oxygen atom or a sulphur atom,
$Q_1$ represents a cyclopropanediyl radical, which is either non substituted, or substituted,
$Q_2$ represents an arylene radical or a radical derived from a condensed bicyclic hydrocarbon comprising a benzene ring, which is linked to the adjacent groups $Q_1$ and $C(X_2)$ by two carbon atoms of said benzene ring, $Q_2$ comprises from 6 to 12 carbon atoms and can independently of Q, either be non substituted, or substituted
$R_1$ and $R_2$ represent independently one another:

**either** a hydrogen atom,

**or** a radical derived from a cyclic or acyclic, aromatic or non aromatic, linear or branched, saturated or unsaturated hydrocarbon, and comprising 1 to 12 carbon atoms, itself being able to be either non substituted, or substituted,

**or** a radical derived from a linear or branched structure comprising 1 to 20 carbon atoms and 1 to 6 heteroatoms chosen from oxygen, nitrogen or sulphur atoms, itself being able to be either non substituted, or substituted,
**or** a radical derived from an aromatic or non aromatic heterocycle comprising 5 to 9 carbon atoms and 1 to 4 heteroatoms chosen from oxygen, nitrogen or sulphur atoms, this radical being able to be non substituted or substituted,
**or** a group (A):

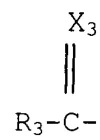

in which $X_3$ represents an oxygen atom or a sulphur atom, and $R_3$ represents a hydrogen atom or an $R_4$-$(T_1)_i$ group in which i is equal to 0 or 1, $T_1$ represents a divalent oxy, carbonyl or oxycarbonyl group and $R_4$ represents a hydrogen atom or a radical derived from a cyclic or acyclic, aromatic or non aromatic, linear or branched, saturated or unsaturated hydrocarbon, and comprising 1 to 20 carbon atoms, itself being able to be either non substituted, or substituted,
**or** a group (B):

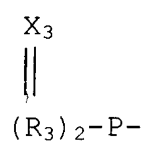

in which $R_3$ and $X_3$ are as defined above,
**or** a group (C):

$$-T_2-R_5$$

in which $T_2$ represents a divalent thio, sulphinyl, sulphonyl or sulphonyloxy radical and $R_5$ represents a hydrogen atom or a radical derived from a cyclic or acyclic, aromatic or non aromatic, linear or branched, saturated or unsaturated hydrocarbon, and comprising 1 to 8 carbon atoms, itself being able to be either non substituted, or substituted,
**or** a group (D):

$$[S(O)_j]_k-N(R_6)(R_7)$$

in which j is equal to 0, 1 or 2, k is equal to 0 or 1, $R_6$ represents a hydrogen atom or a radical derived from a cyclic or acyclic, aromatic or non aromatic, linear or branched, saturated or unsaturated hydrocarbon, and comprising 1 to 8 carbon atoms, itself being able to be either non substituted, or substituted, and $R_7$ represents a carboxy, fluorocarbonyl, alkoxycarbonyl group comprising 2 to 5 carbon atoms, or an acyl radical comprising 1 to 5 carbon atoms, or an alkyl radical comprising 1 to 4 carbon atoms substituted by a cyano group or by an alkoxycarbonyl radical comprising 2 to 5 carbon atoms, or an acyl comprising 1 to 5 carbon atoms, or by an aryl radical comprising 6 to 10 carbon atoms, non substituted or substituted by one or more groups chosen from the halo, cyano, nitro, fluoromethyl, trifluoromethoxy, trifluoromethylthio groups or the alkyl radicals comprising 1 to 4 carbon atoms or alkoxy radicals comprising 1 to 4 carbon atoms or $R_6$ and $R_7$ form together with the nitrogen atom to which they are linked a nitrogenous heterocycle comprising 3 to 10 members optionally containing one or more other cyclic heteroatoms chosen from oxygen, nitrogen and sulphur and optionally substituted,
**or** $R_1$ and $R_2$ represent together with the nitrogen atom to which they are linked a nitrogenous heterocycle comprising 3 to 10 members optionally containing one or more other cyclic heteroatoms chosen from oxygen, nitrogen and sulphur and optionally substituted, it being understood that in said formula (I) when Q represents

a 2-(phenylaminocarbonyl) phenyl radical, Q1 a 1,2-cyclopropanediyl radical, $Q_2$ an ortho-phenylene radical, $X_2$ an oxygen atom, $R_2$ a hydrogen atom and that a and b are equal to 0, $R_1$ does not represent a phenyl radical.

**2.** Compound of formula (I) as defined in claim 1 in which Q is non substituted or substituted by one or more substituents chosen from halogen atoms or the methylenedioxy, difluoromethylenedioxy, tetrafluoro ethylenedioxy, cyano, nitro, cyanato, thiocyanato, pentafluorothio, fluorosulphonyl, or $R-(T)_c-$ groups in which R represents a hydrogen atom or a radical derived from a cyclic or acyclic, aromatic or non aromatic linear or branched, saturated or unsaturated hydrocarbon, and comprising 1 to 8 carbon atoms, itself being able to be either non substituted, or substituted by one or more halogen atoms, c is equal to 0 or to 1 and T represents a divalent oxy, carbonyl, carbonyloxy, oxycarbonyl, thio, sulphinyl, sulphonyl, sulphonyloxy, $-(CO)_d-N(R')-(CO)_e-(O)_f-$ or $-N(R'')-S(O)_g-$ group in which R' and R" represent independently of R a hydrogen atom or a radical derived from a cyclic or acyclic, aromatic or non aromatic, linear or branched, saturated or unsaturated hydrocarbon, and comprising 1 to 8 carbon atoms, itself being able to be either non substituted, or substituted by one or more halogen atoms, d, e and f are equal to 0 or 1, f is equal to 0 when e is equal to 0, the sum e + d is equal to 0 or 1, and g is equal to 0, 1 or 2, $Q_1$ is non substituted or substituted by one or more substituents chosen from halogen atoms, the cyano, azido, or saturated or unsaturated aliphatic radicals comprising 1 to 4 carbon atoms non substituted or substituted by one or more halogen atoms and $Q_2$ is non substituted or substituted by one or more substituents chosen from halogen atoms or the methylenedioxy, difluoromethylenedioxy, tetrafluoro ethylenedioxy, cyano, nitro, amino, alkylamino, alkenylamino, cyanato, thiocyanato, pentafluorothio, fluorosulphonyl, or $R-(T)_c-$ groups in which R represents a hydrogen atom or a radical derived from a cyclic or acyclic, aromatic or non aromatic linear or branched, saturated or unsaturated hydrocarbon, and comprising 1 to 8 carbon atoms, itself being able to be either non substituted, or substituted by one or more halogen atoms, c is equal to 0 or 1 and T represents a divalent oxy, carbonyl, carbonyloxy, oxycarbonyl, thio, sulphinyl, sulphonyl, sulphonyloxy, $-(CO)_d-N(R')-(CO)_e-(O)_f-$ or $-N(R'')-S(O)_g-$ group in which R', R", d, e, f and g are as defined previously.

**3.** Compound of formula (I) as defined in claim 1 or 2, in which Q is a phenyl radical or a naphthyl radical, non substituted or substituted by 1 to 3 substituents.

**4.** Compound of formula (I) as defined in claim 3 in which Q represents a radical chosen from the phenyl, 2-chloro phenyl, 3-chloro phenyl, 3-bromo phenyl, 3-(trifluoromethyl) phenyl, 4-chloro phenyl, 4-bromo phenyl, 4-iodo phenyl, 4-(trifluoromethyl) phenyl, 4-nitrophenyl, 4-methoxy phenyl, 4-(difluoromethoxy) phenyl, 4-(trifluoromethoxy) phenyl, 3-bromo 4-(difluoromethoxy) phenyl, 4-(2,2-dibromo ethenyl) phenyl, 4-ethynyl phenyl, 4-benzyl phenyl, 3,4-dibromo phenyl, 2,4-dichloro phenyl, 3,4-dichloro phenyl, 3,4-difluorophenyl, 3-chloro 4-iodo phenyl, 4-bromo 3-chloro phenyl, 4-bromo 2-fluoro phenyl, 4-bromo 3-fluoro phenyl, 4-bromo 3-(trifluoromethyl) phenyl, 4-chloro 3-(trifluoromethyl) phenyl, 3,5-bis(trifluoromethyl) phenyl, 3,4,5-trichloro phenyl, 4-bromo 3,5-dichloro phenyl, 3-phenoxyphenyl, 4-(fluoro 3-phenoxy) phenyl, 3-bromo 4-(trifluoromethylsulphonyloxy) phenyl, 3,4-bis (trifluoromethylsulphonyloxy) phenyl, 2-naphthyl or 5-bromo 2-naphthyl or 6-bromo 1-naphthyl radicals.

**5.** Compound of formula (I) as defined in any one of claims 1 to 4, in which the steric configuration of $Q_1$ is such that the $Q-(CH_2)_a-(X_1)_b-$ group is in trans position relative to the $Q_2-C(X_2)-N(R_1)(R_2)$ group.

**6.** Compound of formula (I) as defined in any one of claims 1 to 5 in which $Q_1$ is non substituted or substituted by 1 or 2 halogen atoms or 1 or 2 methyl radicals.

**7.** Compound of formula (I) as defined in claim 6 in which $Q_1$ represents a radical chosen from the 1,2-cyclopropanediyl, 1-fluoro 1,2-cyclopropanediyl, 1-chloro 1,2-cyclopropanediyl, 1-bromo 1,2-cyclopropanediyl, 1-methyl 1,2-cyclopropanediyl, 3,3-dibromo 1,2-cyclopropanediyl and 1,2-difluoro 1,2-cyclopropanediyl radicals.

**8.** Compound of formula (I) as defined in any one of claims 1 to 7, in which $Q_2$ represents one of the ortho-phenylene, meta-phenylene, para-phenylene, 1,2-naphthylene, 1,3-naphthylene, 1,4-naphthylene, 1,5-naphthylene, 2,5-naphthylene, 4,5-indenylene, 4,6-indenylene, 4,7-indenylene, 4,5-indanylene, 4,6-indanylene, 4,7-indanylene, 1,2,3,4-tetrahydro 5,6-naphthylene, 1,2,3,4-tetrahydro 5,7-naphthylene, 1,2,3,4-tetrahydro 5,8-naphthylene radicals, non substituted or substituted by 1 to 4 substituents.

**9.** Compounds of formula (I) as defined in claim 8 in which $Q_2$ represents one of the 2-methyl 1,4-phenylene, 2,3,5,6-tetrafluoro 1,4-phenylene, 3-methyl 1,4-phenylene, 2-chloro 1,4-phenylene, 2-acetylamino 5-chloro 1,4-phenylene, 2-amino 5-chloro 1,4-phenylene, 2-dimethylamino 5-chloro 1,4-phenylene radicals.

**10.** Compound of formula (I) as defined in any one of claims 1 to 9, in which $R_1$ represents a branched alkyl radical comprising 1 to 6 carbon atoms such as the isopropyl, isobutyl, 1,2-dimethyl propyl, 1,1,2-trimethyl propyl or 2,2-dimethyl propyl, 1-methylpropyl, 2-methylbutyl, 2,2-dimethyl 1-methylethyl, 3-chloropropyl, cyclobutyl, cyclohexyl, cyclopropylmethyl radical, a (2-methyl 1,3-dioxolan 2-yl) methyl radical, or a branched alkenyl radical comprising 2 to 5 carbon atoms such as the 2-methyl 2-propenyl, 2-chloro 2-propenyl, 2-bromo 2-propenyl radical or also a non substituted phenyl radical or a substituted phenyl radical such as the 2-fluoro phenyl, 2-chloro phenyl, 2-bromo phenyl, 2-iodo phenyl, 2-nitro phenyl, 2-hydroxy phenyl, 2-methoxyphenyl, 2-cyano phenyl, 2-amino phenyl, 2-(dimethylamino) phenyl, 2-(trifluoromethoxy) phenyl, 2-phenoxy phenyl, 2-methoxycarbonyl phenyl, 2-ethoxycarbonyl phenyl, 2-phenylcarbonylphenyl, 2-formyl phenyl, 2-acetyl phenyl, 2-(methylthio) phenyl, 2-fluorosulphonyl phenyl, 2-ethynyl phenyl, 2-(1-methyl 2-propenyl) phenyl, 2-(hydroxymethyl) phenyl, 2-methyl phenyl, 2-(terbutyl) phenyl, 2-(fluoromethyl) phenyl, 2-(difluoromethyl) phenyl, 2-(trifluoromethyl) phenyl, 2-ethyl phenyl, 2-propyl phenyl, 2-isopropyl phenyl, 2-(pyridin-1-yl) phenyl, 3-chloro phenyl, 3-bromophenyl, 3-methoxy phenyl, 3-fluoro phenyl, 3-methyl phenyl, 3-phenoxyphenyl, 4-fluoro phenyl, 4-chloro phenyl, 4-cyano phenyl, 4-(cyanomethyl) phenyl, 4-methoxy phenyl, 4-methyl phenyl, 4-(terbutyl) phenyl), 4-(trifluoromethyl) phenyl, 4-phenoxyphenyl, 4-benzyloxyphenyl, 4-methoxycarbonylphenyl, 4-cyclohexylphenyl, 2,4-dichlorophenyl, 2,5-dichlorophenyl, 2,6-dichlorophenyl, 3,4-dichlorophenyl, 3,5-dichlorophenyl, 2,3-dimethylphenyl, 2,4-dimethylphenyl, 2,5-dimethylphenyl, 2,6-dimethylphenyl, 3,5-dimethylphenyl, 2,4,6-trimethylphenyl, 2,5-dimethoxyphenyl, 3,4-dimethoxyphenyl, 3,5-dimethoxyphenyl, 2-methoxy 5-methylphenyl, 2-methoxy 5-trifluoromethyl, 3-methoxy 5-trifluoromethylphenyl, 2-methoxy 5-nitrophenyl, 2-nitro 4-methoxyphenyl, 2-methyl 4-methoxyphenyl, 3,5-dichloro 4-methoxyphenyl, 4,5-dichloro 2-methoxyphenyl, 4-bromo 3,5-dichlorophenyl, 2-(1-methylethyl) phenyl, 3-chloro 2-methyl phenyl, 3-fluoro 2-methyl phenyl, 3-bromo 2-methyl phenyl, 2,3-dichloro phenyl, 2,3-dibromo phenyl, 3,4-dibromophenyl, 4-fluoro 2-methyl phenyl, 4-chloro 2-fluoro phenyl, 4-fluoro 2-(trifluoromethyl) phenyl, 2,4-difluoro phenyl, 2-chloro 4-methylphenyl, 2-chloro 5-methyl phenyl, 5-chloro 2-methyl phenyl, 5-fluoro 2-methyl phenyl, 5-iodo 2-methyl phenyl, 2,6-difluoro phenyl, 2-fluoro 4-methylphenyl, 2-fluoro 6-methyl phenyl, 2-chloro 6-methyl phenyl radical or a radical derived from a heterocycle such as the 2-chloro 3-pyridyl, 3-pyridyl, 2-pyridyl, 4-pyridyl, 2-chloro 3-pyridyl, 3-methyl pyridyl, 5-methyl isoxazol-3-yl, 1,3,4-thiadiazol-2-yl, 4-pyrimidinyl, 3-pyrazolyl, 4-(trifluoromethyl) thiazol-2-yl, 5-chloro 4-(trifluoromethyl) thiazol-2-yl, 1-piperidinyl, 2,6-dimethyl 1-piperidinyl, 1,4-oxazin-4-yl or 1-perhydroazepinyl radical, or finally $R_1$ and $R_2$ represent together with the nitrogen atom to which they are linked a heterocycle such as the 1,4-thiazin-4-yl, 1,4-oxazin-4-yl, 1-aziridinyl, 1-piperidinyl, 1-pyrrolidinyl, 2-methylpiperidin-1-yl, 2-hydroxymethylpyrrolidin-1-yl radical.

**11.** Compound of formula (I) as defined in any one of claims 1 to 10, in which $R_2$ represents a hydrogen atom, an alkyl radical comprising 1 to 4 carbon atoms, in particular the ethyl radical, a benzyl radical, an ethoxymethyl radical, a propoxymethyl radical or a group (A) in which $X_3$ represents an oxygen atom and $R_3$ represents an alkyl radical comprising 1 to 11 carbon atoms, an alkoxycarbonyl radical comprising 2 to 5 carbon atoms or phenoxycarbonyl radical, and $R_2$ represents in particular in this case, the acetyl hexanoyl, decanoyl, 2-methoxy 2-oxo acetyl, 2-ethoxy 2-oxo acetyl, 2-phenoxy 2-oxo acetyl radical or a group (B) in which $R_3$ represents an alkoxy radical comprising 1 to 4 carbon atoms and $R_2$ represents in particular in this case the diethoxyphosphonyl radical or a group (C) in which $T_2$ represents a divalent thio radical and $R_5$ a trifluoromethyl or pentafluoroethyl radical or a phenyl radical non substituted or substituted by one or more halogen atoms or one or more alkyl radicals comprising 1 to 4 carbon atoms, and in this case $R_2$ represents in particular the trifluoromethylthio, pentafluoroethylthio radical or a phenylthio, (4-chloro phenyl) thio, (2-methyl phenyl) thio, (4-methyl phenyl) thio or (4-tertbutyl phenyl) thio radical or a group (D) in which j is equal to O and $R_2$ represents in particular in this case the (N-formyl N-methyl amino) thio or (N-methoxycarbonyl N-methyl amino) thio radical.

**12.** Compound of formula (I) as defined in any one of claims 1 to 11 in which a and b are equal to 0.

**13.** Process for the preparation of the compound of formula (I) as defined in any one of claims 1 to 12, characterized in that the acid or the acid derivative corresponding to formula (II):

$$Q\text{-}(CH_2)_a\text{-}(X_1)_b\text{-}Q_1\text{-}Q_2\text{-}C(O)\text{-}Z \qquad (II)$$

in which Q, $X_1$, a, b, $Q_1$ and $Q_2$ are as defined in claims 1 to 12 and Z represents a hydroxy group, a halogenated group, an alkoxy radical comprising 1 to 4 carbon atoms or a -P(O)(OΦ)-NHΦ group, in which Φ represents a phenyl group, is reacted with an amine of formula (III):

$$HN(R_1)(R_2) \quad (III)$$

in which $R_1$ and $R_2$ are as defined previously, in order to obtain a corresponding product of formula (I), in which $X_2$ represents an oxygen atom, which if necessary, is converted into a product of formula (I) in which ($X_2$) represents a sulphur atom.

**14.** Use of the compounds of formula (I) as defined in any one of claims 1 to 13 as a pesticide in particular as an insecticide, acaricide and nematicide for the protection of crops in particular rice and cotton crops, or for the treatment of storage areas for the products of said crops and in particular as an insecticide or acaricide in domestic or public premises.

**15.** Pesticide composition comprising:

a) a compound of formula (I) as defined in any one of claims 1 to 13,
b) inert excipients appropriate for the use of said product of formula (I) as pesticide.

**16.** Pesticide composition comprising:

a) a compound of formula (I) as defined in any one of claims 1 to 13,
b) inert excipients appropriate for the use of said product of formula (I) in the veterinary field.

**17.** Compound of formula (I) as defined in one of claims 1 to 13, for the implementation of a treatment method for the human or animal body characterized in that a pharmaceutically acceptable formulation of said compound is applied on said body.